# EUROPEAN PATENT SPECIFICATION

(11) **EP 2 475 427 B1**
(45) Date of publication and mention of the grant of the patent: **30.11.2016**
(21) Application number: 10816109.2
(22) Date of filing: 09.09.2010
(51) Int. Cl.: A61P 19/08, A61K 38/18, A61K 38/17, A61P 21/00, A61P 19/10, A61K 31/663, C07K 14/705

(54) **ACTRIIB ANTAGONISTS AND DOSING AND USES THEREOF**
ACTRIIB-ANTAGONISTEN, IHRE DOSIERUNG UND ANWENDUNGEN DAVON
ANTAGONISTES D'ACTRIIB, ET DOSAGE ET ADMINISTRATION ASSOCIÉS

(30) Priority: 09.09.2009 US 276287 P
(43) Date of publication of application: 18.07.2012
(60) Divisional application: 16195379.9
(73) Proprietor: Acceleron Pharma, Inc., Cambridge, MA 02139 (US)
(72) Inventor: SEEHRA, Jasbir, Lexington MA 02421-6818 (US); KNOPF, John, Carlisle MA 01741 (US); ATTIE, Ken, Boston, MA 02116 (US)
(74) Representative: Evenson, Jane Harriet
(86) International application number: PCT/US2010/048322
(87) International publication number: WO 2011/031901

(56) References cited:
- WO-A1-2010/151426
- WO-A2-2004/039948
- WO-A2-2006/012627
- WO-A2-2008/076437
- WO-A2-2009/158025
- DATABASE BIOSIS [Online] BIOSCIENCES INFORMATION SERVICE, PHILADELPHIA, PA, US; April 2008 (2008-04), PEARSALL SCOTT ET AL: "An investigative pharmacology study of a GDF-8 (myostatin) inhibitor, ACE-031, in the Common Marmoset (Callithrix Jacchus)", XP002696892, Database accession no. PREV201200750016 & FASEB JOURNAL, vol. 22, April 2008 (2008-04), EXPERIMENTAL BIOLOGY ANNUAL MEETING; SAN DIEGO, CA, USA; APRIL 05 -09, 2008 ISSN: 0892-6638(print)
- JON RUCKLE ET AL: "Single-Dose, Randomized, Double-Blind, Placebo-Controlled Study of ACE-011 (ActRIIA-IgG1) in Postmenopausal Women*", JOURNAL OF BONE AND MINERAL RESEARCH, vol. 24, no. 4, 1 December 2008 (2008-12-01), pages 744-752, XP055031032, ISSN: 0884-0431, DOI: 10.1359/jbmr.081208

### SEQUENCE LISTING

<110> ACCELERON PHARMA INC.
<120> ACTRIIB ANTAGONISTS AND DOSING AND USES THEREOF
<130> P38540EP-PCT
<140> EP10816109.2
   <141> 2010-09-09
<150> US 61/276,287
   <151> 2009-09-09
<160> 35
<170> PatentIn version 3.5
<210> 1
   <211> 512
   <212> PRT
   <213> Homo sapiens
<400> 1
   Met Thr Ala Pro Trp Val Ala Leu Ala Leu Leu Trp Gly Ser Leu Trp
   1               5                   10                  15
   Pro Gly Ser Gly Arg Gly Glu Ala Glu Thr Arg Glu Cys Ile Tyr Tyr
               20                  25                  30
   Asn Ala Asn Trp Glu Leu Glu Arg Thr Asn Gln Ser Gly Leu Glu Arg
           35                  40                  45
   Cys Glu Gly Glu Gln Asp Lys Arg Leu His Cys Tyr Ala Ser Trp Ala
       50                  55                  60
   Asn Ser Ser Gly Thr Ile Glu Leu Val Lys Lys Gly Cys Trp Leu Asp
   65                  70                  75                  80
   Asp Phe Asn Cys Tyr Asp Arg Gln Glu Cys Val Ala Thr Glu Glu Asn
                   85                  90                  95
   Pro Gln Val Tyr Phe Cys Cys Cys Glu Gly Asn Phe Cys Asn Glu Arg
               100                 105                 110
   Phe Thr His Leu Pro Glu Ala Gly Gly Pro Glu Val Thr Tyr Glu Pro
           115                 120                 125
   Pro Pro Thr Ala Pro Thr Leu Leu Thr Val Leu Ala Tyr Ser Leu Leu
       130                 135                 140
   Pro Ile Gly Gly Leu Ser Leu Ile Val Leu Leu Ala Phe Trp Met Tyr
   145                 150                 155                 160
   Arg His Arg Lys Pro Pro Tyr Gly His Val Asp Ile His Glu Asp Pro
                   165                 170                 175
   Gly Pro Pro Pro Pro Ser Pro Leu Val Gly Leu Lys Pro Leu Gln Leu
               180                 185                 190
   Leu Glu Ile Lys Ala Arg Gly Arg Phe Gly Cys Val Trp Lys Ala Gln
           195                 200                 205
   Leu Met Asn Asp Phe Val Ala Val Lys Ile Phe Pro Leu Gln Asp Lys
       210                 215                 220
   Gln Ser Trp Gln Ser Glu Arg Glu Ile Phe Ser Thr Pro Gly Met Lys
   225                 230                 235                 240
   His Glu Asn Leu Leu Gln Phe Ile Ala Ala Glu Lys Arg Gly Ser Asn
                   245                 250                 255
   Leu Glu Val Glu Leu Trp Leu Ile Thr Ala Phe His Asp Lys Gly Ser
               260                 265                 270
   Leu Thr Asp Tyr Leu Lys Gly Asn Ile Ile Thr Trp Asn Glu Leu Cys
           275                 280                 285
   His Val Ala Glu Thr Met Ser Arg Gly Leu Ser Tyr Leu His Glu Asp
       290                 295                 300
   Val Pro Trp Cys Arg Gly Glu Gly His Lys Pro Ser Ile Ala His Arg
   305                 310                 315                 320
   Asp Phe Lys Ser Lys Asn Val Leu Leu Lys Ser Asp Leu Thr Ala Val
                   325                 330                 335
   Leu Ala Asp Phe Gly Leu Ala Val Arg Phe Glu Pro Gly Lys Pro Pro
               340                 345                 350
   Gly Asp Thr His Gly Gln Val Gly Thr Arg Arg Tyr Met Ala Pro Glu
           355                 360                 365
   Val Leu Glu Gly Ala Ile Asn Phe Gln Arg Asp Ala Phe Leu Arg Ile
       370                 375                 380
   Asp Met Tyr Ala Met Gly Leu Val Leu Trp Glu Leu Val Ser Arg Cys
   385                 390                 395                 400
   Lys Ala Ala Asp Gly Pro Val Asp Glu Tyr Met Leu Pro Phe Glu Glu
                   405                 410                 415
   Glu Ile Gly Gln His Pro Ser Leu Glu Glu Leu Gln Glu Val Val Val
               420                 425                 430
   His Lys Lys Met Arg Pro Thr Ile Lys Asp His Trp Leu Lys His Pro
           435                 440                 445
   Gly Leu Ala Gln Leu Cys Val Thr Ile Glu Glu Cys Trp Asp His Asp
       450                 455                 460
   Ala Glu Ala Arg Leu Ser Ala Gly Cys Val Glu Glu Arg Val Ser Leu
   465                 470                 475                 480
   Ile Arg Arg Ser Val Asn Gly Thr Thr Ser Asp Cys Leu Val Ser Leu
                   485                 490                 495
   Val Thr Ser Val Thr Asn Val Asp Leu Pro Pro Lys Glu Ser Ser Ile
               500                 505                 510
<210> 2
   <211> 115
   <212> PRT
   <213> Homo sapiens
<400> 2
   Gly Arg Gly Glu Ala Glu Thr Arg Glu Cys Ile Tyr Tyr Asn Ala Asn
   1               5                   10                  15
   Trp Glu Leu Glu Arg Thr Asn Gln Ser Gly Leu Glu Arg Cys Glu Gly
               20                  25                  30
   Glu Gln Asp Lys Arg Leu His Cys Tyr Ala Ser Trp Ala Asn Ser Ser
           35                  40                  45
   Gly Thr Ile Glu Leu Val Lys Lys Gly Cys Trp Leu Asp Asp Phe Asn
       50                  55                  60
   Cys Tyr Asp Arg Gln Glu Cys Val Ala Thr Glu Glu Asn Pro Gln Val
   65                  70                  75                  80
   Tyr Phe Cys Cys Cys Glu Gly Asn Phe Cys Asn Glu Arg Phe Thr His
                   85                  90                  95
   Leu Pro Glu Ala Gly Gly Pro Glu Val Thr Tyr Glu Pro Pro Pro Thr
               100                 105                 110
   Ala Pro Thr
           115
<210> 3
   <211> 100
   <212> PRT
   <213> Homo sapiens
<210> 4
   <211> 1539
   <212> DNA
   <213> Homo sapiens
<400> 4
   atgacggcgc cctgggtggc cctcgccctc ctctggggat cgctgtggcc cggctctggg 60
   cgtggggagg ctgagacacg ggagtgcatc tactacaacg ccaactggga gctggagcgc 120
   accaaccaga gcggcctgga gcgctgcgaa ggcgagcagg acaagcggct gcactgctac 180
   gcctcctggg ccaacagctc tggcaccatc gagctcgtga agaagggctg ctggctagat 240
   gacttcaact gctacgatag gcaggagtgt gtggccactg aggagaaccc ccaggtgtac 300
   ttctgctgct gtgaaggcaa cttctgcaac gagcgcttca ctcatttgcc agaggctggg 360
   ggcccggaag tcacgtacga gccacccccg acagccccca ccctgctcac ggtgctggcc 420
   tactcactgc tgcccatcgg gggcctttcc ctcatcgtcc tgctggcctt ttggatgtac 480
   cggcatcgca agccccccta cggtcatgtg gacatccatg aggaccctgg gcctccacca 540
   ccatcccctc tggtgggcct gaagccactg cagctgctgg agatcaaggc tcgggggcgc 600
   tttggctgtg tctggaaggc ccagctcatg aatgactttg tagctgtcaa gatcttccca 660
   ctccaggaca agcagtcgtg gcagagtgaa cgggagatct tcagcacacc tggcatgaag 720
   cacgagaacc tgctacagtt cattgctgcc gagaagcgag gctccaacct cgaagtagag 780
   ctgtggctca tcacggcctt ccatgacaag ggctccctca cggattacct caaggggaac 840
   atcatcacat ggaacgaact gtgtcatgta gcagagacga tgtcacgagg cctctcatac 900
   ctgcatgagg atgtgccctg gtgccgtggc gagggccaca agccgtctat tgcccacagg 960
   gactttaaaa gtaagaatgt attgctgaag agcgacctca cagccgtgct ggctgacttt 1020
   ggcttggctg ttcgatttga gccagggaaa cctccagggg acacccacgg acaggtaggc 1080
   acgagacggt acatggctcc tgaggtgctc gagggagcca tcaacttcca gagagatgcc 1140
   ttcctgcgca ttgacatgta tgccatgggg ttggtgctgt gggagcttgt gtctcgctgc 1200
   aaggctgcag acggacccgt ggatgagtac atgctgccct ttgaggaaga gattggccag 1260
   cacccttcgt tggaggagct gcaggaggtg gtggtgcaca agaagatgag gcccaccatt 1320
   aaagatcact ggttgaaaca cccgggcctg gcccagcttt gtgtgaccat cgaggagtgc 1380
   tgggaccatg atgcagaggc tcgcttgtcc gcgggctgtg tggaggagcg ggtgtccctg 1440
   attcggaggt cggtcaacgg cactacctcg gactgtctcg tttccctggt gacctctgtc 1500
   accaatgtgg acctgccccc taaagagtca agcatctaa 1539
<210> 5
   <211> 348
   <212> DNA
   <213> Homo sapiens
<400> 5
   tctgggcgtg gggaggctga gacacgggag tgcatctact acaacgccaa ctgggagctg        60
   gagcgcacca accagagcgg cctggagcgc tgcgaaggcg agcaggacaa gcggctgcac       120
   tgctacgcct cctgggccaa cagctctggc accatcgagc tcgtgaagaa gggctgctgg       180
   ctagatgact tcaactgcta cgataggcag gagtgtgtgg ccactgagga gaacccccag       240
   gtgtacttct gctgctgtga aggcaacttc tgcaacgagc gcttcactca tttgccagag       300
   gctgggggcc cggaagtcac gtacgagcca cccccgacag cccccacc                    348
<210> 6
   <211> 225
   <212> PRT
   <213> Artificial Sequence
<220>
   <223> Description of Artificial Sequence: Synthetic polypeptide
<220>
   <221> variant
   <222> (43)..(43)
   <223> can be Ala
<220>
   <221> variant
   <222> (100)..(100)
   <223> can be Ala
<220>
   <221> variant
   <222> (212)..(212)
   <223> can be Ala
<400> 6 Lys
   225
<210> 7
   <211> 343
   <212> PRT
   <213> Homo sapiens
<400> 7
   Met Val Arg Ala Arg His Gln Pro Gly Gly Leu Cys Leu Leu Leu Leu
   1               5                   10                  15
   Leu Leu Cys Gln Phe Met Glu Asp Arg Ser Ala Gln Ala Gly Asn Cys
               20                  25                  30
   Trp Leu Arg Gln Ala Lys Asn Gly Arg Cys Gln Val Leu Tyr Lys Thr
           35                  40                  45
   Glu Leu Ser Lys Glu Glu Cys Cys Ser Thr Gly Arg Leu Ser Thr Ser
       50                  55                  60
   Trp Thr Glu Glu Asp Val Asn Asp Asn Thr Leu Phe Lys Trp Met Ile
   65                  70                  75                  80
   Phe Asn Gly Gly Ala Pro Asn Cys Ile Pro Cys Lys Glu Thr Cys Glu
                   85                  90                  95
   Asn Val Asp Cys Gly Pro Gly Lys Lys Cys Arg Met Asn Lys Lys Asn
               100                 105                 110
   Lys Pro Arg Cys Val Cys Ala Pro Asp Cys Ser Asn Ile Thr Trp Lys
           115                 120                 125
   Gly Pro Val Cys Gly Leu Asp Gly Lys Thr Tyr Arg Asn Glu Cys Ala
       130                 135                 140
   Leu Leu Lys Ala Arg Cys Lys Glu Gln Pro Glu Leu Glu Val Gln Tyr
   145                 150                 155                 160
   Gln Gly Arg Cys Lys Lys Thr Cys Arg Asp Val Phe Cys Pro Gly Ser
                   165                 170                 175
   Ser Thr Cys Val Val Asp Gln Thr Asn Asn Ala Tyr Cys Val Thr Cys
               180                 185                 190
   Asn Arg Ile Cys Pro Glu Pro Ala Ser Ser Glu Gln Tyr Leu Cys Gly
           195                 200                 205
   Asn Asp Gly Val Thr Tyr Ser Ser Ala Cys His Leu Arg Lys Ala Thr
       210                 215                 220
   Cys Leu Leu Gly Arg Ser Ile Gly Leu Ala Tyr Glu Gly Lys Cys Ile
   225                 230                 235                 240
   Lys Ala Lys Ser Cys Glu Asp Ile Gln Cys Thr Gly Gly Lys Lys Cys
               245                 250                 255
   Leu Trp Asp Phe Lys Val Gly Arg Gly Arg Cys Ser Leu Cys Asp Glu
               260                 265                 270
   Leu Cys Pro Asp Ser Lys Ser Asp Glu Pro Val Cys Ala Ser Asp Asn
           275                 280                 285
   Ala Thr Tyr Ala Ser Glu Cys Ala Met Lys Glu Ala Ala Cys Ser Ser
       290                 295                 300
   Gly Val Leu Leu Glu Val Lys His Gly Ser Cys Asn Ser Ile Ser Glu
   305                 310                 315                 320
   Asp Thr Glu Glu Glu Glu Glu Asp Glu Asp Gln Asp Tyr Ser Phe Pro
                   325                 330                 335
   Ile Ser Ser Ile Leu Glu Trp
               340
<210> 8
   <211> 317
   <212> PRT
   <213> Homo sapiens
<400> 8
   Met Val Arg Ala Arg His Gln Pro Gly Gly Leu Cys Leu Leu Leu Leu
   1               5                   10                  15
   Leu Leu Cys Gln Phe Met Glu Asp Arg Ser Ala Gln Ala Gly Asn Cys
               20                  25                  30
   Trp Leu Arg Gln Ala Lys Asn Gly Arg Cys Gln Val Leu Tyr Lys Thr
           35                  40                  45
   Glu Leu Ser Lys Glu Glu Cys Cys Ser Thr Gly Arg Leu Ser Thr Ser
       50                  55                  60
   Trp Thr Glu Glu Asp Val Asn Asp Asn Thr Leu Phe Lys Trp Met Ile
   65                  70                  75                  80
   Phe Asn Gly Gly Ala Pro Asn Cys Ile Pro Cys Lys Glu Thr Cys Glu
                   85                  90                  95
   Asn Val Asp Cys Gly Pro Gly Lys Lys Cys Arg Met Asn Lys Lys Asn
               100                 105                 110
   Lys Pro Arg Cys Val Cys Ala Pro Asp Cys Ser Asn Ile Thr Trp Lys
           115                 120                 125
   Gly Pro Val Cys Gly Leu Asp Gly Lys Thr Tyr Arg Asn Glu Cys Ala
   130 135 140
   Leu Leu Lys Ala Arg Cys Lys Glu Gln Pro Glu Leu Glu Val Gln Tyr
   145 150 155 160
   Gln Gly Arg Cys Lys Lys Thr Cys Arg Asp Val Phe Cys Pro Gly Ser
   165 170 175
   Ser Thr Cys Val Val Asp Gln Thr Asn Asn Ala Tyr Cys Val Thr Cys
   180 185 190
   Asn Arg Ile Cys Pro Glu Pro Ala Ser Ser Glu Gln Tyr Leu Cys Gly
   195 200 205
   Asn Asp Gly Val Thr Tyr Ser Ser Ala Cys His Leu Arg Lys Ala Thr
   210 215 220
   Cys Leu Leu Gly Arg Ser Ile Gly Leu Ala Tyr Glu Gly Lys Cys Ile
   225 230 235 240
   Lys Ala Lys Ser Cys Glu Asp Ile Gln Cys Thr Gly Gly Lys Lys Cys
   245 250 255
   Leu Trp Asp Phe Lys Val Gly Arg Gly Arg Cys Ser Leu Cys Asp Glu
   260 265 270
   Leu Cys Pro Asp Ser Lys Ser Asp Glu Pro Val Cys Ala Ser Asp Asn
   275 280 285
   Ala Thr Tyr Ala Ser Glu Cys Ala Met Lys Glu Ala Ala Cys Ser Ser
   290 295 300
   Gly Val Leu Leu Glu Val Lys His Ser Gly Ser Cys Asn
   305 310 315
<210> 9
   <211> 63
   <212> PRT
   <213> Homo sapiens
<400> 9
   Gly Asn Cys Trp Leu Arg Gln Ala Lys Asn Gly Arg Cys Gln Val Leu
   1               5                   10                  15
   Tyr Lys Thr Glu Leu Ser Lys Glu Glu Cys Cys Ser Thr Gly Arg Leu
               20                  25                  30
   Ser Thr Ser Trp Thr Glu Glu Asp Val Asn Asp Asn Thr Leu Phe Lys
           35                  40                  45
   Trp Met Ile Phe Asn Gly Gly Ala Pro Asn Cys Ile Pro Cys Lys
       50                  55                  60
<210> 10
   <211> 25
   <212> PRT
   <213> Homo sapiens
<400> 10
   Glu Thr Cys Glu Asn Val Asp Cys Gly Pro Gly Lys Lys Cys Arg Met
   1               5                   10                  15
   Asn Lys Lys Asn Lys Pro Arg Cys Val
               20                  25
<210> 11
   <211> 26
   <212> PRT
   <213> Homo sapiens
<400> 11
   Lys Thr Cys Arg Asp Val Phe Cys Pro Gly Ser Ser Thr Cys Val Val
   1               5                   10                  15
   Asp Gln Thr Asn Asn Ala Tyr Cys Val Thr
               20                  25
<210> 12
   <211> 262
   <212> PRT
   <213> Homo sapiens
<400> 12
   Met Arg Pro Gly Ala Pro Gly Pro Leu Trp Pro Leu Pro Trp Gly Ala
   1               5                   10                  15
   Leu Ala Trp Ala Val Gly Phe Val Ser Ser Met Gly Ser Gly Asn Pro
               20                  25                  30
   Ala Pro Gly Gly Val Cys Trp Leu Gln Gln Gly Gln Glu Ala Thr Cys
           35                  40                  45
   Ser Leu Val Leu Gln Thr Asp Val Thr Arg Ala Glu Cys Cys Ala Ser
       50                  55                  60
   Gly Asn Ile Asp Thr Ala Trp Ser Asn Leu Thr His Pro Gly Asn Lys
   65                  70                  75                  80
   Ile Asn Leu Leu Gly Phe Leu Gly Leu Val His Cys Leu Pro Cys Lys
                   85                  90                  95
   Asp Ser Cys Asp Gly Val Glu Cys Gly Pro Gly Lys Ala Cys Arg Met
               100                 105                 110
   Leu Gly Gly Arg Pro Arg Cys Glu Cys Ala Pro Asp Cys Ser Gly Leu
           115                 120                 125
   Pro Ala Arg Leu Gln Val Cys Gly Ser Asp Gly Ala Thr Tyr Arg Asp
       130                 135                 140
   Glu Cys Glu Leu Arg Ala Ala Arg Cys Arg Gly His Pro Asp Leu Ser
   145                 150                 155                 160
   Val Met Tyr Arg Gly Arg Cys Arg Lys Ser Cys Glu His Val Val Cys
                   165                 170                 175
   Pro Arg Pro Gln Ser Cys Val Val Asp Gln Thr Gly Ser Ala His Cys
               180                 185                 190
   Val Val Cys Arg Ala Ala Pro Cys Val Pro Ser Ser Pro Gly Gln Glu
           195                 200                 205
   Leu Cys Gly Asn Asn Asn Val Thr Tyr Ile Ser Ser Cys His Met Arg
       210                 215                 220
   Gln Ala Thr Cys Phe Leu Gly Arg Ser Ile Gly Val Arg His Ala Gly
   225                 230                 235                 240
   Ser Cys Ala Gly Thr Pro Glu Glu Pro Pro Gly Gly Glu Ser Ala Glu
                   245                 250                 255
   Glu Glu Glu Asn Phe Val
               260
<210> 13
   <211> 343
   <212> PRT
   <213> Artificial Sequence
<220>
   <223> Description of Artificial Sequence: Synthetic
   polypeptide
<400> 13
   Gly Arg Gly Glu Ala Glu Thr Arg Glu Cys Ile Tyr Tyr Asn Ala Asn
   1               5                   10                  15
   Trp Glu Leu Glu Arg Thr Asn Gln Ser Gly Leu Glu Arg Cys Glu Gly
               20                  25                  30
   Glu Gln Asp Lys Arg Leu His Cys Tyr Ala Ser Trp Arg Asn Ser Ser
           35                  40                  45
   Gly Thr Ile Glu Leu Val Lys Lys Gly Cys Trp Leu Asp Asp Phe Asn
   50 55 60
   Cys Tyr Asp Arg Gln Glu Cys Val Ala Thr Glu Glu Asn Pro Gln Val
   65 70 75 80
   Tyr Phe Cys Cys Cys Glu Gly Asn Phe Cys Asn Glu Arg Phe Thr His
   85 90 95
   Leu Pro Glu Ala Gly Gly Pro Glu Val Thr Tyr Glu Pro Pro Pro Thr
   100 105 110
   Ala Pro Thr Gly Gly Gly Thr His Thr Cys Pro Pro Cys Pro Ala Pro
   115 120 125
   Glu Leu Leu Gly Gly Pro Ser Val Phe Leu Phe Pro Pro Lys Pro Lys
   130 135 140
   Asp Thr Leu Met Ile Ser Arg Thr Pro Glu Val Thr Cys Val Val Val
   145 150 155 160
   Asp Val Ser His Glu Asp Pro Glu Val Lys Phe Asn Trp Tyr Val Asp
   165 170 175
   Gly Val Glu Val His Asn Ala Lys Thr Lys Pro Arg Glu Glu Gln Tyr
   180 185 190
   Asn Ser Thr Tyr Arg Val Val Ser Val Leu Thr Val Leu His Gln Asp
   195 200 205
   Trp Leu Asn Gly Lys Glu Tyr Lys Cys Lys Val Ser Asn Lys Ala Leu
   210 215 220
   Pro Val Pro Ile Glu Lys Thr Ile Ser Lys Ala Lys Gly Gln Pro Arg
   225 230 235 240
   Glu Pro Gln Val Tyr Thr Leu Pro Pro Ser Arg Glu Glu Met Thr Lys
   245 250 255
   Asn Gln Val Ser Leu Thr Cys Leu Val Lys Gly Phe Tyr Pro Ser Asp
   260 265 270
   Ile Ala Val Glu Trp Glu Ser Asn Gly Gln Pro Glu Asn Asn Tyr Lys
   275 280 285
   Thr Thr Pro Pro Val Leu Asp Ser Asp Gly Ser Phe Phe Leu Tyr Ser
   290 295 300
   Lys Leu Thr Val Asp Lys Ser Arg Trp Gln Gln Gly Asn Val Phe Ser
   305 310 315 320
   Cys Ser Val Met His Glu Ala Leu His Asn His Tyr Thr Gln Lys Ser
   325 330 335
   Leu Ser Leu Ser Pro Gly Lys
   340
<210> 14
   <211> 21
   <212> PRT
   <213> Unknown
<220>
   <223> Description of Unknown: Honey bee mellitin peptide
<400> 14
   Met Lys Phe Leu Val Asn Val Ala Leu Val Phe Met Val Val Tyr Ile
   1 5 10 15
   Ser Tyr Ile Tyr Ala
   20
<210> 15
   <211> 22
   <212> PRT
   <213> Unknown
<220>
   <223> Description of Unknown: Tissue plasminogen activator
   peptide
<400> 15
   Met Asp Ala Met Lys Arg Gly Leu Cys Cys Val Leu Leu Leu Cys Gly
   1 5 10 15
   Ala Val Phe Val Ser Pro
   20
<210> 16
   <211> 20
   <212> PRT
   <213> Unknown
<220>
   <223> Description of Unknown: Native leader peptide
<400> 16
   Met Gly Ala Ala Ala Lys Leu Ala Phe Ala Val Phe Leu Ile Ser Cys
   1 5 10 15
   Ser Ser Gly Ala
   20
<210> 17
   <211> 368
   <212> PRT
   <213> Artificial Sequence
<220>
   <223> Description of Artificial Sequence: Synthetic
   polypeptide
<400> 17
   Met Asp Ala Met Lys Arg Gly Leu Cys Cys Val Leu Leu Leu Cys Gly
   1               5                   10                  15
   Ala Val Phe Val Ser Pro Gly Ala Ser Gly Arg Gly Glu Ala Glu Thr
               20                  25                  30
   Arg Glu Cys Ile Tyr Tyr Asn Ala Asn Trp Glu Leu Glu Arg Thr Asn
           35                  40                  45
   Gln Ser Gly Leu Glu Arg Cys Glu Gly Glu Gln Asp Lys Arg Leu His
       50                  55                  60
   Cys Tyr Ala Ser Trp Arg Asn Ser Ser Gly Thr Ile Glu Leu Val Lys
   65                  70                  75                  80
   Lys Gly Cys Trp Leu Asp Asp Phe Asn Cys Tyr Asp Arg Gln Glu Cys
                   85                  90                  95
   Val Ala Thr Glu Glu Asn Pro Gln Val Tyr Phe Cys Cys Cys Glu Gly
               100                 105                 110
   Asn Phe Cys Asn Glu Arg Phe Thr His Leu Pro Glu Ala Gly Gly Pro
           115                 120                 125
   Glu Val Thr Tyr Glu Pro Pro Pro Thr Ala Pro Thr Gly Gly Gly Thr
       130                 135                 140
   His Thr Cys Pro Pro Cys Pro Ala Pro Glu Leu Leu Gly Gly Pro Ser
   145                 150                 155                 160
   Val Phe Leu Phe Pro Pro Lys Pro Lys Asp Thr Leu Met Ile Ser Arg
                   165                 170                 175
   Thr Pro Glu Val Thr Cys Val Val Val Asp Val Ser His Glu Asp Pro
               180                 185                 190
   Glu Val Lys Phe Asn Trp Tyr Val Asp Gly Val Glu Val His Asn Ala
           195                 200                 205
   Lys Thr Lys Pro Arg Glu Glu Gln Tyr Asn Ser Thr Tyr Arg Val Val
       210                 215                 220
   Ser Val Leu Thr Val Leu His Gln Asp Trp Leu Asn Gly Lys Glu Tyr
   225                 230                 235                 240
   Lys Cys Lys Val Ser Asn Lys Ala Leu Pro Val Pro Ile Glu Lys Thr
                   245                 250                 255
   Ile Ser Lys Ala Lys Gly Gln Pro Arg Glu Pro Gln Val Tyr Thr Leu
               260                 265                 270
   Pro Pro Ser Arg Glu Glu Met Thr Lys Asn Gln Val Ser Leu Thr Cys
           275                 280                 285
   Leu Val Lys Gly Phe Tyr Pro Ser Asp Ile Ala Val Glu Trp Glu Ser
       290                 295                 300
   Asn Gly Gln Pro Glu Asn Asn Tyr Lys Thr Thr Pro Pro Val Leu Asp
   305                 310                 315                 320
   Ser Asp Gly Ser Phe Phe Leu Tyr Ser Lys Leu Thr Val Asp Lys Ser
                   325                 330                 335
   Arg Trp Gln Gln Gly Asn Val Phe Ser Cys Ser Val Met His Glu Ala
               340                 345                 350
   Leu His Asn His Tyr Thr Gln Lys Ser Leu Ser Leu Ser Pro Gly Lys
           355                 360                 365
<210> 18
   <211> 1107
   <212> DNA
   <213> Artificial Sequence
<220>
   <223> Description of Artificial Sequence: Synthetic polynucleotide
<400> 18
   atggatgcaa tgaagagagg gctctgctgt gtgctgctgc tgtgtggagc agtcttcgtt      60
   tcgcccggcg cctctgggcg tggggaggct gagacacggg agtgcatcta ctacaacgcc     120
   aactgggagc tggagcgcac caaccagagc ggcctggagc gctgcgaagg cgagcaggac     180
   aagcggctgc actgctacgc ctcctggcgc aacagctctg gcaccatcga gctcgtgaag     240
   aagggctgct ggctagatga cttcaactgc tacgataggc aggagtgtgt ggccactgag     300
   gagaaccccc aggtgtactt ctgctgctgt gaaggcaact tctgcaacga gcgcttcact     360
   catttgccag aggctggggg cccggaagtc acgtacgagc cacccccgac agcccccacc     420
   ggtggtggaa ctcacacatg cccaccgtgc ccagcacctg aactcctggg gggaccgtca     480
   gtcttcctct tccccccaaa acccaaggac accctcatga tctcccggac ccctgaggtc     540
   acatgcgtgg tggtggacgt gagccacgaa gaccctgagg tcaagttcaa ctggtacgtg     600
   gacggcgtgg aggtgcataa tgccaagaca aagccgcggg aggagcagta caacagcacg     660
   taccgtgtgg tcagcgtcct caccgtcctg caccaggact ggctgaatgg caaggagtac     720
   aagtgcaagg tctccaacaa agccctccca gtccccatcg agaaaaccat ctccaaagcc     780
   aaagggcagc cccgagaacc acaggtgtac accctgcccc catcccggga ggagatgacc     840
   aagaaccagg tcagcctgac ctgcctggtc aaaggcttct atcccagcga catcgccgtg     900
   gagtgggaga gcaatgggca gccggagaac aactacaaga ccacgcctcc cgtgctggac     960
   tccgacggct ccttcttcct ctatagcaag ctcaccgtgg acaagagcag gtggcagcag    1020
   gggaacgtct tctcatgctc cgtgatgcat gaggctctgc acaaccacta cacgcagaag    1080
   agcctctccc tgtctccggg taaatga                                        1107
<210> 19
   <211> 6
   <212> PRT
   <213> Artificial Sequence
<220>
   <223> Description of Artificial Sequence: Synthetic
   peptide
<400> 19
   Gly Arg Gly Glu Ala Glu
   1               5
<210> 20
   <211> 344
   <212> PRT
   <213> Artificial Sequence
<220>
   <223> Description of Artificial Sequence: Synthetic
   polypeptide
<400> 20
   Ser Gly Arg Gly Glu Ala Glu Thr Arg Glu Cys Ile Tyr Tyr Asn Ala
   1               5                   10                  15
   Asn Trp Glu Leu Glu Arg Thr Asn Gln Ser Gly Leu Glu Arg Cys Glu
               20                  25                  30
   Gly Glu Gln Asp Lys Arg Leu His Cys Tyr Ala Ser Trp Arg Asn Ser
           35                  40                  45
   Ser Gly Thr Ile Glu Leu Val Lys Lys Gly Cys Trp Leu Asp Asp Phe
       50                  55                  60
   Asn Cys Tyr Asp Arg Gln Glu Cys Val Ala Thr Glu Glu Asn Pro Gln
   65                  70                  75                  80
   Val Tyr Phe Cys Cys Cys Glu Gly Asn Phe Cys Asn Glu Arg Phe Thr
                   85                  90                  95
   His Leu Pro Glu Ala Gly Gly Pro Glu Val Thr Tyr Glu Pro Pro Pro
               100                 105                 110
   Thr Ala Pro Thr Gly Gly Gly Thr His Thr Cys Pro Pro Cys Pro Ala
           115                 120                 125
   Pro Glu Leu Leu Gly Gly Pro Ser Val Phe Leu Phe Pro Pro Lys Pro
       130                 135                 140
   Lys Asp Thr Leu Met Ile Ser Arg Thr Pro Glu Val Thr Cys Val Val
   145                 150                 155                 160
   Val Asp Val Ser His Glu Asp Pro Glu Val Lys Phe Asn Trp Tyr Val
                   165                 170                 175
   Asp Gly Val Glu Val His Asn Ala Lys Thr Lys Pro Arg Glu Glu Gln
               180                 185                 190
   Tyr Asn Ser Thr Tyr Arg Val Val Ser Val Leu Thr Val Leu His Gln
           195                 200                 205
   Asp Trp Leu Asn Gly Lys Glu Tyr Lys Cys Lys Val Ser Asn Lys Ala
       210                 215                 220
   Leu Pro Val Pro Ile Glu Lys Thr Ile Ser Lys Ala Lys Gly Gln Pro
   225                 230                 235                 240
   Arg Glu Pro Gln Val Tyr Thr Leu Pro Pro Ser Arg Glu Glu Met Thr
                   245                 250                 255
   Lys Asn Gln Val Ser Leu Thr Cys Leu Val Lys Gly Phe Tyr Pro Ser
               260                 265                 270
   Asp Ile Ala Val Glu Trp Glu Ser Asn Gly Gln Pro Glu Asn Asn Tyr
           275                 280                 285
   Lys Thr Thr Pro Pro Val Leu Asp Ser Asp Gly Ser Phe Phe Leu Tyr
       290                 295                 300
   Ser Lys Leu Thr Val Asp Lys Ser Arg Trp Gln Gln Gly Asn Val Phe
   305                 310                 315                 320
   Ser Cys Ser Val Met His Glu Ala Leu His Asn His Tyr Thr Gln Lys
                   325                 330                 335
   Ser Leu Ser Leu Ser Pro Gly Lys
               340
<210> 21
   <211> 4
   <212> PRT
   <213> Artificial Sequence
<220>
   <223> Description of Artificial Sequence: Synthetic
   peptide
<400> 21
   Thr Gly Gly Gly
   1
<210> 22
   <211> 5
   <212> PRT
   <213> Artificial Sequence
<220>
   <223> Description of Artificial Sequence: Synthetic
   peptide
<400> 22
   Thr Gly Gly Gly Gly
   1 5
<210> 23
   <211> 4
   <212> PRT
   <213> Artificial Sequence
<220>
   <223> Description of Artificial Sequence: Synthetic
   peptide
<400> 23
   Ser Gly Gly Gly
   1
<210> 24
   <211> 5
   <212> PRT
   <213> Artificial Sequence
<220>
   <223> Description of Artificial Sequence: Synthetic
   peptide
<400> 24
   Ser Gly Gly Gly Gly
   1 5
<210> 25
   <211> 6
   <212> PRT
   <213> Artificial Sequence
<220>
   <223> Description of Artificial Sequence: Synthetic
   6xHis tag
<400> 25
   His His His His His His
   1               5
<210> 26
   <211> 150
   <212> PRT
   <213> Rattus sp.
<400> 26
   Met Thr Ala Pro Trp Ala Ala Leu Ala Leu Leu Trp Gly Ser Leu Cys
   1               5                   10                  15
   Ala Gly Ser Gly Arg Gly Glu Ala Glu Thr Arg Glu Cys Ile Tyr Tyr
               20                  25                  30
   Asn Ala Asn Trp Glu Leu Glu Arg Thr Asn Gln Ser Gly Leu Glu Arg
           35                  40                  45
   Cys Glu Gly Glu Gln Asp Lys Arg Leu His Cys Tyr Ala Ser Trp Pro
       50                  55                  60
   Asn Ser Ser Gly Thr Ile Glu Leu Val Lys Lys Gly Cys Trp Leu Asp
   65                  70                  75                  80
   Asp Phe Asn Cys Tyr Asp Arg Gln Glu Cys Val Ala Thr Glu Glu Asn
                   85                  90                  95
   Pro Gln Val Tyr Phe Cys Cys Cys Glu Gly Asn Phe Cys Asn Glu Arg
               100                 105                 110
   Phe Thr His Leu Pro Glu Pro Gly Gly Pro Glu Val Thr Tyr Glu Pro
           115                 120                 125
   Pro Pro Thr Ala Pro Thr Leu Leu Thr Val Leu Ala Tyr Ser Leu Leu
       130                 135                 140
   Pro Ile Gly Gly Leu Ser
   145                 150
<210> 27
   <211> 150
   <212> PRT
   <213> Sus sp.
<400> 27
   Met Thr Ala Pro Trp Ala Ala Leu Ala Leu Leu Trp Gly Ser Leu Cys
   1               5                   10                  15
   Val Gly Ser Gly Arg Gly Glu Ala Glu Thr Arg Glu Cys Ile Tyr Tyr
               20                  25                  30
   Asn Ala Asn Trp Glu Leu Glu Arg Thr Asn Gln Ser Gly Leu Glu Arg
           35                  40                  45
   Cys Glu Gly Glu Gln Asp Lys Arg Leu His Cys Tyr Ala Ser Trp Arg
       50                  55                  60
   Asn Ser Ser Gly Thr Ile Glu Leu Val Lys Lys Gly Cys Trp Leu Asp
   65                  70                  75                  80
   Asp Phe Asn Cys Tyr Asp Arg Gln Glu Cys Val Ala Thr Glu Glu Asn
                   85                  90                  95
   Pro Gln Val Tyr Phe Cys Cys Cys Glu Gly Asn Phe Cys Asn Glu Arg
               100                 105                 110
   Phe Thr His Leu Pro Glu Ala Gly Gly Pro Glu Val Thr Tyr Glu Pro
           115                 120                 125
   Pro Pro Thr Ala Pro Thr Leu Leu Thr Val Leu Ala Tyr Ser Leu Leu
       130                 135                 140
   Pro Ile Gly Gly Leu Ser
   145                 150
<210> 28
   <211> 150
   <212> PRT
   <213> Mus sp.
<400> 28
   Met Thr Ala Pro Trp Ala Ala Leu Ala Leu Leu Trp Gly Ser Leu Cys
   1               5                   10                  15
   Ala Gly Ser Gly Arg Gly Glu Ala Glu Thr Arg Glu Cys Ile Tyr Tyr
               20                  25                  30
   Asn Ala Asn Trp Glu Leu Glu Arg Thr Asn Gln Ser Gly Leu Glu Arg
           35                  40                  45
   Cys Glu Gly Glu Gln Asp Lys Arg Leu His Cys Tyr Ala Ser Trp Arg
       50                  55                  60
   Asn Ser Ser Gly Thr Ile Glu Leu Val Lys Lys Gly Cys Trp Leu Asp
   65                  70                  75                  80
   Asp Phe Asn Cys Tyr Asp Arg Gln Glu Cys Val Ala Thr Glu Glu Asn
                   85                  90                  95
   Pro Gln Val Tyr Phe Cys Cys Cys Glu Gly Asn Phe Cys Asn Glu Arg
               100                 105                 110
   Phe Thr His Leu Pro Glu Pro Gly Gly Pro Glu Val Thr Tyr Glu Pro
           115                 120                 125
   Pro Pro Thr Ala Pro Thr Leu Leu Thr Val Leu Ala Tyr Ser Leu Leu
       130                 135                 140
   Pro Ile Gly Gly Leu Ser
   145                 150
<210> 29
   <211> 150
   <212> PRT
   <213> Homo sapiens
<400> 29
   Met Thr Ala Pro Trp Val Ala Leu Ala Leu Leu Trp Gly Ser Leu Cys
   1               5                   10                  15
   Ala Gly Ser Gly Arg Gly Glu Ala Glu Thr Arg Glu Cys Ile Tyr Tyr
               20                  25                  30
   Asn Ala Asn Trp Glu Leu Glu Arg Thr Asn Gln Ser Gly Leu Glu Arg
           35                  40                  45
   Cys Glu Gly Glu Gln Asp Lys Arg Leu His Cys Tyr Ala Ser Trp Arg
       50                  55                  60
   Asn Ser Ser Gly Thr Ile Glu Leu Val Lys Lys Gly Cys Trp Leu Asp
   65                  70                  75                  80
   Asp Phe Asn Cys Tyr Asp Arg Gln Glu Cys Val Ala Thr Glu Glu Asn
                   85                  90                  95
   Pro Gln Val Tyr Phe Cys Cys Cys Glu Gly Asn Phe Cys Asn Glu Arg
               100                 105                 110
   Phe Thr His Leu Pro Glu Ala Gly Gly Pro Glu Val Thr Tyr Glu Pro
           115                 120                 125
   Pro Pro Thr Ala Pro Thr Leu Leu Thr Val Leu Ala Tyr Ser Leu Leu
       130                 135                 140
   Pro Ile Gly Gly Leu Ser
   145                 150
<210> 30
   <211> 150
   <212> PRT
   <213> Unknown
<220>
   <223> Description of Unknown: Bovine ActRIIb polypeptide
<400> 30
   Met Thr Ala Pro Trp Ala Ala Leu Ala Leu Leu Trp Gly Ser Leu Cys
   1               5                   10                  15
   Ala Gly Ser Gly Arg Gly Glu Ala Glu Thr Arg Glu Cys Ile Tyr Tyr
               20                  25                  30
   Asn Ala Asn Trp Glu Leu Glu Arg Thr Asn Gln Ser Gly Leu Glu Arg
           35                  40                  45
   Cys Glu Gly Glu Arg Asp Lys Arg Leu His Cys Tyr Ala Ser Trp Arg
       50                  55                  60
   Asn Ser Ser Gly Thr Ile Glu Leu Val Lys Lys Gly Cys Trp Leu Asp
   65                  70                  75                  80
   Asp Phe Asn Cys Tyr Asp Arg Gln Glu Cys Val Ala Thr Glu Glu Asn
                   85                  90                  95
   Pro Gln Val Tyr Phe Cys Cys Cys Glu Gly Asn Phe Cys Asn Glu Arg
               100                 105                 110
   Phe Thr His Leu Pro Glu Ala Gly Gly Pro Glu Val Thr Tyr Glu Pro
           115                 120                 125
   Pro Pro Thr Ala Pro Thr Leu Leu Thr Val Leu Ala Tyr Ser Leu Leu
       130                 135                 140
   Pro Val Gly Gly Leu Ser
   145                 150
<210> 31
   <211> 150
   <212> PRT
   <213> Xenopus sp.
<400> 31
   Met Gly Ala Ser Val Ala Leu Thr Phe Leu Leu Leu Leu Ala Thr Phe
   1               5                   10                  15
   Arg Ala Gly Ser Gly His Asp Glu Val Glu Thr Arg Glu Cys Ile Tyr
               20                  25                  30
   Tyr Asn Ala Asn Trp Glu Leu Glu Lys Thr Asn Gln Ser Gly Val Glu
           35                  40                  45
   Arg Leu Val Glu Gly Lys Lys Asp Lys Arg Leu His Cys Tyr Ala Ser
       50                  55                  60
   Trp Arg Asn Asn Ser Gly Phe Ile Glu Leu Val Lys Lys Gly Cys Trp
   65                  70                  75                  80
   Leu Asp Asp Phe Asn Cys Tyr Asp Arg Gln Glu Cys Ile Ala Lys Glu
                   85                  90                  95
   Glu Asn Pro Gln Val Phe Phe Cys Cys Cys Glu Gly Asn Tyr Cys Asn
               100                 105                 110
   Lys Lys Phe Thr His Leu Pro Glu Val Glu Thr Phe Asp Pro Lys Pro
           115                 120                 125
   Gln Pro Ser Ala Ser Val Leu Asn Ile Leu Ile Tyr Ser Leu Leu Pro
       130                 135                 140
   Ile Val Gly Leu Ser Met
   145                 150
<210> 32
   <211> 150
   <212> PRT
   <213> Homo sapiens
<400> 32
   Met Gly Ala Ala Ala Lys Leu Ala Phe Ala Val Phe Leu Ile Ser Cys
   1               5                   10                  15
   Ser Ser Gly Ala Ile Leu Gly Arg Ser Glu Thr Gln Glu Cys Leu Phe
               20                  25                  30
   Phe Asn Ala Asn Trp Glu Lys Asp Arg Thr Asn Gln Thr Gly Val Glu
           35                  40                  45
   Pro Cys Tyr Gly Asp Lys Asp Lys Arg Arg His Cys Phe Ala Thr Trp
       50                  55                  60
   Lys Asn Ile Ser Gly Ser Ile Glu Ile Val Lys Gln Gly Cys Trp Leu
   65                  70                  75                  80
   Asp Asp Ile Asn Cys Tyr Asp Arg Thr Asp Cys Val Glu Lys Lys Asp
                   85                  90                  95
   Ser Pro Glu Val Tyr Phe Cys Cys Cys Glu Gly Asn Met Cys Asn Glu
               100                 105                 110
   Lys Phe Ser Tyr Phe Pro Glu Met Glu Val Thr Gln Pro Thr Ser Asn
           115                 120                 125
   Pro Val Thr Pro Lys Pro Pro Tyr Tyr Asn Ile Leu Leu Tyr Ser Leu
       130                 135                 140
   Val Pro Leu Met Leu Ile
   145                 150
<210> 33
   <211> 154
   <212> PRT
   <213> Artificial Sequence
<220>
   <223> Description of Artificial Sequence: Synthetic
   consensus polypeptide
<220>
   <221> variant
   <222> (8)..(8)
   <223> can be Ala
<220>
   <221> variant
   <222> (121)..(121)
   <223> can be Ala, Val or Met
<400> 33
   Met Thr Ala Pro Trp Ala Ala Thr Leu Ala Leu Leu Trp Gly Ser Leu
   1               5                   10                  15
   Cys Ala Gly Ser Gly Arg Gly Glu Ala Glu Thr Arg Glu Cys Ile Tyr
               20                  25                  30
   Tyr Asn Ala Asn Trp Glu Leu Glu Arg Thr Asn Gln Ser Gly Leu Glu
           35                  40                  45
   Arg Leu Cys Glu Gly Glu Gln Asp Lys Arg Leu His Cys Tyr Ala Ser
       50                  55                  60
   Trp Arg Asn Ser Ser Gly Thr Ile Glu Leu Val Lys Lys Gly Cys Trp
   65                  70                  75                  80
   Leu Asp Asp Phe Asn Cys Tyr Asp Arg Gln Glu Cys Val Ala Thr Glu
                   85                  90                  95
   Glu Asn Pro Gln Val Tyr Phe Cys Cys Cys Glu Gly Asn Phe Cys Asn
               100                 105                 110
   Glu Arg Phe Thr His Leu Pro Glu Pro Gly Gly Pro Glu Val Thr Tyr
           115                 120                 125
   Glu Pro Lys Pro Pro Thr Ala Pro Thr Leu Leu Thr Val Leu Ala Tyr
       130                 135                 140
   Ser Leu Leu Pro Ile Gly Gly Leu Ser Met
   145                 150
<210> 34
   <211> 116
   <212> PRT
   <213> Homo sapiens
<400> 34
   Ile Leu Gly Arg Ser Glu Thr Gln Glu Cys Leu Phe Phe Asn Ala Asn
   1               5                   10                  15
   Trp Glu Lys Asp Arg Thr Asn Gln Thr Gly Val Glu Pro Cys Tyr Gly
               20                  25                  30
   Asp Lys Asp Lys Arg Arg His Cys Phe Ala Thr Trp Lys Asn Ile Ser
           35                  40                  45
   Gly Ser Ile Glu Ile Val Lys Gln Gly Cys Trp Leu Asp Asp Ile Asn
       50                  55                  60
   Cys Tyr Asp Arg Thr Asp Cys Val Glu Lys Lys Asp Ser Pro Glu Val
   65                  70                  75                  80
   Tyr Phe Cys Cys Cys Glu Gly Asn Met Cys Asn Glu Lys Phe Ser Tyr
                   85                  90                  95
   Phe Pro Glu Met Glu Val Thr Gln Pro Thr Ser Asn Pro Val Thr Pro
               100                 105                 110
   Lys Pro Pro Thr
           115
<210> 35
   <211> 115
   <212> PRT
   <213> Homo sapiens
<400> 35
   Gly Arg Gly Glu Ala Glu Thr Arg Glu Cys Ile Tyr Tyr Asn Ala Asn
   1               5                   10                  15
   Trp Glu Leu Glu Arg Thr Asn Gln Ser Gly Leu Glu Arg Cys Glu Gly
               20                  25                  30
   Glu Gln Asp Lys Arg Leu His Cys Tyr Ala Ser Trp Arg Asn Ser Ser
           35                  40                  45
   Gly Thr Ile Glu Leu Val Lys Lys Gly Cys Trp Leu Asp Asp Phe Asn
       50                  55                  60
   Cys Tyr Asp Arg Gln Glu Cys Val Ala Thr Glu Glu Asn Pro Gln Val
   65                  70                  75                  80
   Tyr Phe Cys Cys Cys Glu Gly Asn Phe Cys Asn Glu Arg Phe Thr His
                   85                  90                  95
   Leu Pro Glu Ala Gly Gly Pro Glu Val Thr Tyr Glu Pro Pro Pro Thr
               100                 105                 110
   Ala Pro Thr
           115

## Description

### BACKGROUND OF THE INVENTION

Disorders of the bone, ranging from osteoporosis to fractures, represent a set of pathological states for which there are few effective pharmaceutical agents. Treatment instead focuses on physical and behavioral interventions, including immobilization, exercise and changes in diet. It would be beneficial to have therapeutic agents that promote bone growth and increase bone density for the purpose of treating a variety of bone disorders.

Bone growth and mineralization are dependent on the activities of two cell types, osteoclasts and osteoblasts, although chondrocytes and cells of the vasculature also participate in critical aspects of these processes. Developmentally, bone formation occurs through two mechanisms, endochondral ossification and intramembranous ossification, with the former responsible for longitudinal bone formation and the later responsible for the formation of topologically flat bones, such as the bones of the skull. Endochondral ossification requires the sequential formation and degradation of cartilaginous structures in the growth plates that serve as templates for the formation of osteoblasts, osteoclasts, the vasculature and subsequent mineralization. During intramembranous ossification, bone is formed directly in the connective tissues. Both processes require the infiltration of osteoblasts and subsequent matrix deposition.

Fractures and other structural disruptions of bone are healed through a process that, at least superficially, resembles the sequence of developmental events of osteogenesis, including the formation of cartilaginous tissue and subsequent mineralization. The process of fracture healing can occur in two ways. Direct or primary bone healing occurs without callus formation. Indirect or secondary bone healing occurs with a callus precursor stage. Primary healing of fractures involves the reformation of mechanical continuity across a closely-set disruption. Under suitable conditions, bone-resorbing cells surrounding the disruption show a tunnelling resorptive response and establish pathways for the penetration of blood vessels and subsequent healing. Secondary healing of bones follows a process of inflammation, soft callus formation, callus mineralisation and callus remodelling. In the inflammation stage, haematoma and haemorrhage formation results from the disruption of periosteal and endosteal blood vessels at the site of injury. Inflammatory cells invade the area. In soft callus formation stage, the cells produce new vessels, fibroblasts, intracellular material and supporting cells, forming granulation tissue in the space between the fracture fragments. Clinical union across the disruption is established by fibrous or cartilaginous tissue (soft callus). Osteoblasts are formed and mediate the mineralization of soft callus, which is then replaced by lamellar bone and subjected to the normal remodeling processes.

In addition to fractures and other physical disruptions of bone structure, loss of bone mineral content and bone mass can be caused by a wide variety of conditions and may result in significant medical problems. Changes to bone mass occur in a relatively predictable way over the life of an individual. Up to about age 30, bones of both men and women grow to maximal mass through linear growth of the endochondral growth plates and radial growth. After about age 30 (for trabecular bone, e.g., flat bones such as the vertebrae and pelvis) and age 40 (for cortical bone, e.g., long bones found in the limbs), slow bone loss occurs in both men and women. In women, a final phase of substantial bone loss also occurs, probably due to postmenopausal estrogen deficiencies. During this phase, women may lose an additional 10% of bone mass from the cortical bone and 25% from the trabecular compartment. Whether progressive bone loss results in a pathological condition such as osteoporosis depends largely on the initial bone mass of the individual and whether there are exacerbating conditions.

Bone loss is sometimes characterized as an imbalance in the normal bone remodeling process. Healthy bone is constantly subject to remodeling. Remodeling begins with resorption of bone by osteoclasts. The resorbed bone is then replaced by new bone tissue, which is characterized by collagen formation by osteoblasts, and subsequent calcification. In healthy individuals the rates of resorption and formation are balanced. Osteoporosis is a chronic, progressive condition, marked by a shift towards resorption, resulting in an overall decrease in bone mass and bone mineralization. Osteoporosis in humans is preceded by clinical osteopenia (bone mineral density that is greater than one standard deviation but less than 2.5 standard deviations below the mean value for young adult bone). Worldwide, approximately 75 million people are at risk for osteoporosis.

Thus, methods for controlling the balance between osteoclast and osteoblast activity can be useful for promoting the healing of fractures and other damage to bone as well as the treatment of disorders, such as osteoporosis, associated with loss of bone mass and bone mineralization.

With respect to osteoporosis, estrogen, calcitonin, osteocalcin with vitamin K, or high doses of dietary calcium are all used as therapeutic interventions. Other therapeutic approaches to osteoporosis include bisphosphonates, parathyroid hormone, calcimimetics, statins, anabolic steroids, lanthanum and strontium salts, and sodium fluoride. Such therapeutics, however, are often associated with undesirable side effects.

Thus, it is an object of the present disclosure to provide compositions and methods for promoting bone growth and mineralization.

### SUMMARY OF THE INVENTION

The present invention is defined by the claims.

In part, the disclosure demonstrates that ActRIIb antagonists, including ActRIIb-based molecules (e.g., molecules comprising a ligand-binding portion of an ActRIIb extracellular domain or a variant thereof, such as an ActRIIb-Fc) and other antagonists of ActRIIb signaling, can be used to increase bone density, promote bone growth, and/or increase bone strength. In particular, the disclosure demonstrates that a soluble form of ActRIIb promotes increased bone density, bone growth, and bone strength in vivo. While most pharmaceutical agents that promote bone growth or inhibit bone loss act as either anti-catabolic agents (also commonly referred to as "catabolic agents") (e.g., bisphosphonates) or anabolic agents (e.g., parathyroid hormone, PTH, when appropriately dosed), the soluble ActRIIb protein exhibits dual activity, having both anti-catabolic and anabolic effects. Thus, ActRIIb antagonists may be used to increase bone density and promote bone growth. Anti-ActRIIb antibodies, and ActRIIb-targeted small molecules and aptamers, and nucleic acids that decrease expression of ActRIIb may also be used to treat disorders associated with low bone density or low bone strength, such as osteoporosis, or to promote bone growth in patients in need thereof, such as in patients having a bone fracture (such molecules are also included in the term "ActRIIb antagonists" as used herein). The disclosure further indicates that ActRIIb antagonists are effective in preventing and/or repairing bone damage caused by multiple myeloma tumors and metastases to the bone (e.g., in breast, esophageal, colon, prostate or lung cancer) and, additionally, that ActRIIb antagonists diminish the tumor load in multiple myeloma.

ActRIIb antagonists may also be used for the treatment of a variety of disorders or conditions, in particular, muscle and neuromuscular disorders (e.g., muscular dystrophy, amyotrophic lateral sclerosis (ALS), and muscle atrophy), adipose tissue disorders (e.g., obesity), metabolic disorders (e.g., type 2 diabetes), neurodegenerative disorders, and muscle wasting associated with old age (sarcopenia), prostate cancer therapy, and cancer cachexia. In specific embodiments, ActRIIb antagonists of the invention (e.g., soluble ActRIIb polypeptides) can antagonize an ActRIIb receptor in any process associated with ActRIIb activity. Optionally, ActRIIb antagonists of the invention may be designed to preferentially antagonize one or more ligands of ActRIIb receptors, such as GDF8 (also called myostatin), GDF11, activin A, activin B, activin AB, Nodal, and BMP7 (also called OP-1), and may therefore be useful in the treatment of additional disorders. Examples of ActRIIb antagonists include the naturally occurring ActRIIb polypeptides as well as functional variants thereof.

In certain aspects, the disclosure provides polypeptides comprising a soluble, ActRIIb polypeptide. ActRIIb polypeptides may be formulated as a pharmaceutical preparation comprising the ActRIIb polypeptide and a pharmaceutically acceptable carrier. Optionally, the ActRIIb polypeptide binds to activin with a K_{D} less than 1 micromolar or less than 100, 10 or 1 nanomolar. Preferably the composition is at least 95% pure, with respect to other polypeptide components, as assessed by size exclusion chromatography, and more preferably, the composition is at least 98% pure. An ActRIIb polypeptide for use in such a preparation may be any of those disclosed herein, such as a polypeptide having an amino acid sequence selected from SEQ ID NOs: 2, 3, 13, 17, or 20, or having an amino acid sequence that is at least 80%, 85%, 90%, 95%, 97% or 99% identical to an amino acid sequence selected from SEQ ID NOs: 2, 3, 13, 17, or 20. An ActRIIb polypeptide may include a functional fragment of a natural ActRIIb polypeptide, such as one comprising at least 10, 20 or 30 amino acids of a sequence selected from SEQ ID NOs: 1-3 or a sequence of SEQ ID NO: 2, lacking the C-terminal 10 to 15 amino acids (the "tail"). An ActRIIb polypeptide may be encoded by a nucleic acid that hybridizes under stringent conditions to the nucleic acid of SEQ ID NO: 3 or 5.

An ActRIIb polypeptide may include one or more alterations in the amino acid sequence (e.g., in the ligand-binding domain) relative to a naturally occurring ActRIIb polypeptide. Examples of altered ActRIIb polypeptides are provided in WO 2006/012627, pp. 59-60. The alteration in the amino acid sequence may, for example, alter glycosylation of the polypeptide when produced in a mammalian, insect or other eukaryotic cell or alter proteolytic cleavage of the polypeptide relative to the naturally occurring ActRIIb polypeptide.

An ActRIIb polypeptide may be a fusion protein that has, as one domain, an ActRIIb polypeptide (e.g., a ligand-binding portion of an ActRIIb or variant thereof) and one or more additional domains that provide a desirable property, such as improved pharmacokinetics, easier purification, targeting to particular tissues, etc. For example, a domain of a fusion protein may enhance one or more of in vivo stability, in vivo half life, uptake/administration, tissue localization or distribution, formation of protein complexes, multimerization of the fusion protein, and/or purification. Dimerization or multimerization may provide increased ligand-binding affinity. An ActRIIb fusion protein may include an immunoglobulin Fc domain (wild-type or mutant) or a serum albumin or other polypeptide portion that provides desirable properties such as improved pharmacokinetics, improved solubility or improved stability. Typically, an ActRIIb-Fc fusion protein will be produced as a homodimeric complex. Optionally, an ActRIIb-Fc fusion comprises a relatively unstructured linker positioned between the Fc domain and the extracellular ActRIIb domain. This unstructured linker may correspond to the roughly 15 amino acid unstructured region at the C-terminal end of the extracellular domain of ActRIIb (the "tail"), or it may be an artificial sequence of 1, 2, 3, 4 or 5 amino acids or a length of between 5 and 15, 20, 30, 50 or more amino acids that are relatively free of secondary structure, or a mixture of both. A linker may be rich in glycine and proline residues and may, for example, contain a single sequence of threonine/serine and glycines or repeating sequences of threonine/serine and glycines (e.g., TG₃, TG₄, SG₃ or SG₄ singlets or repeats). A fusion protein may include a purification subsequence, such as an epitope tag, a FLAG tag, a polyhistidine sequence, and a GST fusion. Optionally, a soluble ActRIIb polypeptide includes one or more modified amino acid residues selected from: a glycosylated amino acid, a PEGylated amino acid, a farnesylated amino acid, an acetylated amino acid, a biotinylated amino acid, an amino acid conjugated to a lipid moiety, and an amino acid conjugated to an organic derivatizing agent. A pharmaceutical preparation may also include one or more additional compounds such as a compound that is used to treat a bone disorder. Preferably, a pharmaceutical preparation is substantially pyrogen free. In general, it is preferable that an ActRIIb protein be expressed in a mammalian cell line that mediates suitably natural glycosylation of the ActRIIb protein so as to diminish the likelihood of an unfavorable immune response in a patient. Human and CHO cell lines have been used successfully, and it is expected that other common mammalian expression systems will be useful.

In certain aspects, the disclosure provides nucleic acids encoding an ActRIIb polypeptide. An isolated polynucleotide may comprise a coding sequence for an ActRIIb polypeptide, such as described above. For example, an isolated nucleic acid may include a sequence coding for an extracellular domain (e.g., ligand-binding domain) of an ActRIIb and a sequence that would code for part or all of the transmembrane domain and/or the cytoplasmic domain of an ActRIIb, but for a stop codon positioned within the transmembrane domain or the cytoplasmic domain, or positioned between the extracellular domain and the transmembrane domain or cytoplasmic domain. For example, an isolated polynucleotide may comprise a full-length ActRIIb polynucleotide sequence such as SEQ ID NO: 4 or 5, or a partially truncated version, said isolated polynucleotide further comprising a transcription termination codon at least six hundred nucleotides before the 3'-terminus or otherwise positioned such that translation of the polynucleotide gives rise to an extracellular domain optionally fused to a truncated portion of a full-length ActRIIb. A preferred nucleic acid sequence is SEQ ID NO: 18. Nucleic acids disclosed herein may be operably linked to a promoter for expression, and the disclosure provides cells transformed with such recombinant polynucleotides. Preferably the cell is a mammalian cell such as a CHO cell.

In certain aspects, the disclosure provides methods for making an ActRIIb polypeptide. Such a method may include expressing any of the nucleic acids (e.g., SEQ ID NO: 4, 5 or 18) disclosed herein in a suitable cell, such as a Chinese hamster ovary (CHO) cell. Such a method may comprise: a) culturing a cell under conditions suitable for expression of the ActRIIb polypeptide, wherein said cell is transformed with an ActRIIb expression construct; and b) recovering the ActRIIb polypeptide so expressed. ActRIIb polypeptides may be recovered as crude, partially purified or highly purified fractions. Purification may be achieved by a series of purification steps, including, for example, one, two or three or more of the following, in any order: protein A chromatography, anion exchange chromatography (e.g., Q sepharose), hydrophobic interaction chromatography (e.g., phenylsepharose), size exclusion chromatography, and cation exchange chromatography.

In certain aspects, an ActRIIb antagonist disclosed herein, such as an ActRIIb polypeptide, may be used in a method for promoting bone growth or increasing bone density in a subject. In certain instances, the disclosure provides methods for treating a disorder associated with low bone density, or to promote bone growth, in patients in need thereof. A method may comprise administering to a subject in need thereof an effective amount of ActRIIb antagonist. In certain aspects, the disclosure provides uses of ActRIIb antagonist for making a medicament for the treatment of a disorder or condition as described herein.

In certain aspects, the disclosure provides a method for identifying an agent that stimulates growth of, or increased mineralization of, bone. The method comprises: a) identifying a test agent that binds to a ligand-binding domain of an ActRIIb polypeptide; and b) evaluating the effect of the agent on growth of, or mineralization of, bone.

In certain aspects, the disclosure provides methods for dosing a patient with an ActRIIb-Fc fusion protein, comprising administering an ActRIIb-Fc fusion protein to the patient on a dosing schedule that maintains a serum concentration of the ActRIIb-Fc fusion protein of at least 8 µg/mL.

In certain aspects, the disclosure provides a compound for use in dosing a patient with an ActRIIb-Fc fusion protein, wherein the ActRIIb-Fc fusion protein is dosed on a schedule that maintains a serum concentration of the ActRIIb-Fc fusion protein of at least 8 µg/mL. Other exemplary compounds, uses and dosing regimens are described herein and in the claims.

### BRIEF DESCRIPTION OF THE DRAWINGS

The patent or application file contains at least one drawing executed in color. Copies of this patent or patent application publication with color drawing(s) will be provided by the Office upon request any payment of the necessary fee.
Figure 1 shows a multiple sequence alignment of various vertebrate ActRIIb proteins and human ActRIIA.
Figure 2 shows a pharmacokinetic (PK) profile in human patients for a single dose of ActRIIb-Fc administered subcutaneously.
Figure 3 shows the mean percent change in lean body mass at 15, 29 or 57 days after subcutaneous administration to human patients of a single dose of ActRIIb-Fc.
Figure 4 shows the percent of subjects with ≥ 0.5 kg (top panel) or ≥ 1.0 kg (bottom panel) increase in lean body mass at 15, 29 or 57 days after subcutaneous administration to human patients of a single dose of ActRIIb-Fc.
Figure 5 shows the change in leptin, a serum biomarker of fat metabolism, at various time points after subcutaneous administration to human patients of a single dose of ActRIIb-Fc.
Figure 6 shows the change in adiponectin, a serum biomarker of fat metabolism, at various time points after subcutaneous administration to human patients of a single dose of ActRIIb-Fc.
Figure 7 shows the mean percent change in bone-specific alkaline phosphatase (BSAP), a serum biomarker of bone formation, at various time points after subcutaneous administration to human patients of a single dose of ActRIIb-Fc.
Figure 8 shows the mean percent change in C-terminal type 1 collagen telopeptide (CTX), a serum biomarker of bone resoprtion, at various time points after subcutaneous administration to human patients of a single dose of ActRIIb-Fc.
Figure 9 shows the mean percent change in serum follicle-stimulating hormone (FSH) at various time points after subcutaneous administration to human patients of a single dose of ActRIIb-Fc.
Figure 10 shows a magnetic resonance imaging (MRI) scan of a thigh cross-section in a representative human subject dosed with a single subcutaneous administration of 3 mg/kg of ActRIIb-Fc. The top panel shows an MRI evaluation of a thigh, the middle panel shows the baseline at day 9, the bottom panel shows the results at day 29 after dosing. Muscle is shown in gray, subcutaneous fat is pink, and intramuscular fat is green.
Figure 11 shows the mean percent change from baseline in thigh muscle volume at day 29 for human subjects dosed with a single subcutaneous administration of 1 mg/kg ActRIIb-Fc or 3 mg/kg ActRIIb-Fc as compared to placebo.
Figure 12 shows an alignment of the signal sequence and extracellular domain of ActRIIb from SEQ ID NO: 1 (residues 1-134 of SEQ IDNO: 1) with the extracellular domain of ActRIIb as represented by SEQ ID NO: 2. As shown, the signal sequence is 19 amino acids in length. Therefore, an amino acid at position X in SEQ ID NO: 1 has the corresponding position at X-19 in SEQ ID NO: 2. To illustrate, amino acid position 30 in SEQ ID NO: 1 is amino acid position 11 in SEQ ID NO: 2. A similar correlation may be determined for other ActRIIb sequences described herein.
Figure 13 shows an alignment of human ActRIIa and ActRIIb. Boxed residues represent amino acid residues believed to directly contact ligand (i.e., the ligand binding pocket) based on composite analysis of multiple ActRIIb and ActRIIa crystal structures.
Figure 14 shows the pharmacokinetic profile of ActRIIb-hFc in the serum at various days during the multiple ascending dose study described in Example 5.
Figure 15 shows serum concentrations of ActRIIb-hFc on the upper axis and the percentage change in follicle stimulating hormone (FSH) on the lower axis.
Figure 16 shows the percent change in total body lean mass caused by varying doses of ActRIIb-hFc, as measured by DXA.

### DETAILED DESCRIPTION OF THE INVENTION

### 1. Overview

The transforming growth factor-beta (TGF-beta) superfamily contains a variety of growth factors that share common sequence elements and structural motifs. These proteins are known to exert biological effects on a large variety of cell types in both vertebrates and invertebrates. Members of the superfamily perform important functions during embryonic development in pattern formation and tissue specification and can influence a variety of differentiation processes, including adipogenesis, myogenesis, chondrogenesis, cardiogenesis, hematopoiesis, neurogenesis, and epithelial cell differentiation. By manipulating the activity of a member of the TGF-beta family, it is often possible to cause significant physiological changes in an organism. For example, the Piedmontese and Belgian Blue cattle breeds carry a loss-of-function mutation in the GDF8 (also called myostatin) gene that causes a marked increase in muscle mass. Grobet et al., Nat Genet. 1997, 17(1):71-4. Furthermore, in humans, inactive alleles of GDF8 are associated with increased muscle mass and, reportedly, exceptional strength. Schuelke et al., N Engl J Med 2004, 350:2682-8.

Activins are dimeric polypeptide growth factors that belong to the TGF-beta superfamily. There are three principle activin forms (A, B, and AB) that are homo/heterodimers of two closely related β subunits (β_{A}β_{A}, β_{B}β_{B}, and β_{A}β_{B}). The human genome also encodes an activin C and an activin E, which are primarily expressed in the liver. In the TGF-beta superfamily, activins are unique and multifunctional factors that can stimulate hormone production in ovarian and placental cells, support neuronal cell survival, influence cell-cycle progress positively or negatively depending on cell type, and induce mesodermal differentiation at least in amphibian embryos (DePaolo et al., 1991, Proc Soc Ep Biol Med. 198:500-512; Dyson et al., 1997, Curr Biol. 7:81-84; Woodruff, 1998, Biochem Pharmacol. 55:953-963). Moreover, erythroid differentiation factor (EDF) isolated from the stimulated human monocytic leukemic cells was found to be identical to activin A (Murata et al., 1988, PNAS, 85:2434). It has been suggested that activin A acts as a natural, positive regulator of erythropoiesis in the bone marrow. In several tissues, activin signaling is antagonized by its related heterodimer, inhibin. For example, during the release of follicle-stimulating hormone (FSH) from the pituitary, activin promotes FSH secretion and synthesis, while inhibin prevents FSH secretion and synthesis. Other proteins that may regulate activin bioactivity and/or bind to activin include follistatin (FS), follistatin-related protein (FSRP), and α₂-macroglobulin.

TGF-β family signals are mediated by heteromeric complexes of type I and type II serine/ threonine kinase receptors, which phosphorylate and activate downstream Smad proteins upon ligand stimulation (Massagué, 2000, Nat. Rev. Mol. Cell Biol. 1:169-178). These type I and type II receptors are transmembrane proteins, composed of a ligand-binding extracellular domain with cysteine-rich region, a transmembrane domain, and a cytoplasmic domain with predicted serine/threonine specificity. Type I receptors are essential for signaling; and type II receptors are required for binding ligands and for expression of type I receptors. Type I and II activin receptors form a stable complex after ligand binding, resulting in phosphorylation of type I receptors by type II receptors.

Two related type II receptors, ActRIIa and ActRIIb, have been identified as the type II receptors for activins (Mathews and Vale, 1991, Cell 65:973-982; Attisano et al., 1992, Cell 68: 97-108). Besides activins, ActRIIa and ActRIIb can biochemically interact with several other TGF-β family proteins, including BMP7, Nodal, GDF8, and GDF11 (Yamashita et al., 1995, J. Cell Biol. 130:217-226; Lee and McPherron, 2001, Proc. Natl. Acad. Sci. 98:9306-9311; Yeo and Whitman, 2001, Mol. Cell 7: 949-957; Oh et al., 2002, Genes Dev. 16:2749-54). ALK4 is the primary type I receptor for activins, particularly for activin A, and ALK-7 may serve as a receptor for activins as well, particularly for activin B.

As demonstrated herein, an ActRIIb polypeptide (ActRIIb[20-134]-Fc) is effective to promote bone growth and increase bone density in vivo, while also increasing muscle mass. While not wishing to be bound to any particular mechanism, it is expected that the effect of ActRIIb on bone is caused primarily by an activin antagonist effect. Regardless of mechanism, it is apparent from the data presented herein that ActRIIb antagonists increase bone density, and affect bone biomarkers in manner that is consistent with a combine anabolic/anti-resorptive effect in humans. It should be noted that bone is a dynamic tissue, with growth or shrinkage and increased or decreased density depending on a balance of factors that produce bone and stimulate mineralization (primarily osteoblasts) and factors that destroy and demineralize bone (primarily osteoclasts). Bone growth and mineralization may be increased by increasing the productive factors, by decreasing the destructive factors, or both. The terms "promote bone growth" and "increase bone mineralization" refer to the observable physical changes in bone and are intended to be neutral as to the mechanism by which changes in bone occur.

In addition to promoting bone growth, the disclosure contemplates using ActRIIb antagonists in treating or preventing diseases or conditions that are associated with abnormal activity of an ActRIIb or an ActRIIb ligand. For example, ActRIIb antagonists may be used to treat human or animal disorders or conditions. Examples of such disorders or conditions include, but are not limited to, metabolic disorders such as type 2 diabetes, impaired glucose tolerance, metabolic syndrome (e.g., syndrome X), and insulin resistance induced by trauma (e.g., burns or nitrogen imbalance); adipose tissue disorders (e.g., obesity); muscle and neuromuscular disorders such as muscular dystrophy (including Duchenne muscular dystrophy); amyotrophic lateral sclerosis (ALS); muscle atrophy; organ atrophy; frailty; carpal tunnel syndrome; congestive obstructive pulmonary disease; and sarcopenia, cachexia and other muscle wasting syndromes. Other examples include osteoporosis, especially in the elderly and/or postmenopausal women; glucocorticoid-induced osteoporosis; osteopenia; osteoarthritis; and osteoporosis-related fractures. Yet further examples include low bone mass due to chronic glucocorticoid therapy, premature gonadal failure, androgen suppression, vitamin D deficiency, secondary hyperparathyroidism, nutritional deficiencies, and anorexia nervosa.

This disclosure provides methods for using ActRIIb polypeptides and other ActRIIb antagonists to promote bone growth and increase bone density in humans, and also to increase muscle mass. ActRIIb antagonists include, for example, ligand-binding ActRIIb polypeptides (e.g., ActRIIb-Fc), antibodies that bind to ActRIIb and disrupt binding of one or more ligands, such as activin or myostatin, non-antibody proteins selected for ActRIIb binding (see e.g., WO/2002/088171, WO/2006/055689, and WO/2002/032925 for examples of such proteins and methods for design and selection of same), randomized peptides selected for ActRIIb binding, often affixed to an Fc domain. Two different proteins (or other moieties) with ActRIIb binding activity, especially activin binders that block the type I (e.g., a soluble type I activin receptor) and type II (e.g., a soluble type II activin receptor) binding sites, respectively, may be linked together to create a bifunctional binding molecule. Nucleic acid aptamers, small molecules and other agents that inhibit the ActRIIb signaling axis are included as ActRIIb antagonists.

The terms used in this specification generally have their ordinary meanings in the art, within the context of this invention and in the specific context where each term is used. Certain terms are discussed below or elsewhere in the specification, to provide additional guidance to the practitioner in describing the compositions of the invention and how to make and use them. The scope or meaning of any use of a term will be apparent from the specific context in which the term is used.

"About" and "approximately" shall generally mean an acceptable degree of error for the quantity measured given the nature or precision of the measurements. Typically, exemplary degrees of error are within 20 percent (%), preferably within 10%, and more preferably within 5% of a given value or range of values.

Alternatively, and particularly in biological systems, the terms "about" and "approximately" may mean values that are within an order of magnitude, preferably within 5-fold and more preferably within 2-fold of a given value. Numerical quantities given herein are approximate unless stated otherwise, meaning that the term "about" or "approximately" can be inferred when not expressly stated.

The methods of the disclosure may include steps of comparing sequences to each other, including wild-type sequence to one or more mutants (sequence variants). Such comparisons typically comprise alignments of polymer sequences, e.g., using sequence alignment programs and/or algorithms that are well known in the art (for example, BLAST, FASTA and MEGALIGN, to name a few). The skilled artisan can readily appreciate that, in such alignments, where a mutation contains a residue insertion or deletion, the sequence alignment will introduce a "gap" (typically represented by a dash, or "A") in the polymer sequence not containing the inserted or deleted residue.

"Homologous," in all its grammatical forms and spelling variations, refers to the relationship between two proteins that possess a "common evolutionary origin," including proteins from superfamilies in the same species of organism, as well as homologous proteins from different species of organism. Such proteins (and their encoding nucleic acids) have sequence homology, as reflected by their sequence similarity, whether in terms of percent identity or by the presence of specific residues or motifs and conserved positions.

The term "sequence similarity," in all its grammatical forms, refers to the degree of identity or correspondence between nucleic acid or amino acid sequences that may or may not share a common evolutionary origin.

However, in common usage and in the instant application, the term "homologous," when modified with an adverb such as "highly," may refer to sequence similarity and may or may not relate to a common evolutionary origin.

### 2. ActRIIb Polypeptides

The present invention relates to ActRIIb polypeptides. As used herein, the term "ActRIIb" refers to a family of activin receptor type IIb (ActRIIb) proteins from any species and variants derived from such ActRIIb proteins by mutagenesis or other modification. Reference to ActRIIb herein is understood to be a reference to any one of the currently identified forms. Members of the ActRIIb family are generally transmembrane proteins, composed of a ligand-binding extracellular domain with a cysteine-rich region, a transmembrane domain, and a cytoplasmic domain with predicted serine/threonine kinase activity.

The term "ActRIIb polypeptide" includes polypeptides comprising any naturally occurring polypeptide of an ActRIIb family member as well as any variants thereof (including mutants, fragments, fusions, and peptidomimetic forms) that retain a useful activity. See, for example, WO/2006/012627.

For example, ActRIIb polypeptides include polypeptides derived from the sequence of any known ActRIIb having a sequence at least about 80% identical to the sequence of an ActRIIb polypeptide, and optionally at least 85%, 90%, 95%, 97%, 99% or greater identity. For example, an ActRIIb polypeptide of the invention may bind to and inhibit the function of an ActRIIb protein. An ActRIIb polypeptide may be selected for activity in promoting red blood cell formation in vivo. Examples of ActRIIb polypeptides include human ActRIIb precursor polypeptide (SEQ ID NO: 1) and soluble human ActRIIb polypeptides (e.g., SEQ ID NO: 2, 3, 13, 17, or 20).

The human ActRIIb precursor protein sequence is as follows:

The signal peptide is single underlined; the extracellular domain is in bold and the potential N-linked glycosylation sites are in boxes.

The human ActRIIb soluble (extracellular), processed polypeptide sequence is as follows:

In some conditions, the protein may be produced with an "SGR..." sequence at the N-terminus. The C-terminal "tail" of the extracellular domain is underlined. The sequence with the "tail" deleted (a Δ15 sequence) is as follows:

In some conditions, the protein may be produced with an "SGR..." sequence at the N-terminus. The nucleic acid sequence encoding a human ActRIIb precursor protein is as follows: (nucleotides 5-1543 of Genbank entry NM_001106)

The nucleic acid sequence encoding a human ActRIIb soluble (extracellular) polypeptide is as follows:

The invention relates to soluble ActRIIb polypeptides. As described herein, the term "soluble ActRIIb polypeptide" generally refers to polypeptides comprising an extracellular domain of an ActRIIb protein. The term "soluble ActRIIb polypeptide," as used herein, includes any naturally occurring extracellular domain of an ActRIIb protein as well as any variants thereof (including mutants, fragments and peptidomimetic forms). An activin-binding ActRIIb polypeptide is one that retains the ability to bind to activin, including, for example, activin AA, AB, BB, or forms that include a C or E subunit. Optionally, an activin-binding ActRIIb polypeptide will bind to activin AA with a dissociation constant of 1 nM or less. The extracellular domain of an ActRIIb protein binds to activin and is generally soluble in physiological conditions, and thus can be termed a soluble, activin-binding ActRIIb polypeptide. Examples of soluble, activin-binding ActRIIb polypeptides include the soluble polypeptides illustrated in SEQ ID NOs: 2, 3, 13, 17, or 20. SEQ ID NO: 13 is referred to as ActRIIb(20-134)-hFc, and is described further in the Examples. Other examples of soluble ActRIIb polypeptides comprise a signal sequence in addition to the extracellular domain of an ActRIIb protein, for example, the honey bee mellitin leader sequence (SEQ ID NO: 14), the tissue plaminogen activator (TPA) leader (SEQ ID NO: 15) or the native ActRIIb leader (SEQ ID NO: 16). The ActRIIb-hFc polypeptide illustrated in SEQ ID NO: 17 uses a TPA leader.

In certain embodiments, the invention relates to ActRIIb variant polypeptides (e.g., soluble ActRIIb polypeptides). Optionally, the fragments, functional variants, and modified forms have similar or the same biological activities of their corresponding wild-type ActRIIb polypeptides. For example, an ActRIIb variant of the invention may bind to and inhibit function of an ActRIIb ligand (e.g., activin A, activin AB, activin B, Nodal, GDF8, GDF11 or BMP7). Optionally, an ActRIIb polypeptide modulates growth of tissues such as bone, cartilage, muscle or fat. Examples of ActRIIb polypeptides include human ActRIIb precursor polypeptide (SEQ ID NO: 1), and soluble human ActRIIb polypeptides (e.g., SEQ ID NOs: 2, 3, 13, 17, or 20).

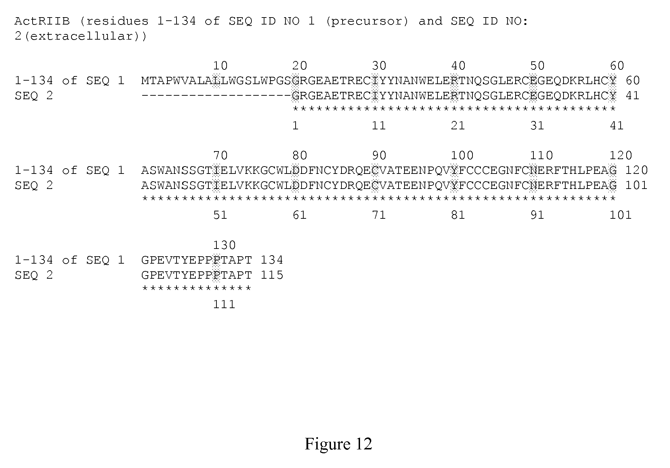

Unless otherwise stated, amino acid positions in an ActRIIb polypeptide discussed herein are made with reference to the precursor ActRIIb polypeptide (i.e., SEQ ID NO: 1). It should be understood that the corresponding position in another ActRIIb polypeptide may be readily identified based on the information provided herein. For example, the precursor ActRIIb polypeptide contains a 19 amino acid leader sequence that is not contained in the soluble extracellular portion of ActRIIb as shown in SEQ ID NO: 2. Accordingly, a residue at position X in SEQ ID NO: 1 corresponds to residue X-19 in SEQ ID NO: 2. For example, residue 30 of SEQ ID NO: 1 corresponds to residue 11 in SEQ ID NO: 2 (see Figure 12). A similar correlation may be determined for other ActRIIb sequences described herein.

The disclosure identifies functionally active portions and variants of ActRIIb. Applicants have ascertained that an Fc fusion protein having the sequence disclosed by Hilden et al. (Blood. 1994 Apr 15;83(8):2163-70), which has an Alanine at the position corresponding to amino acid 64 of SEQ ID NO: 1 (A64), has a relatively low affinity for activin and GDF-11. By contrast, the same Fc fusion protein with an Arginine at position 64 (R64) has an affinity for activin and GDF-11 in the low nanomolar to high picomolar range. Therefore, a sequence with an R64 is used as the wild-type reference sequence for human ActRIIb in this disclosure.

Attisano et al. (Cell. 1992 Jan 10;68(1):97-108) showed that a deletion of the proline knot at the C-terminus of the extracellular domain of ActRIIb reduced the affinity of the receptor for activin. An ActRIIb-Fc fusion protein containing amino acids 20-119 of SEQ ID NO: 1, "ActRIIb(20-119)-Fc" has reduced binding to GDF-11 and activin relative to an ActRIIb(20-134)-Fc, which includes the proline knot region and the complete juxtamembrane domain (see U.S. Publication No. 2009/0005308). However, an ActRIIb(20-129)-Fc protein retains similar but somewhat reduced activity relative to the wild type, even though the proline knot region is disrupted. Thus, ActRIIb extracellular domains that stop at amino acid 134, 133, 132, 131, 130 and 129 are all expected to be active, but constructs stopping at 134 or 133 may be most active. Similarly, mutations at any of residues 129-134 are not expected to alter ligand binding affinity by large margins. In support of this, mutations of P129 and P130 do not substantially decrease ligand binding. Therefore, an ActRIIb-Fc fusion protein may end as early as amino acid 109 (the final cysteine), however, forms ending at or between 109 and 119 are expected to have reduced ligand binding. Amino acid 119 is poorly conserved and so is readily altered or truncated. Forms ending at 128 or later retain ligand binding activity. Forms ending at or between 119 and 127 will have an intermediate binding ability. It has been reported that an ActRIIb(25-118)-hFc is effective for inhibiting activin and other ligands and for promoting muscle mass, and accordingly, such truncated ActRIIb-hFc proteins may be used in the methods described herein (see Zhou et al., 2010, Cell 142:531-543). Any of these forms may be desirable to use, depending on the clinical or experimental setting.

At the N-terminus of ActRIIb, it is expected that a protein beginning at amino acid 29 or before will retain ligand binding activity. Amino acid 29 represents the initial cysteine. An alanine to asparagine mutation at position 24 introduces an N-linked glycosylation sequence without substantially affecting ligand binding. This confirms that mutations in the region between the signal cleavage peptide and the cysteine cross-linked region, corresponding to amino acids 20-29 are well tolerated. In particular, constructs beginning at position 20, 21, 22, 23 and 24 will retain activity, and constructs beginning at positions 25, 26, 27, 28 and 29 are also expected to retain activity. A construct beginning at 22, 23, 24 or 25 will have the most activity (see U.S. Publication No. 2009/0005308).

Taken together, an active portion of ActRIIb comprises amino acids 29-109 of SEQ ID NO: 1, and constructs may, for example, begin at a residue corresponding to amino acids 20-29 and end at a position corresponding to amino acids 109-134. Other examples include constructs that begin at a position from 20-29 or 21-29 and end at a position from 118-134, 118-133, 119-134, 119-133 or 129-134, 129-133. Other examples include constructs that begin at a position from 20-24 (or 21-24, or 22-25) and end at a position from 109-134 (or 109-133), 118-134 (or 118-133), 119-134 (or 119-133) or 129-134 (or 129-133). Variants within these ranges are also contemplated, particularly those having at least 80%, 85%, 90%, 95% or 99% identity to the corresponding portion of SEQ ID NO: 2.

Extensive analysis of structure function analysis of ActRIIb is provided in U.S. Publication No. 2009/0005308. ActRIIb residues likely to be in contact with ligands in the binding pocket have been defined as residues Y31, N33, N35, L38 through T41, E47, E50, Q53 through K55, L57, H58, Y60, S62, K74, W78 through N83, Y85, R87, A92, and E94 through F101 of SEQ ID NO: 1. At these positions, it is expected that conservative mutations will be tolerated, although a K74A mutation is well-tolerated, as are R40A, K55A, F82A and mutations at position L79. R40 is a K in Xenopus, indicating that basic amino acids at this position will be tolerated. Q53 is R in bovine ActRIIb and K in Xenopus ActRIIb, and therefore amino acids including R, K, Q, N and H will be tolerated at this position. Outside of these residues, it is expected that modifications will be relatively well-tolerated, provided that such alterations do not disrupt the structure of the protein as a whole. It is readily apparent when a protein structure is disrupted because the protein will tend to express poorly or be degraded in the culture media. Thus, a general formula for an active ActRIIb variant protein is one that comprises amino acids 29-109, but optionally beginning at a position ranging from 20-24 or 22-25 and ending at a position ranging from 129-134, and comprising no more than 1, 2, 5, 10 or 15 conservative amino acid changes in the ligand binding pocket, and zero, one or more non-conservative alterations at positions 40, 53, 55, 74, 79 and/or 82 in the ligand binding pocket. Such a protein may retain greater than 80%, 90%, 95% or 99% sequence identity to the sequence of amino acids 29-109 of SEQ ID NO: 1. Sites outside the binding pocket, at which variability may be particularly well tolerated, include the amino and carboxy termini of the extracellular domain (as noted above), and positions 42-46 and 65-73 of SEQ ID NO: 1. An asparagine to alanine alteration at position 65 (N65A) actually improves ligand binding in the A64 background, and is thus expected to have no detrimental effect on ligand binding in the R64 background. This change probably eliminates glycosylation at N65 in the A64 background, thus demonstrating that a significant change in this region is likely to be tolerated. While an R64A change is poorly tolerated, R64K is well-tolerated, and thus another basic residue, such as H may be tolerated at position 64.

ActRIIb is well-conserved across nearly all vertebrates, with large stretches of the extracellular domain conserved completely. Many of the ligands that bind to ActRIIb are also highly conserved. Accordingly, comparisons of ActRIIb sequences from various vertebrate organisms provide insights into residues that may be altered. Therefore, an active, human ActRIIb variant may include one or more amino acids at corresponding positions from the sequence of another vertebrate ActRIIb, or may include a residue that is similar to that in the human or other vertebrate sequence. The following examples illustrate this approach to defining an active ActRIIb variant. L46 of human ActRIIb is a valine in Xenopus ActRIIb, and so this position may be altered, and optionally may be altered to another hydrophobic residue, such as V, I or F, or a non-polar residue such as A. E52 of human ActRIIb is a K in Xenopus ActRIIb, indicating that this site may be tolerant of a wide variety of changes, including polar residues, such as E, D, K, R, H, S, T, P, G, Y and probably A. T93 of human ActRIIb is a K in Xenopus ActRIIb, indicating that a wide structural variation is tolerated at this position, with polar residues favored, such as S, K, R, E, D, H, G, P, G and Y. F108 of human ActRIIb is a Y in Xenopus ActRIIb, and therefore Y or other hydrophobic group, such as I, V or L should be tolerated. E111 of human ActRIIb is K in Xenopus ActRIIb, indicating that charged residues will be tolerated at this position, including D, R, K and H, as well as Q and N. R112 of human ActRIIb is K in Xenopus ActRIIb, indicating that basic residues are tolerated at this position, including R and H. A at position 119 of human ActRIIb is relatively poorly conserved, and appears as P in rodent ActRIIb and V in Xenopus ActRIIb, thus essentially any amino acid should be tolerated at this position.

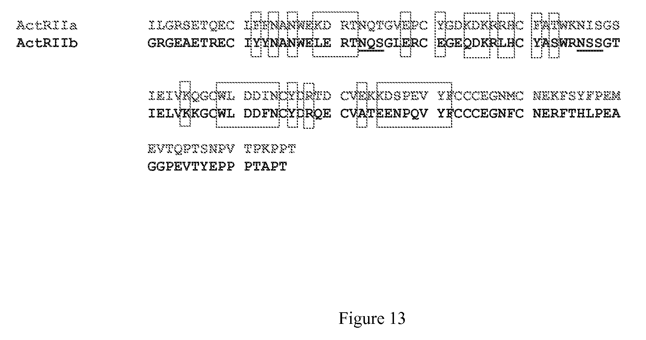

The addition of a further N-linked glycosylation site (N-X-S/T) increases the serum half-life of an ActRIIb-Fc fusion protein, relative to the ActRIIb(R64)-Fc form (see U.S. Publication No. 2009/0005308). By introducing an asparagine at position 24 (A24N construct), an NXT sequence is created that confers a longer half-life. Other NX(T/S) sequences are found at 42-44 (NQS) and 65-67 (NSS) of SEQ ID NO: 1, although the latter may not be efficiently glycosylated with the R at position 64. N-X-S/T sequences may be generally introduced at positions outside the ligand binding pocket defined in Figure 13. Particularly suitable sites for the introduction of non-endogenous N-X-S/T sequences include amino acids 20-29, 20-24, 22-25, 109-134, 120-134 or 129-134 of SEQ ID NO: 1. N-X-S/T sequences may also be introduced into the linker between the ActRIIb sequence and the Fc or other fusion component. Such a site may be introduced with minimal effort by introducing an N in the correct position with respect to a pre-existing S or T, or by introducing an S or T at a position corresponding to a pre-existing N. Thus, desirable alterations that would create an N-linked glycosylation site are: A24N, R64N, S67N (possibly combined with an N65A alteration), E106N, R112N, G120N, E123N, P129N, A132N, R112S and R112T. Any S that is predicted to be glycosylated may be altered to a T without creating an immunogenic site, because of the protection afforded by the glycosylation. Likewise, any T that is predicted to be glycosylated may be altered to an S. Thus the alterations S67T and S44T are contemplated. Likewise, in an A24N variant, an S26T alteration may be used. Accordingly, an ActRIIb variant may include one or more additional, non-endogenous N-linked glycosylation consensus sequences.

Position L79 may be altered to confer altered activin - myostatin (GDF-11) binding properties. L79A or L79P reduces GDF-11 binding to a greater extent than activin binding. L79E or L79D retains GDF-11 binding. Remarkably, the L79E and L79D variants have greatly reduced activin binding. In vivo experiments indicate that these non-activin receptors retain significant ability to increase muscle mass but show decreased effects on other tissues. These data demonstrate the desirability and feasibility for obtaining polypeptides with reduced effects on activin.

The variations described may be combined in various ways. Additionally, the results of mutagenesis program described herein indicate that there are amino acid positions in ActRIIb that are often beneficial to conserve. These include position 64 (basic amino acid), position 80 (acidic or hydrophobic amino acid), position 78 (hydrophobic, and particularly tryptophan), position 37 (acidic, and particularly aspartic or glutamic acid), position 56 (basic amino acid), position 60 (hydrophobic amino acid, particularly phenylalanine or tyrosine). Thus, in each of the variants disclosed herein, the disclosure provides a framework of amino acids that may be conserved. Other positions that may be desirable to conserve are as follows: position 52 (acidic amino acid), position 55 (basic amino acid), position 81 (acidic), 98 (polar or charged, particularly E, D, R or K).

In certain embodiments, isolated fragments of the ActRIIb polypeptides can be obtained by screening polypeptides recombinantly produced from the corresponding fragment of the nucleic acid encoding an ActRIIb polypeptide (e.g., SEQ ID NOs: 4 and 5). In addition, fragments can be chemically synthesized using techniques known in the art such as conventional Merrifield solid phase f-Moc or t-Boc chemistry. The fragments can be produced (recombinantly or by chemical synthesis) and tested to identify those peptidyl fragments that can function, for example, as antagonists (inhibitors) or agonists (activators) of an ActRIIb protein or an ActRIIb ligand.

In certain embodiments, a functional variant of the ActRIIb polypeptides has an amino acid sequence that is at least 75% identical to an amino acid sequence selected from SEQ ID NOs: 2, 3, 13, 17, or 20. In certain cases, the functional variant has an amino acid sequence at least 80%, 85%, 90%, 95%, 97%, 98%, 99% or 100% identical to an amino acid sequence selected from SEQ ID NOs: 2, 3, 13, 17, or 20.

In certain instances, the present disclosure contemplates making functional variants by modifying the structure of an ActRIIb polypeptide for such purposes as enhancing therapeutic efficacy, or stability (e.g., ex vivo shelf life and resistance to proteolytic degradation in vivo). Modified ActRIIb polypeptides can also be produced, for instance, by amino acid substitution, deletion, or addition. For instance, it is reasonable to expect that an isolated replacement of a leucine with an isoleucine or valine, an aspartate with a glutamate, a threonine with a serine, or a similar replacement of an amino acid with a structurally related amino acid (e.g., conservative mutations) will not have a major effect on the biological activity of the resulting molecule. Conservative replacements are those that take place within a family of amino acids that are related in their side chains. Whether a change in the amino acid sequence of an ActRIIb polypeptide results in a functional homolog can be readily determined by assessing the ability of the variant ActRIIb polypeptide to produce a response in cells in a fashion similar to the wild-type ActRIIb polypeptide, or to bind to one or more ligands, such as activin, GDF-11 or myostatin in a fashion similar to wild type.

In certain specific instances, the present disclosure contemplates making mutations in the extracellular domain (also referred to as ligand-binding domain) of an ActRIIb polypeptide such that the variant (or mutant) ActRIIb polypeptide has altered ligand-binding activities (e.g., binding affinity or binding specificity). In certain cases, such variant ActRIIb polypeptides have altered (elevated or reduced) binding affinity for a specific ligand. In other cases, the variant ActRIIb polypeptides have altered binding specificity for their ligands.

For example, the disclosure provides variant ActRIIb polypeptides that preferentially bind to GDFB/GDF11 relative to activins. The disclosure further establishes the desirability of such polypeptides for reducing off-target effects, although such selective variants may be less desirable for the treatment of severe diseases where very large gains in muscle mass may be needed for therapeutic effect and where some level of off-target effect is acceptable. For example, amino acid residues of the ActRIIb protein, such as E39, K55, Y60, K74, W78, D80, and F101, are in the ligand-binding pocket and mediate binding to its ligands such as activin and GDF8. Thus, the present invention provides an altered ligand-binding domain (e.g., GDF8-binding domain) of an ActRIIb receptor, which comprises one or more mutations at those amino acid residues. Optionally, the altered ligand-binding domain can have increased selectivity for a ligand such as GDF8 relative to a wild-type ligand-binding domain of an ActRIIb receptor. To illustrate, these mutations increase the selectivity of the altered ligand-binding domain for GDF8 over activin. Optionally, the altered ligand-binding domain has a ratio of K_{d} for activin binding to K_{d} for GDF8 binding that is at least 2, 5, 10, or even 100 fold greater relative to the ratio for the wild-type ligand-binding domain. Optionally, the altered ligand-binding domain has a ratio of IC₅₀ for inhibiting activin to IC₅₀ for inhibiting GDF8 that is at least 2, 5, 10, or even 100 fold greater relative to the wild-type ligand-binding domain. Optionally, the altered ligand-binding domain inhibits GDF8 with an IC₅₀ at least 2, 5, 10, or even 100 times less than the IC₅₀ for inhibiting activin.

As a specific example, the positively-charged amino acid residue Asp (D80) of the ligand-binding domain of ActRIIb can be mutated to a different amino acid residue such that the variant ActRIIb polypeptide preferentially binds to GDF8, but not activin. Preferably, the D80 residue is changed to an amino acid residue selected from the group consisting of: an uncharged amino acid residue, a negative amino acid residue, and a hydrophobic amino acid residue. As a further specific example, the hydrophobic residue, L79, can be altered to the acidic amino acids aspartic acid or glutamic acid to greatly reduce activin binding while retaining GDF11 binding. As will be recognized by one of skill in the art, most of the described mutations, variants or modifications may be made at the nucleic acid level or, in some cases, by post translational modification or chemical synthesis. Such techniques are well known in the art.

In certain embodiments, the present invention contemplates specific mutations of the ActRIIb polypeptides of the invention so as to alter the glycosylation of the polypeptide. Exemplary glycosylation sites in ActRIIb polypeptides are shown in SEQ ID NO: 1. Such mutations may be selected so as to introduce or eliminate one or more glycosylation sites, such as O-linked or N-linked glycosylation sites. Asparagine-linked glycosylation recognition sites generally comprise a tripeptide sequence, asparagine-X-threonine (where "X" is any amino acid) which is specifically recognized by appropriate cellular glycosylation enzymes. The alteration may also be made by the addition of, or substitution by, one or more serine or threonine residues to the sequence of the wild-type ActRIIb polypeptide (for O-linked glycosylation sites). A variety of amino acid substitutions or deletions at one or both of the first or third amino acid positions of a glycosylation recognition site (and/or amino acid deletion at the second position) results in non-glycosylation at the modified tripeptide sequence. Another means of increasing the number of carbohydrate moieties on an ActRIIb polypeptide is by chemical or enzymatic coupling of glycosides to the ActRIIb polypeptide. Depending on the coupling mode used, the sugar(s) may be attached to (a) arginine and histidine; (b) free carboxyl groups; (c) free sulfhydryl groups such as those of cysteine; (d) free hydroxyl groups such as those of serine, threonine, or hydroxyproline; (e) aromatic residues such as those of phenylalanine, tyrosine, or tryptophan; or (f) the amide group of glutamine. These methods are described in WO 87/05330 published Sep. 11, 1987, and in Aplin and Wriston (1981) CRC Crit. Rev. Biochem., pp. 259-306.

Removal of one or more carbohydrate moieties present on an ActRIIb polypeptide may be accomplished chemically and/or enzymatically. Chemical deglycosylation may involve, for example, exposure of the ActRIIb polypeptide to the compound trifluoromethanesulfonic acid, or an equivalent compound. This treatment results in the cleavage of most or all sugars except the linking sugar (N-acetylglucosamine or N-acetylgalactosamine), while leaving the amino acid sequence intact. Chemical deglycosylation is further described by Hakimuddin et al. (1987) Arch. Biochem. Biophys. 259:52 and by Edge et al. (1981) Anal. Biochem. 118:131. Enzymatic cleavage of carbohydrate moieties on ActRIIb polypeptides can be achieved by the use of a variety of endo- and exo-glycosidases as described by Thotakura et al. (1987) Meth. Enzymol. 138:350. The sequence of an ActRIIb polypeptide may be adjusted, as appropriate, depending on the type of expression system used, as mammalian, yeast, insect and plant cells may all introduce differing glycosylation patterns that can be affected by the amino acid sequence of the peptide. In general, ActRIIb proteins for use in humans will be expressed in a mammalian cell line that provides proper glycosylation, such as HEK293 or CHO cell lines, although other mammalian expression cell lines are expected to be useful as well.

This disclosure further contemplates a method of generating variants, particularly sets of combinatorial variants of an ActRIIb polypeptide, including, optionally, truncation variants; pools of combinatorial mutants are especially useful for identifying functional variant sequences. The purpose of screening such combinatorial libraries may be to generate, for example, ActRIIb polypeptide variants which have altered properties, such as altered pharmacokinetics, or altered ligand binding. A variety of screening assays are provided below, and such assays may be used to evaluate variants. For example, an ActRIIb polypeptide variant may be screened for ability to bind to an ActRIIb polypeptide, to prevent binding of an ActRIIb ligand to an ActRIIb polypeptide.

The activity of an ActRIIb polypeptide or its variants may also be tested in a cell-based or in vivo assay. For example, the effect of an ActRIIb polypeptide variant on the expression of genes involved in bone production in an osteoblast or precursor may be assessed. This may, as needed, be performed in the presence of one or more recombinant ActRIIb ligand protein (e.g., BMP7), and cells may be transfected so as to produce an ActRIIb polypeptide and/or variants thereof, and optionally, an ActRIIb ligand. Likewise, an ActRIIb polypeptide may be administered to a mouse or other animal, and one or more bone properties, such as density or volume may be assessed. The healing rate for bone fractures may also be evaluated. Similarly, the activity of an ActRIIb polypeptide or its variants may be tested in muscle cells, adipocytes, and neuronal cells for any effect on growth of these cells, for example, by the assays as described below. Such assays are well known and routine in the art. A SMAD-responsive reporter gene may be used in such cell lines to monitor effects on downstream signaling.

Combinatorially-derived variants can be generated which have a selective potency relative to a naturally occurring ActRIIb polypeptide. Such variant proteins, when expressed from recombinant DNA constructs, can be used in gene therapy protocols. Likewise, mutagenesis can give rise to variants which have intracellular half-lives dramatically different than the corresponding a wild-type ActRIIb polypeptide. For example, the altered protein can be rendered either more stable or less stable to proteolytic degradation or other processes which result in destruction of, or otherwise inactivation of a native ActRIIb polypeptide. Such variants, and the genes which encode them, can be utilized to alter ActRIIb polypeptide levels by modulating the half-life of the ActRIIb polypeptides. For instance, a short half-life can give rise to more transient biological effects and, when part of an inducible expression system, can allow tighter control of recombinant ActRIIb polypeptide levels within the cell.

In certain embodiments, the ActRIIb polypeptides of the invention may further comprise post-translational modifications in addition to any that are naturally present in the ActRIIb polypeptides. Such modifications include, but are not limited to, acetylation, carboxylation, glycosylation, phosphorylation, lipidation, and acylation. As a result, the modified ActRIIb polypeptides may contain non-amino acid elements, such as polyethylene glycols, lipids, poly- or mono-saccharide, and phosphates. Effects of such non-amino acid elements on the functionality of a ActRIIb polypeptide may be tested as described herein for other ActRIIb polypeptide variants. When an ActRIIb polypeptide is produced in cells by cleaving a nascent form of the ActRIIb polypeptide, post-translational processing may also be important for correct folding and/or function of the protein. Different cells (such as CHO, HeLa, MDCK, 293, WI38, NIH-3T3 or HEK293) have specific cellular machinery and characteristic mechanisms for such post-translational activities and may be chosen to ensure the correct modification and processing of the ActRIIb polypeptides.

In certain aspects, functional variants or modified forms of the ActRIIb polypeptides include fusion proteins having at least a portion of the ActRIIb polypeptides and one or more fusion domains. Well known examples of such fusion domains include, but are not limited to, polyhistidine, Glu-Glu, glutathione S transferase (GST), thioredoxin, protein A, protein G, an immunoglobulin heavy chain constant region (e.g., an Fc), maltose binding protein (MBP), or human serum albumin. A fusion domain may be selected so as to confer a desired property. For example, some fusion domains are particularly useful for isolation of the fusion proteins by affinity chromatography. For the purpose of affinity purification, relevant matrices for affinity chromatography, such as glutathione-, amylase-, and nickel- or cobalt-conjugated resins are used. Many of such matrices are available in "kit" form, such as the Pharmacia GST purification system and the QIAexpress™ system (Qiagen) useful with (HIS₆) fusion partners. As another example, a fusion domain may be selected so as to facilitate detection of the ActRIIb polypeptides. Examples of such detection domains include the various fluorescent proteins (e.g., GFP) as well as "epitope tags," which are usually short peptide sequences for which a specific antibody is available. Well known epitope tags for which specific monoclonal antibodies are readily available include FLAG, influenza virus haemagglutinin (HA), and c-myc tags. In some cases, the fusion domains have a protease cleavage site, such as for Factor Xa or Thrombin, which allows the relevant protease to partially digest the fusion proteins and thereby liberate the recombinant proteins therefrom. The liberated proteins can then be isolated from the fusion domain by subsequent chromatographic separation. In certain preferred instances, an ActRIIb polypeptide is fused with a domain that stabilizes the ActRIIb polypeptide in vivo (a "stabilizer" domain). By "stabilizing" is meant anything that increases serum half life, regardless of whether this is because of decreased destruction, decreased clearance by the kidney, or other pharmacokinetic effect. Fusions with the Fc portion of an immunoglobulin are known to confer desirable pharmacokinetic properties on a wide range of proteins. Likewise, fusions to human serum albumin can confer desirable properties. Other types of fusion domains that may be selected include multimerizing (e.g., dimerizing, tetramerizing) domains and functional domains (that confer an additional biological function, such as further stimulation of muscle growth).

As a specific example, the present invention provides a fusion protein of the invention as a GDF8 antagonist which comprises an extracellular (e.g., GDF8-binding) domain fused to an Fc domain (e.g., SEQ ID NO: 6).

Preferably, the Fc domain has one or more mutations at residues such as Asp-265, lysine 322, and Asn-434. In certain cases, the mutant Fc domain having one or more of these mutations (e.g., Asp-265 mutation) has reduced ability of binding to the Fcγ receptor relative to a wildtype Fc domain. In other cases, the mutant Fc domain having one or more of these mutations (e.g., Asn-434 mutation) has increased ability of binding to the MHC class I-related Fc-receptor (FcRN) relative to a wildtype Fc domain.

It is understood that different elements of the fusion proteins may be arranged in any manner that is consistent with the desired functionality. For example, an ActRIIb polypeptide may be placed C-terminal to a heterologous domain, or, alternatively, a heterologous domain may be placed C-terminal to an ActRIIb polypeptide. The ActRIIb polypeptide domain and the heterologous domain need not be adjacent in a fusion protein, and additional domains or amino acid sequences may be included C- or N-terminal to either domain or between the domains.

In certain instances, the ActRIIb polypeptides of the present disclosure contain one or more modifications that are capable of stabilizing the ActRIIb polypeptides. For example, such modifications enhance the in vitro half life of the ActRIIb polypeptides, enhance circulatory half life of the ActRIIb polypeptides or reducing proteolytic degradation of the ActRIIb polypeptides. Such stabilizing modifications include, but are not limited to, fusion proteins (including, for example, fusion proteins comprising an ActRIIb polypeptide and a stabilizer domain), modifications of a glycosylation site (including, for example, addition of a glycosylation site to an ActRIIb polypeptide), and modifications of carbohydrate moiety (including, for example, removal of carbohydrate moieties from an ActRIIb polypeptide). In the case of fusion proteins, an ActRIIb polypeptide is fused to a stabilizer domain such as an IgG molecule (e.g., an Fc domain). As used herein, the term "stabilizer domain" not only refers to a fusion domain (e.g., Fc) as in the case of fusion proteins, but also includes nonproteinaceous modifications such as a carbohydrate moiety, or nonproteinaceous polymer, such as polyethylene glycol.

The present invention makes available isolated and/or purified forms of the ActRIIb polypeptides of the invention, which are isolated from, or otherwise substantially free of, other proteins. ActRIIb polypeptides will generally be produced by expression from recombinant nucleic acids.

In certain instances, ActRIIb polypeptides (unmodified or modified) of the invention can be produced by a variety of art-known techniques. For example, such ActRIIb polypeptides can be synthesized using standard protein chemistry techniques such as those described in Bodansky, M. Principles of Peptide Synthesis, Springer Verlag, Berlin (1993) and Grant G. A. (ed.), Synthetic Peptides: A User's Guide, W. H. Freeman and Company, New York (1992). In addition, automated peptide synthesizers are commercially available (e.g., Advanced ChemTech Model 396; Milligen/Biosearch 9600). Alternatively, the ActRIIb polypeptides, fragments or variants thereof may be recombinantly produced using various expression systems (e.g., E. coli, Chinese Hamster Ovary cells, COS cells, baculovirus) as is well known in the art (also see below). In a further embodiment, the modified or unmodified ActRIIb polypeptides may be produced by digestion of naturally occurring or recombinantly produced full-length ActRIIb polypeptides by using, for example, a protease, e.g., trypsin, thermolysin, chymotrypsin, pepsin, or paired basic amino acid converting enzyme (PACE). Computer analysis (using a commercially available software, e.g., MacVector, Omega, PCGene, Molecular Simulation, Inc.) can be used to identify proteolytic cleavage sites. Alternatively, such ActRIIb polypeptides may be produced from naturally occurring or recombinantly produced full-length ActRIIb polypeptides such as standard techniques known in the art, such as by chemical cleavage (e.g., cyanogen bromide, hydroxylamine).

### 3. Nucleic Acids Encoding ActRIIb Polypeptides

In certain aspects, the disclosure provides isolated and/or recombinant nucleic acids encoding any of the ActRIIb polypeptides (e.g., full-length and soluble ActRIIb polypeptides), including fragments, functional variants and fusion proteins disclosed herein. For example, SEQ ID NO: 4 encodes the naturally occurring human ActRIIb precursor polypeptide, while SEQ ID NO: 5 encodes the processed extracellular domain of ActRIIb. The subject nucleic acids may be single-stranded or double stranded. Such nucleic acids may be DNA or RNA molecules. These nucleic acids may be used, for example, in methods for making ActRIIb polypeptides or as direct therapeutic agents (e.g., in a gene therapy approach).

In certain aspects, the subject nucleic acids encoding ActRIIb polypeptides are further understood to include nucleic acids that are variants of SEQ ID NO: 4 or 5. Variant nucleotide sequences include sequences that differ by one or more nucleotide substitutions, additions or deletions, such as allelic variants.

In certain instances, the disclosure provides isolated or recombinant nucleic acid sequences that are at least 80%, 85%, 90%, 95%, 97%, 98%, 99% or 100% identical to SEQ ID NOs: 4, 5, or 18. One of ordinary skill in the art will appreciate that nucleic acid sequences complementary to SEQ ID NOs: 4, 5, or 18 and variants of SEQ ID NOs: 4, 5, or 18 are also within the scope of this disclosure. In further instances, the nucleic acid sequences of the disclosure can be isolated, recombinant, and/or fused with a heterologous nucleotide sequence, or in a DNA library.

In other instances, nucleic acids of the disclosure also include nucleotide sequences, and the ActRIIb polypeptides encoded by such nucleic acids that hybridize under highly stringent conditions to the nucleotide sequence designated in SEQ ID NOs: 4, 5, or 18, the complement sequence of SEQ ID NOs: 4, 5, or 18, or fragments of any of the foregoing. As discussed above, one of ordinary skill in the art will understand readily that appropriate stringency conditions which promote DNA hybridization can be varied. One of ordinary skill in the art will understand readily that appropriate stringency conditions which promote DNA hybridization can be varied. For example, one could perform the hybridization at 6.0 x sodium chloride/sodium citrate (SSC) at about 45 °C, followed by a wash of 2.0 x SSC at 50 °C. For example, the salt concentration in the wash step can be selected from a low stringency of about 2.0 x SSC at 50 °C to a high stringency of about 0.2 x SSC at 50 °C. In addition, the temperature in the wash step can be increased from low stringency conditions at room temperature, about 22 °C, to high stringency conditions at about 65 °C. Both temperature and salt may be varied, or temperature or salt concentration may be held constant while the other variable is changed. In one instance, the disclosure provides nucleic acids which hybridize under low stringency conditions of 6 x SSC at room temperature followed by a wash at 2 x SSC at room temperature.

Isolated nucleic acids which differ from the nucleic acids as set forth in SEQ ID NOs: 4, 5, or 18 due to degeneracy in the genetic code are also within the scope of the disclosure. For example, a number of amino acids are designated by more than one triplet. Codons that specify the same amino acid, or synonyms (for example, CAU and CAC are synonyms for histidine) may result in "silent" mutations which do not affect the amino acid sequence of the protein. However, it is expected that DNA sequence polymorphisms that do lead to changes in the amino acid sequences of the subject proteins will exist among mammalian cells. One skilled in the art will appreciate that these variations in one or more nucleotides (up to about 3-5% of the nucleotides) of the nucleic acids encoding a particular protein may exist among individuals of a given species due to natural allelic variation. Any and all such nucleotide variations and resulting amino acid polymorphisms are within the scope of this disclosure.

In certain instances, the recombinant nucleic acids of the disclosure may be operably linked to one or more regulatory nucleotide sequences in an expression construct. Regulatory nucleotide sequences will generally be appropriate to the host cell used for expression. Numerous types of appropriate expression vectors and suitable regulatory sequences are known in the art for a variety of host cells. Typically, said one or more regulatory nucleotide sequences may include, but are not limited to, promoter sequences, leader or signal sequences, ribosomal binding sites, transcriptional start and termination sequences, translational start and termination sequences, and enhancer or activator sequences. Constitutive or inducible promoters as known in the art are contemplated by the disclosure. The promoters may be either naturally occurring promoters, or hybrid promoters that combine elements of more than one promoter. An expression construct may be present in a cell on an episome, such as a plasmid, or the expression construct may be inserted in a chromosome. In a preferred instance, the expression vector contains a selectable marker gene to allow the selection of transformed host cells. Selectable marker genes are well known in the art and will vary with the host cell used.

In certain aspects of the disclosure, the subject nucleic acid is provided in an expression vector comprising a nucleotide sequence encoding an ActRIIb polypeptide and operably linked to at least one regulatory sequence. Regulatory sequences are art-recognized and are selected to direct expression of the ActRIIb polypeptide. Accordingly, the term regulatory sequence includes promoters, enhancers, and other expression control elements. Exemplary regulatory sequences are described in Goeddel; Gene Expression Technology: Methods in Enzymology, Academic Press, San Diego, CA (1990). For instance, any of a wide variety of expression control sequences that control the expression of a DNA sequence when operatively linked to it may be used in these vectors to express DNA sequences encoding an ActRIIb polypeptide. Such useful expression control sequences, include, for example, the early and late promoters of SV40, tet promoter, adenovirus or cytomegalovirus immediate early promoter, RSV promoters, the lac system, the trp system, the TAC or TRC system, T7 promoter whose expression is directed by T7 RNA polymerase, the major operator and promoter regions of phage lambda , the control regions for fd coat protein, the promoter for 3-phosphoglycerate kinase or other glycolytic enzymes, the promoters of acid phosphatase, e.g., Pho5, the promoters of the yeast α-mating factors, the polyhedron promoter of the baculovirus system and other sequences known to control the expression of genes of prokaryotic or eukaryotic cells or their viruses, and various combinations thereof. It should be understood that the design of the expression vector may depend on such factors as the choice of the host cell to be transformed and/or the type of protein desired to be expressed. Moreover, the vector's copy number, the ability to control that copy number and the expression of any other protein encoded by the vector, such as antibiotic markers, should also be considered.

A recombinant nucleic acid of the disclosure can be produced by ligating the cloned gene, or a portion thereof, into a vector suitable for expression in either prokaryotic cells, eukaryotic cells (yeast, avian, insect or mammalian), or both. Expression vehicles for production of a recombinant ActRIIb polypeptide include plasmids and other vectors. For instance, suitable vectors include plasmids of the types: pBR322-derived plasmids, pEMBL-derived plasmids, pEX-derived plasmids, pBTac-derived plasmids and pUC-derived plasmids for expression in prokaryotic cells, such as *E. coli.*

Some mammalian expression vectors contain both prokaryotic sequences to facilitate the propagation of the vector in bacteria, and one or more eukaryotic transcription units that are expressed in eukaryotic cells. The pcDNAI/amp, pcDNAI/neo, pRc/CMV, pSV2gpt, pSV2neo, pSV2-dhfr, pTk2, pRSVneo, pMSG, pSVT7, pko-neo and pHyg derived vectors are examples of mammalian expression vectors suitable for transfection of eukaryotic cells. Some of these vectors are modified with sequences from bacterial plasmids, such as pBR322, to facilitate replication and drug resistance selection in both prokaryotic and eukaryotic cells. Alternatively, derivatives of viruses such as the bovine papilloma virus (BPV-1), or Epstein-Barr virus (pHEBo, pREP-derived and p205) can be used for transient expression of proteins in eukaryotic cells. Examples of other viral (including retroviral) expression systems can be found below in the description of gene therapy delivery systems. The various methods employed in the preparation of the plasmids and in transformation of host organisms are well known in the art. For other suitable expression systems for both prokaryotic and eukaryotic cells, as well as general recombinant procedures, see Molecular Cloning A Laboratory Manual, 3rd Ed., ed. by Sambrook, Fritsch and Maniatis (Cold Spring Harbor Laboratory Press, 2001). In some instances, it may be desirable to express the recombinant polypeptides by the use of a baculovirus expression system. Examples of such baculovirus expression systems include pVL-derived vectors (such as pVL1392, pVL1393 and pVL941), pAcUW-derived vectors (such as pAcUW1), and pBlueBac-derived vectors (such as the ß-gal containing pBlueBac III).

In a preferred instance, a vector will be designed for production of the subject ActRIIb polypeptides in CHO cells, such as a Pcmv-Script vector (Stratagene, La Jolla, Calif.), pcDNA4 vectors (Invitrogen, Carlsbad, Calif.) and pCI-neo vectors (Promega, Madison, Wisc.). As will be apparent, the subject gene constructs can be used to cause expression of the subject ActRIIb polypeptides in cells propagated in culture, e.g., to produce proteins, including fusion proteins or variant proteins, for purification.

This disclosure also pertains to a host cell transfected with a recombinant gene including a coding sequence (e.g., SEQ ID NO: 4, 5, or 18) for one or more of the subject ActRIIb polypeptides. The host cell may be any prokaryotic or eukaryotic cell. For example, an ActRIIb polypeptide of the invention may be expressed in bacterial cells such as *E. coli,* insect cells (e.g., using a baculovirus expression system), yeast, or mammalian cells. Other suitable host cells are known to those skilled in the art.

Accordingly, the present disclosure further pertains to methods of producing the subject ActRIIb polypeptides. For example, a host cell transfected with an expression vector encoding an ActRIIb polypeptide can be cultured under appropriate conditions to allow expression of the ActRIIb polypeptide to occur. The ActRIIb polypeptide may be secreted and isolated from a mixture of cells and medium containing the ActRIIb polypeptide. Alternatively, the ActRIIb polypeptide may be retained cytoplasmically or in a membrane fraction and the cells harvested, lysed and the protein isolated. A cell culture includes host cells, media and other byproducts. Suitable media for cell culture are well known in the art. The subject ActRIIb polypeptides can be isolated from cell culture medium, host cells, or both, using techniques known in the art for purifying proteins, including ion-exchange chromatography, gel filtration chromatography, ultrafiltration, electrophoresis, immunoaffinity purification with antibodies specific for particular epitopes of the ActRIIb polypeptides and affinity purification with an agent that binds to a domain fused to the ActRIIb polypeptide (e.g., a protein A column may be used to purify an ActRIIb-Fc fusion). In a preferred embodiment, the ActRIIb polypeptide of the invention is a fusion protein containing a domain which facilitates its purification. In a preferred instance, purification is achieved by a series of column chromatography steps, including, for example, three or more of the following, in any order: protein A chromatography, Q sepharose chromatography, phenylsepharose chromatography, size exclusion chromatography, and cation exchange chromatography. The purification could be completed with viral filtration and buffer exchange. Optionally, a purification scheme involves the following: a MabSelect™ protein A column (GE Healthcare) with protein in a Tris-buffered saline solution (pH 8.0) (TBS), washed in TBS (150 mM NaCl, pH 8.0) and TBS (50 mM NaCl, pH 8.0), eluted in 0.1 mM glycine at pH 3.0; the eluate may be passed over a Q sepharose column in TBS pH 8.0 and eluted in TBS containing anywhere from 200-250, 210-250, 220-250, 210-230, 215-225 or 225-235 mM NaCl; the eluate may be changed into 1.1 M NH₄SO₄, 50 mM Tris pH 8.0 and washed in the same buffer, and eluted in phosphate buffered saline at pH 7.2 or 7.4 at anywhere from 200-250, 210-250, 220-250, 210-230, 215-225 or 225-235 mM NH₄SO₄. The eluted material may be dialyzed, subjected to viral filtration and used for final formulation.

In another instance, a fusion gene coding for a purification leader sequence, such as a poly-(His)/enterokinase cleavage site sequence at the N-terminus of the desired portion of the recombinant ActRIIb polypeptide, can allow purification of the expressed fusion protein by affinity chromatography using a Ni²⁺ metal resin. The purification leader sequence can then be subsequently removed by treatment with enterokinase to provide the purified ActRIIb polypeptide (e.g., see Hochuli et al., (1987) J. Chromatography 411:177; and Janknecht et al., PNAS USA 88:8972).

Techniques for making fusion genes are well known. Essentially, the joining of various DNA fragments coding for different polypeptide sequences is performed in accordance with conventional techniques, employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another instance, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed to generate a chimeric gene sequence (see, for example, Current Protocols in Molecular Biology, eds. Ausubel et al., John Wiley & Sons: 1992).

### 4. Alternative Activin ActRIIb Antagonists

Although soluble ActRIIb polypeptides, and particularly ActRIIb-Fc fusions, are preferred antagonists, other types of ActRIIb antagonists are expected to be useful, including anti-ActRIIb antibodies, antisense, RNAi or ribozyme nucleic acids that inhibit the production of ActRIIb, and other inhibitors of ActRIIb, particularly those that disrupt ActRIIb binding.

An antibody that is specifically reactive with an ActRIIb polypeptide (e.g., a soluble ActRIIbpolypeptide) and which either binds competitively to ligand with the ActRIIb polypeptide or otherwise inhibits ActRIIb -mediated signaling may be used as an antagonist of ActRIIb polypeptide activities.

By using immunogens derived from an ActRIIb polypeptide, anti-protein/anti-peptide antisera or monoclonal antibodies can be made by standard protocols (see, for example, Antibodies: A Laboratory Manual ed. by Harlow and Lane (Cold Spring Harbor Press: 1988)). A mammal, such as a mouse, a hamster or rabbit can be immunized with an immunogenic form of the ActRIIb polypeptide, an antigenic fragment which is capable of eliciting an antibody response, or a fusion protein. Techniques for conferring immunogenicity on a protein or peptide include conjugation to carriers or other techniques well known in the art. An immunogenic portion of an ActRIIb polypeptide can be administered in the presence of adjuvant. The progress of immunization can be monitored by detection of antibody titers in plasma or serum. Standard ELISA or other immunoassays can be used with the immunogen as antigen to assess the levels of antibodies.

Following immunization of an animal with an antigenic preparation of an ActRIIb polypeptide, antisera can be obtained and, if desired, polyclonal antibodies can be isolated from the serum. To produce monoclonal antibodies, antibody-producing cells (lymphocytes) can be harvested from an immunized animal and fused by standard somatic cell fusion procedures with immortalizing cells such as myeloma cells to yield hybridoma cells. Such techniques are well known in the art, and include, for example, the hybridoma technique (originally developed by Kohler and Milstein, (1975) Nature, 256: 495-497), the human B cell hybridoma technique (Kozbar et al., (1983) Immunology Today, 4: 72), and the EBV-hybridoma technique to produce human monoclonal antibodies (Cole et al., (1985) Monoclonal Antibodies and Cancer Therapy, Alan R. Liss, Inc. pp. 77-96). Hybridoma cells can be screened immunochemically for production of antibodies specifically reactive with an ActRIIb polypeptide and monoclonal antibodies isolated from a culture comprising such hybridoma cells.

The term "antibody" as used herein is intended to include whole antibodies, e.g., of any isotype (IgG, IgA, IgM, IgE, etc.), and includes fragments or domains of immunoglobulins which are reactive with a selected antigen. Antibodies can be fragmented using conventional techniques and the fragments screened for utility and/or interaction with a specific epitope of interest. Thus, the term includes segments of proteolytically-cleaved or recombinantly-prepared portions of an antibody molecule that are capable of selectively reacting with a certain protein. Non-limiting examples of such proteolytic and/or recombinant fragments include Fab, F(ab')2, Fab', Fv, and single chain antibodies (scFv) containing a V[L] and/or V[H] domain joined by a peptide linker. The scFv's may be covalently or non-covalently linked to form antibodies having two or more binding sites. The term antibody also includes polyclonal, monoclonal, or other purified preparations of antibodies and recombinant antibodies. The term "recombinant antibody", means an antibody, or antigen binding domain of an immunoglobulin, expressed from a nucleic acid that has been constructed using the techniques of molecular biology, such as a humanized antibody or a fully human antibody developed from a single chain antibody. Single domain and single chain antibodies are also included within the term "recombinant antibody".

In certain instances, an antibody of the disclosure is a monoclonal antibody, and in certain instances, the disclosure makes available methods for generating novel antibodies. For example, a method for generating a monoclonal antibody that binds specifically to an ActRIIb polypeptide may comprise administering to a mouse an amount of an immunogenic composition comprising the antigen polypeptide effective to stimulate a detectable immune response, obtaining antibody-producing cells (e.g., cells from the spleen) from the mouse and fusing the antibody-producing cells with myeloma cells to obtain antibody-producing hybridomas, and testing the antibody-producing hybridomas to identify a hybridoma that produces a monocolonal antibody that binds specifically to the antigen. Once obtained, a hybridoma can be propagated in a cell culture, optionally in culture conditions where the hybridoma-derived cells produce the monoclonal antibody that binds specifically to the antigen. The monoclonal antibody may be purified from the cell culture.

The adjective "specifically reactive with" as used in reference to an antibody is intended to mean, as is generally understood in the art, that the antibody is sufficiently selective between the antigen of interest (e.g., an ActRIIb polypeptide) and other antigens that are not of interest that the antibody is useful for, at minimum, detecting the presence of the antigen of interest in a particular type of biological sample. In certain methods employing the antibody, such as therapeutic applications, a higher degree of specificity in binding may be desirable. Monoclonal antibodies generally have a greater tendency (as compared to polyclonal antibodies) to discriminate effectively between the desired antigens and cross-reacting polypeptides. One characteristic that influences the specificity of an antibody:antigen interaction is the affinity of the antibody for the antigen. Although the desired specificity may be reached with a range of different affinities, generally preferred antibodies will have an affinity (a dissociation constant) of about 10⁻⁶, 10⁻⁷, 10⁻⁸, 10⁻⁹ M or less.

In addition, the techniques used to screen antibodies in order to identify a desirable antibody may influence the properties of the antibody obtained. For example, if an antibody is to be used for binding an antigen in solution, it may be desirable to test solution binding. A variety of different techniques are available for testing interaction between antibodies and antigens to identify particularly desirable antibodies. Such techniques include ELISAs, surface plasmon resonance binding assays (e.g., the Biacore™ binding assay, Biacore AB, Uppsala, Sweden), sandwich assays (e.g., the paramagnetic bead system of IGEN International, Inc., Gaithersburg, Maryland), western blots, immunoprecipitation assays, and immunohistochemistry.

Examples of categories of nucleic acid compounds that are ActRIIb antagonists include antisense nucleic acids, RNAi constructs and catalytic nucleic acid constructs. A nucleic acid compound may be single or double stranded. A double stranded compound may also include regions of overhang or non-complementarity, where one or the other of the strands is single stranded. A single stranded compound may include regions of self-complementarity, meaning that the compound forms a so-called "hairpin" or "stem-loop" structure, with a region of double helical structure. A nucleic acid compound may comprise a nucleotide sequence that is complementary to a region consisting of no more than 1000, no more than 500, no more than 250, no more than 100, or no more than 50, 35, 25, 22, 20, 18 or 15 nucleotides of the full-length ActRIIb nucleic acid sequence or activin β_{A}, β_{B}, β_{C}, or β_{E} nucleic acid sequence. The region of complementarity will preferably be at least 8 nucleotides, and optionally about 18 to 35 nucleotides. A region of complementarity may fall within an intron, a coding sequence or a noncoding sequence of the target transcript, such as the coding sequence portion. Generally, a nucleic acid compound will have a length of about 8 to about 500 nucleotides or base pairs in length, and optionally the length will be about 14 to about 50 nucleotides. A nucleic acid may be a DNA (particularly for use as an antisense), RNA or RNA:DNA hybrid. Any one strand may include a mixture of DNA and RNA, as well as modified forms that cannot readily be classified as either DNA or RNA. Likewise, a double stranded compound may be DNA:DNA, DNA:RNA or RNA:RNA, and any one strand may also include a mixture of DNA and RNA, as well as modified forms that cannot readily be classified as either DNA or RNA. A nucleic acid compound may include any of a variety of modifications, including one or more modifications to the backbone (the sugar-phosphate portion in a natural nucleic acid, including internucleotide linkages) or the base portion (the purine or pyrimidine portion of a natural nucleic acid). An antisense nucleic acid compound will preferably have a length of about 15 to about 30 nucleotides and will often contain one or more modifications to improve characteristics such as stability in the serum, in a cell or in a place where the compound is likely to be delivered, such as the stomach in the case of orally delivered compounds and the lung for inhaled compounds. In the case of an RNAi construct, the strand complementary to the target transcript will generally be RNA or modifications thereof. The other strand may be RNA, DNA or any other variation. The duplex portion of double stranded or single stranded "hairpin" RNAi construct will generally have a length of 18 to 40 nucleotides in length and optionally about 21 to 23 nucleotides in length, so long as it serves as a Dicer substrate. Catalytic or enzymatic nucleic acids may be ribozymes or DNA enzymes and may also contain modified forms. Nucleic acid compounds may inhibit expression of the target by about 50%, 75%, 90% or more when contacted with cells under physiological conditions and at a concentration where a nonsense or sense control has little or no effect. Preferred concentrations for testing the effect of nucleic acid compounds are 1, 5 and 10 micromolar. Nucleic acid compounds may also be tested for effects on, for example, red blood cell levels.

In certain instances, an ActRIIb antagonist may be a follistatin polypeptide that antagonizes activin bioactivity and/or binds to activin. The term "follistatin polypeptide" includes polypeptides comprising any naturally occurring polypeptide of follistatin as well as any variants thereof (including mutants, fragments, fusions, and peptidomimetic forms) that retain a useful activity, and further includes any functional monomer or multimer of follistatin. Variants of follistatin polypeptides that retain activin binding properties can be identified based on previous studies involving follistatin and activin interactions. For example, WO2008/030367 discloses specific follistatin domains ("FSDs") that are shown to be important for activin binding. As shown below in SEQ ID NOs: 9-11, the N-terminus follistatin domain ("FSND" SEQ ID NO: 9), FSD2 (SEQ ID NO: 10), and to a lesser extent FSD1 (SEQ ID NO: 11) represent exemplary domains within follistatin important for activin binding. In addition, methods for making and testing libraries of polypeptides are described above in the context of ActRIIb polypeptides and such methods also pertain to making and testing variants of follistatin. Follistatin polypeptides include polypeptides derived from the sequence of any known follistatin having a sequence at least about 80% identical to the sequence of a follistatin polypeptide, and optionally at least 85%, 90%, 95%, 97%, 99% or greater identity. Examples of follistatin polypeptides include the mature follistatin polypeptide or shorter isoforms or other variants of the human follistatin precursor polypeptide (SEQ ID NO: 7) as described, for example, in WO2005/025601.

The human follistatin precursor polypeptide isoform FST344 is as follows:

The signal peptide is single underlined; the last 27 residues in bold represent additional amino acids as compared to a shorter follistatin isoform FST317 (NP_006341) below.

The human follistatin precursor polypeptide isoform FST317 is as follows:

The signal peptide is single underlined.

N-terminus follistatin domain (FSND) sequence is as follows:

The FSD1 and FSD2 sequences are as follows: ETCENVDCGPGKKCRMNKKNKPRCV (SEQ ID NO: 10; FSD1) KTCRDVFCPGSSTCVVDQTNNAYCVT (SEQ ID NO: 11; FSD2)

In other instances, an antagonist for use herein may be a follistatin-like related gene (FLRG) that antagonizes activin bioactivity and/or binds to activin and other ligands with a spectrum that resembles that of ActRIIb-Fc. The term "FLRG polypeptide" includes polypeptides comprising any naturally occurring polypeptide of FLRG as well as any variants thereof (including mutants, fragments, fusions, and peptidomimetic forms) that retain a useful activity. Variants of FLRG polypeptides that retain activin binding properties can be identified using routine methods to assay FLRG and activin interactions. See, for example, US 6,537,966. In addition, methods for making and testing libraries of polypeptides are described above in the context of ActRIIb polypeptides and such methods also pertain to making and testing variants of FLRG. FLRG polypeptides include polypeptides derived from the sequence of any known FLRG having a sequence at least about 80% identical to the sequence of an FLRG polypeptide, and optionally at least 85%, 90%, 95%, 97%, 99% or greater identity.

The human FLRG precursor polypeptide is as follows:

The signal peptide is single underlined.

In certain instances, functional variants or modified forms of the follistatin polypeptides and FLRG polypeptides include fusion protein having at least a portion of the follistatin polypeptides or FLRG polypeptides and one or more fusion domains, such as, for example, domains that facilitate isolation, detection, stabilization or multimerization of the polypeptide. Suitable fusion domains are discussed in detail above with reference to the ActRIIb polypeptides. In one instance, an antagonist is a fusion protein comprising an activin binding portion of a follistaton polypeptide fused to an Fc domain. In another instance, an antagonist is a fusion protein comprising an activin binding portion of an FLRG polypeptide fused to an Fc domain. Follistatin and FLRG have been shown in the literature, and by the applicants with respect to FLRG, to have affinities for Activin A in the picomolar range, indicating that these agents will inhibit activin A signaling to a similar degree as ActRIIb-Fc.

### 5. Screening Assays

In certain aspects, the present disclosure relates to the use of ActRIIb polypeptides (e.g., soluble ActRIIb polypeptides) and activin or other ActRIIb ligands to identify compounds (agents) which are agonist or antagonists of the ActRIIb signaling pathway. Compounds identified through this screening can be tested to assess their ability to modulate bone growth or mineralization or stimulate muscle growth in vitro. Optionally, these compounds can further be tested in animal models to assess their ability to modulate tissue growth in vivo.

There are numerous approaches to screening for therapeutic agents for modulating tissue growth by targeting ActRIIb polypeptides. In certain instances, high-throughput screening of compounds can be carried out to identify agents that perturb ActRIIb-mediated effects on bone or muscle. In certain instances, the assay is carried out to screen and identify compounds that specifically inhibit or reduce binding of an ActRIIb polypeptide to activin or another ligand. Alternatively, the assay can be used to identify compounds that enhance binding of an ActRIIb polypeptide to activin or another ligand. In a further instance, the compounds can be identified by their ability to interact with an ActRIIb polypeptide.

A variety of assay formats will suffice and, in light of the present disclosure, those not expressly described herein will nevertheless be comprehended by one of ordinary skill in the art. As described herein, the test compounds (agents) of the disclosure may be created by any combinatorial chemical method. Alternatively, the subject compounds may be naturally occurring biomolecules synthesized in vivo or in vitro. Compounds (agents) to be tested for their ability to act as modulators of tissue growth can be produced, for example, by bacteria, yeast, plants or other organisms (e.g., natural products), produced chemically (e.g., small molecules, including peptidomimetics), or produced recombinantly. Test compounds contemplated by the present disclosure include non-peptidyl organic molecules, peptides, polypeptides, peptidomimetics, sugars, hormones, and nucleic acid molecules. In a specific instance, the test agent is a small organic molecule having a molecular weight of less than about 2,000 daltons.

The test compounds of the disclosure can be provided as single, discrete entities, or provided in libraries of greater complexity, such as made by combinatorial chemistry. These libraries can comprise, for example, alcohols, alkyl halides, amines, amides, esters, aldehydes, ethers and other classes of organic compounds. Presentation of test compounds to the test system can be in either an isolated form or as mixtures of compounds, especially in initial screening steps. Optionally, the compounds may be optionally derivatized with other compounds and have derivatizing groups that facilitate isolation of the compounds. Non-limiting examples of derivatizing groups include biotin, fluorescein, digoxygenin, green fluorescent protein, isotopes, polyhistidine, magnetic beads, glutathione S transferase (GST), photoactivatible crosslinkers or any combinations thereof.

In many drug screening programs which test libraries of compounds and natural extracts, high throughput assays are desirable in order to maximize the number of compounds surveyed in a given period of time. Assays which are performed in cell-free systems, such as may be derived with purified or semi-purified proteins, are often preferred as "primary" screens in that they can be generated to permit rapid development and relatively easy detection of an alteration in a molecular target which is mediated by a test compound. Moreover, the effects of cellular toxicity or bioavailability of the test compound can be generally ignored in the in vitro system, the assay instead being focused primarily on the effect of the drug on the molecular target as may be manifest in an alteration of binding affinity between an ActRIIb polypeptide and activin.

Merely to illustrate, in an exemplary screening assay of the present disclosure, the compound of interest is contacted with an isolated and purified ActRIIb polypeptide which is ordinarily capable of binding to activin. To the mixture of the compound and ActRIIb polypeptide is then added a composition containing an ActRIIb ligand. Detection and quantification of ActRIIb/activin complexes provides a means for determining the compound's efficacy at inhibiting (or potentiating) complex formation between the ActRIIb polypeptide and activin. The efficacy of the compound can be assessed by generating dose response curves from data obtained using various concentrations of the test compound. Moreover, a control assay can also be performed to provide a baseline for comparison. For example, in a control assay, isolated and purified activin is added to a composition containing the ActRIIb polypeptide, and the formation of ActRIIb/activin complex is quantitated in the absence of the test compound. It will be understood that, in general, the order in which the reactants may be admixed can be varied, and can be admixed simultaneously. Moreover, in place of purified proteins, cellular extracts and lysates may be used to render a suitable cell-free assay system.

Complex formation between the ActRIIb polypeptide and activin may be detected by a variety of techniques. For instance, modulation of the formation of complexes can be quantitated using, for example, detectably labeled proteins such as radiolabeled (e.g., ³²P, ³⁵S, ¹⁴C or ³H), fluorescently labeled (e.g., FITC), or enzymatically labeled ActRIIb polypeptide or activin, by immunoassay, or by chromatographic detection.

In certain instances, the present disclosure contemplates the use of fluorescence polarization assays and fluorescence resonance energy transfer (FRET) assays in measuring, either directly or indirectly, the degree of interaction between an ActRIIb polypeptide and its binding protein. Further, other modes of detection, such as those based on optical waveguides (PCT Publication WO 96/26432 and U.S. Pat. No. 5,677,196), surface plasmon resonance (SPR), surface charge sensors, and surface force sensors, are compatible with many instances of the disclosure.

Moreover, the present disclosure contemplates the use of an interaction trap assay, also known as the "two hybrid assay," for identifying agents that disrupt or potentiate interaction between an ActRIIb polypeptide and its binding protein. See for example, U.S. Pat. No. 5,283,317; Zervos et al. (1993) Cell 72:223-232; Madura et al. (1993) J Biol Chem 268:12046-12054; Bartel et al. (1993) Biotechniques 14:920-924; and Iwabuchi et al. (1993) Oncogene 8:1693-1696). In a specific instance, the present disclosure contemplates the use of reverse two hybrid systems to identify compounds (e.g., small molecules or peptides) that dissociate interactions between an ActRIIb polypeptide and its binding protein. See for example, Vidal and Legrain, (1999) Nucleic Acids Res 27:919-29; Vidal and Legrain, (1999) Trends Biotechnol 17:374-81; and U.S. Pat. Nos. 5,525,490; 5,955,280; and 5,965,368.

In certain instances, the subject compounds are identified by their ability to interact with an ActRIIb or activin polypeptide of the disclosure. The interaction between the compound and the ActRIIb or activin polypeptide may be covalent or non-covalent. For example, such interaction can be identified at the protein level using in vitro biochemical methods, including photo-crosslinking, radiolabeled ligand binding, and affinity chromatography (Jakoby WB et al., 1974, Methods in Enzymology 46: 1). In certain cases, the compounds may be screened in a mechanism based assay, such as an assay to detect compounds which bind to an activin or ActRIIb polypeptide. This may include a solid phase or fluid phase binding event. Alternatively, the gene encoding an activin or ActRIIb polypeptide can be transfected with a reporter system (e.g., β-galactosidase, luciferase, or green fluorescent protein) into a cell and screened against the library preferably by a high throughput screening or with individual members of the library. Other mechanism based binding assays may be used, for example, binding assays which detect changes in free energy. Binding assays can be performed with the target fixed to a well, bead or chip or captured by an immobilized antibody or resolved by capillary electrophoresis. The bound compounds may be detected usually using colorimetric or fluorescence or surface plasmon resonance.

In certain aspects, the present disclosure provides methods and agents for modulating (stimulating or inhibiting) bone formation and increasing bone mass. Therefore, any compound identified can be tested in whole cells or tissues, in vitro or in vivo, to confirm their ability to modulate bone or cartilage growth. Various methods known in the art can be utilized for this purpose.

For example, the effect of the ActRIIb or activin polypeptides or test compounds on bone or cartilage growth can be determined by measuring induction of Msx2 or differentiation of osteoprogenitor cells into osteoblasts in cell based assays (see, e.g., Daluiski et al., Nat Genet. 2001, 27(1):84-8; Hino et al., Front Biosci. 2004, 9:1520-9). Another example of cell-based assays includes analyzing the osteogenic activity of the subject ActRIIb or activin polypeptides and test compounds in mesenchymal progenitor and osteoblastic cells. To illustrate, recombinant adenoviruses expressing an activin or ActRIIb polypeptide can be constructed to infect pluripotent mesenchymal progenitor C3H10T1/2 cells, preosteoblastic C2C12 cells, and osteoblastic TE-85 cells. Osteogenic activity is then determined by measuring the induction of alkaline phosphatase, osteocalcin, and matrix mineralization (see, e.g., Cheng et al., J bone Joint Surg Am. 2003, 85-A(8): 1544-52).

The present disclosure also contemplates in vivo assays to measure bone or cartilage growth. For example, Namkung-Matthai et al., Bone, 28:80-86 (2001) discloses a rat osteoporotic model in which bone repair during the early period after fracture is studied. Kubo et al., Steroid Biochemistry & Molecular Biology, 68:197-202 (1999) also discloses a rat osteoporotic model in which bone repair during the late period after fracture is studied. Andersson et al., J. Endocrinol. 170:529-537 describe a mouse osteoporosis model in which mice are ovariectomized, which causes the mice to lose substantial bone mineral content and bone mineral density, with the trabecular bone losing roughly 50% of bone mineral density. Bone density could be increased in the ovariectomized mice by administration of factors such as parathyroid hormone. In certain aspects, the present disclosure makes use of fracture healing assays that are known in the art. These assays include fracture technique, histological analysis, and biomechanical analysis, which are described in, for example, U.S. Pat. No. 6,521,750.

In certain aspects, the present disclosure provides methods and agents for stimulating muscle growth and increasing muscle mass, for example, by antagonizing functions of an ActRIIb polypeptide and/or an ActRIIb ligand. Therefore, any compound identified can be tested in whole cells or tissues, in vitro or in vivo, to confirm their ability to modulate muscle growth. Various methods known in the art can be utilized for this purpose. For example, methods of the disclosure are performed such that the signal transduction through an ActRIIb protein activated by binding to an ActRIIb ligand (e.g., GDF8) has been reduced or inhibited. It will be recognized that the growth of muscle tissue in the organism would result in an increased muscle mass in the organism as compared to the muscle mass of a corresponding organism (or population of organisms) in which the signal transduction through an ActRIIb protein had not been so effected.

For example, the effect of the ActRIIb polypeptides or test compounds on muscle cell growth/proliferation can be determined by measuring gene expression of Pax-3 and Myf-5 which are associated with proliferation of myogenic cells, and gene expression of MyoD which is associated with muscle differentiation (e.g., Amthor et al., Dev Biol. 2002, 251:241-57). It is known that GDF8 down-regulates gene expression of Pax-3 and Myf-5, and prevents gene expression of MyoD. The ActRIIb polypeptides or test compounds are expected to antagonize this activity of GDF8. Another example of cell-based assays includes measuring the proliferation of myoblasts such as C(2)C(12) myoblasts in the presence of the ActRIIb polypeptides or test compounds (e.g., Thomas et al., J Biol Chem. 2000, 275:40235-43).

The present disclosure also contemplates in vivo assays to measure muscle mass and strength. For example, Whittemore et al. (Biochem Biophys Res Commun. 2003, 300:965-71) discloses a method of measuring increased skeletal muscle mass and increased grip strength in mice. Optionally, this method can be used to determine therapeutic effects of test compounds (e.g., ActRIIb polypeptides) on muscle diseases or conditions, for example those diseases for which muscle mass is limiting.

It is understood that the screening assays of the present disclosure apply to not only the subject ActRIIb polypeptides and variants of the ActRIIb polypeptides, but also any test compounds including agonists and antagonist of the ActRIIb polypeptides. Further, these screening assays are useful for drug target verification and quality control purposes.

### 6. Exemplary Therapeutic Uses

ActRIIb polypeptides of the present invention can be used for stimulating anabolic bone growth, treating an osteolytic bone tumor, and/or treating or preventing a disease or condition that would benefit from muscle growth. In certain embodiments, ActRIIb antagonists of the invention,
may be used for treating or preventing cancer-related bone loss. In certain instances, the present disclosure provides methods of treating or preventing bone damage in an individual in need thereof through administering to the individual a therapeutically effective amount of an ActRIIb antagonist, particularly an ActRIIb polypeptide. In certain instances, the present disclosure provides methods of promoting bone growth or mineralization in an individual in need thereof through administering to the individual a therapeutically effective amount of an ActRIIb antagonist, particularly an ActRIIb polypeptide. These methods are preferably aimed at therapeutic and prophylactic treatments of animals, and more preferably, humans. In certain embodiments, the disclosure provides for the use of ActRIIb antagonists of the invention particularly soluble ActRIIb polypeptides)
for the treatment of disorders associated with low bone density or decreased bone strength.

As used herein, a therapeutic that "prevents" a disorder or condition refers to a compound that, in a statistical sample, reduces the occurrence of the disorder or condition in the treated sample relative to an untreated control sample, or delays the onset or reduces the severity of one or more symptoms of the disorder or condition relative to the untreated control sample. The term "treating" as used herein includes prophylaxis of the named condition or amelioration or elimination of the condition once it has been established. In either case, prevention or treatment may be discerned in the diagnosis provided by a physician and the intended result of administration of the therapeutic agent.

The disclosure provides methods of inducing bone and/or cartilage formation, preventing bone loss, increasing bone mineralization or preventing the demineralization of bone. For example, the subject ActRIIb antagonists have application in treating osteoporosis and the healing of bone fractures and cartilage defects in humans and other animals. ActRIIb polypeptides may be useful in patients that are diagnosed with subclinical low bone density, as a protective measure against the development of osteoporosis.

In one specific embodiment, compositions of the present invention may find medical utility in the healing of bone fractures and cartilage defects in humans and other animals. The subject compositions may also have prophylactic use in closed as well as open fracture reduction and also in the improved fixation of artificial joints. De novo bone formation induced by an osteogenic agent contributes to the repair of congenital, trauma-induced, or oncologic resection induced craniofacial defects, and also is useful in cosmetic plastic surgery. In certain cases, the subject ActRIIb antagonists may provide an environment to attract bone-forming cells, stimulate growth of bone-forming cells or induce differentiation of progenitors of bone-forming cells. ActRIIb antagonists of the invention may also be useful in the treatment of osteoporosis.

C ompositions of the invention can be applied to conditions characterized by or causing bone loss, such as osteoporosis (including secondary osteoporosis), hyperparathyroidism, Cushing's disease, Paget's disease, thyrotoxicosis, chronic diarrheal state or malabsorption, chronic renal disease, renal tubular acidosis, or anorexia nervosa.

Osteoporosis may be caused by, or associated with, various factors. Being female, particularly a post-menopausal female, having a low body weight, and leading a sedentary lifestyle are all risk factors for osteoporosis (loss of bone mineral density, leading to fracture risk). Persons having any of the following profiles may be candidates for treatment with an ActRIIb antagonist: a post-menopausal woman and not taking estrogen or other hormone replacement therapy; a person with a personal or maternal history of hip fracture or smoking; a post-menopausal woman who is tall (over 5 feet 7 inches) or thin (less than 125 pounds); a man with clinical conditions associated with bone loss; a person using medications that are known to cause bone loss, including corticosteroids such as Prednisone™, various anti-seizure medications such as Dilantin™ and certain barbiturates, or high-dose thyroid replacement drugs; a person liver disease or a family history of osteoporosis; a person having high bone turnover (e.g., excessive collagen in urine samples); a person with a thyroid condition, such as hyperthyroidism; a person who has experienced a fracture after only mild trauma; a person who has had x-ray evidence of vertebral fracture or other signs of osteoporosis.

As noted above, osteoporosis can also result as a condition associated with another disorder or from the use of certain medications. Osteoporosis resulting from drugs or another medical condition is known as secondary osteoporosis. In a condition known as Cushing's disease, the excess amount of cortisol produced by the body results in osteoporosis and fractures. The most common medications associated with secondary osteoporosis are the corticosteroids, a class of drugs that act like cortisol, a hormone produced naturally by the adrenal glands. Although adequate levels of thyroid hormones (which are produced by the thyroid gland) are needed for the development of the skeleton, excess thyroid hormone can decrease bone mass over time. Other medications that can cause secondary osteoporosis include phenytoin (Dilantin) and barbiturates that are used to prevent seizures; methotrexate (Rheumatrex, Immunex, Folex PFS), a drug for some forms of arthritis, cancer, and immune disorders; cyclosporine (Sandimmune, Neoral), a drug used to treat some autoimmune diseases and to suppress the immune system in organ transplant patients; luteinizing hormone-releasing hormone agonists (Lupron, Zoladex), used to treat prostate cancer and endometriosis; heparin (Calciparine, Liquaemin), an anticlotting medication; and cholestyramine (Questran) and colestipol (Colestid), used to treat high cholesterol.

Bone loss resulting from cancer therapy is widely recognized and termed cancer therapy induced bone loss (CTIBL). Bone metastases can create cavities in the bone that may be corrected by treatment with ActRIIb antagonists.

In a preferred embodiment, ActRIIb antagonists of the invention, particularly a soluble ActRIIb, disclosed herein may be used in cancer patients. As demonstrated herein, an ActRIIb-Fc fusion protein is capable of promoting anabolic bone growth in human patients. It has been demonstrated that anabolic bone agents are useful for treating multiple myeloma (see e.g., Yaccoby et al., Blood, 109(5):2106-11 (2007); Oyajobi et al., Br J Haematol. 139(3):434-8 (2007); Fulciniti et al., Blood, 114(2): 371-9 (2009); Stewart et al. Journal of Cellular Biochemistry 98:1-13 (2006)). Accordingly, the Activin-ActRIIb antagonists described herein are believed to be useful for treating multiple myeloma and other bone tumors. Patients having certain tumors (e.g. prostate, breast, multiple myeloma or any tumor causing hyperparathyroidism) are at high risk for bone loss due to tumor-induced bone loss as well as bone metastases and therapeutic agents. Such patients may be treated with ActRIIb antagonists even in the absence of evidence of bone loss or bone metastases. Patients may also be monitored for evidence of bone loss or bone metastases, and may be treated with ActRIIb antagonists in the event that indicators suggest an increased risk. Generally, DEXA scans are employed to assess changes in bone density, while indicators of bone remodeling may be used to assess the likelihood of bone metastases. Serum markers may be monitored. Bone specific alkaline phosphatase (BSAP) is an enzyme that is present in osteoblasts. Blood levels of BSAP are increased in patients with bone metastasis and other conditions that result in increased bone remodeling. Osteocalcin and procollagen peptides are also associated with bone formation and bone metastases. Increases in BSAP have been detected in patients with bone metastasis caused by prostate cancer, and to a lesser degree, in bone metastases from breast cancer. Bone Morphogenetic Protein-7 (BMP-7) levels are high in prostate cancer that has metastasized to bone, but not in bone metastases due to bladder, skin, liver, or lung cancer. Type I Carboxy-terminal telopeptide (ICTP) is a crosslink found in collagen that is formed during to the resorption of bone. Since bone is constantly being broken down and reformed, ICTP will be found throughout the body. However, at the site of bone metastasis, the level will be significantly higher than in an area of normal bone. ICTP has been found in high levels in bone metastasis due to prostate, lung, and breast cancer. Another collagen crosslink, Type I N-terminal telopeptide (NTx), is produced along with ICTP during bone turnover. The amount of NTx is increased in bone metastasis caused by many different types of cancer including lung, prostate, and breast cancer. Also, the levels of NTx increase with the progression of the bone metastasis. Therefore, this marker can be used to both detect metastasis as well as measure the extent of the disease. Other markers of resorption include pyridinoline and deoxypyridinoline. Any increase in resorption markers or markers of bone metastases indicate the need for ActRIIb antagonist therapy in a patient.

Because ActRIIb-Fc has a clear effect on markers of osteoblast activity (e.g., BSAP levels), ActRIIb-Fc molecules may be particularly useful in cancer-related bone loss settings where the bone disease is of an osteolytic nature, rather than an osteoblastic nature. This is typically true of multiple myeloma and bone metastases from a variety of tumors. However, bone metastases from prostate cancer in particular have a tendency to be osteoblastic in nature, and ActRIIb-Fc fusion proteins may not be beneficial in this setting.

ActRIIb antagonists may be conjointly administered with other pharmaceutical agents. Conjoint administration may be accomplished by administration of a single co-formulation, by simultaneous administration or by administration at separate times. ActRIIb antagonists may be particularly advantageous if administered with other bone-active agents. A patient may benefit from conjointly receiving ActRIIb antagonist and taking calcium supplements, vitamin D, appropriate exercise and/or, in some cases, other medication. Examples of other medications incude, bisphosphonates (alendronate, ibandronate and risedronate), calcitonin, estrogens, parathyroid hormone and raloxifene. The bisphosphonates (alendronate, ibandronate and risedronate), calcitonin, estrogens and raloxifene affect the bone remodeling cycle and are classified as anti-resorptive medications. Bone remodeling consists of two distinct stages: bone resorption and bone formation. Anti-resorptive medications slow or stop the bone-resorbing portion of the bone-remodeling cycle but do not slow the bone-forming portion of the cycle. As a result, new formation continues at a greater rate than bone resorption, and bone density may increase over time. Teriparatide, a form of parathyroid hormone, increases the rate of bone formation in the bone remodeling cycle. Alendronate is approved for both the prevention (5 mg per day or 35 mg once a week) and treatment (10 mg per day or 70 mg once a week) of postmenopausal osteoporosis. Alendronate reduces bone loss, increases bone density and reduces the risk of spine, wrist and hip fractures. Alendronate also is approved for treatment of glucocorticoid-induced osteoporosis in men and women as a result of long-term use of these medications (i.e., prednisone and cortisone) and for the treatment of osteoporosis in men. Alendronate plus vitamin D is approved for the treatment of osteoporosis in postmenopausal women (70 mg once a week plus vitamin D), and for treatment to improve bone mass in men with osteoporosis. Ibandronate is approved for the prevention and treatment of postmenopausal osteoporosis. Taken as a once-a-month pill (150 mg), ibandronate should be taken on the same day each month. Ibandronate reduces bone loss, increases bone density and reduces the risk of spine fractures. Risedronate is approved for the prevention and treatment of postmenopausal osteoporosis. Taken daily (5 mg dose) or weekly (35 mg dose or 35 mg dose with calcium), risedronate slows bone loss, increases bone density and reduces the risk of spine and non-spine fractures. Risedronate also is approved for use by men and women to prevent and/or treat glucocorticoid-induced osteoporosis that results from long-term use of these medications (i.e., prednisone or cortisone). Calcitonin is a naturally occurring hormone involved in calcium regulation and bone metabolism. In women who are more than 5 years beyond menopause, calcitonin slows bone loss, increases spinal bone density, and may relieve the pain associated with bone fractures. Calcitonin reduces the risk of spinal fractures. Calcitonin is available as an injection (50-100 IU daily) or nasal spray (200 IU daily). Estrogen therapy (ET)/Hormone therapy (HT) is approved for the prevention of osteoporosis. ET has been shown to reduce bone loss, increase bone density in both the spine and hip, and reduce the risk of hip and spinal fractures in postmenopausal women. ET is administered most commonly in the form of a pill or skin patch that delivers a low dose of approximately 0.3 mg daily or a standard dose of approximately 0.625 mg daily and is effective even when started after age 70. When estrogen is taken alone, it can increase a woman's risk of developing cancer of the uterine lining (endometrial cancer). To eliminate this risk, healthcare providers prescribe the hormone progestin in combination with estrogen (hormone replacement therapy or HT) for those women who have an intact uterus. ET/HT relieves menopause symptoms and has been shown to have a beneficial effect on bone health. Side effects may include vaginal bleeding, breast tenderness, mood disturbances and gallbladder disease. Raloxifene, 60 mg a day, is approved for the prevention and treatment of postmenopausal osteoporosis. It is from a class of drugs called Selective Estrogen Receptor Modulators (SERMs) that have been developed to provide the beneficial effects of estrogens without their potential disadvantages. Raloxifene increases bone mass and reduces the risk of spine fractures. Data are not yet available to demonstrate that raloxifene can reduce the risk of hip and other non-spine fractures. Teriparatide, a form of parathyroid hormone, is approved for the treatment of osteoporosis in postmenopausal women and men who are at high risk for a fracture. This medication stimulates new bone formation and significantly increases bone mineral density. In postmenopausal women, fracture reduction was noted in the spine, hip, foot, ribs and wrist. In men, fracture reduction was noted in the spine, but there were insufficient data to evaluate fracture reduction at other sites. Teriparatide is self-administered as a daily injection for up to 24 months.

As shown herein, ActRIIb antagonists are also useful in promoting muscle growth, and as such may be useful in a host of muscle-related disorders. Exemplary muscle-related conditions include neuromuscular disorders (e.g., muscular dystrophy and muscle atrophy), congestive obstructive pulmonary disease (and muscle wasting associated with COPD), muscle wasting syndrome, sarcopenia, cachexia, adipose tissue disorders (e.g., obesity), type 2 diabetes, and bone degenerative disease (e.g., osteoporosis). Other exemplary conditions include musculodegenerative and neuromuscular disorders, tissue repair (e.g., wound healing), neurodegenerative diseases (e.g., amyotrophic lateral sclerosis), immunologic disorders (e.g., disorders related to abnormal proliferation or function of lymphocytes), and obesity or disorders related to abnormal proliferation of adipocytes.

In certain embodiments, ActRIIb antagonists of the invention are used as part of a treatment for a muscular dystrophy. The term "muscular dystrophy" refers to a group of degenerative muscle diseases characterized by gradual weakening and deterioration of skeletal muscles and sometimes the heart and respiratory muscles. Muscular dystrophies are genetic disorders characterized by progressive muscle wasting and weakness that begin with microscopic changes in the muscle. As muscles degenerate over time, the person's muscle strength declines. Exemplary muscular dystrophies that can be treated with a regimen including the subject ActRIIb polypeptides include: Duchenne Muscular Dystrophy (DMD), Becker Muscular Dystrophy (BMD), Emery-Dreifuss Muscular Dystrophy (EDMD), Limb-Girdle Muscular Dystrophy (LGMD), Facioscapulohumeral Muscular Dystrophy (FSH or FSHD) (also known as Landouzy-Dejerine), Myotonic Dystrophy (MMD) (also known as Steinert's Disease), Oculopharyngeal Muscular Dystrophy (OPMD), Distal Muscular Dystrophy (DD), Congenital Muscular Dystrophy (CMD).

Duchenne Muscular Dystrophy (DMD) was first described by the French neurologist Guillaume Benjamin Amand Duchenne in the 1860s. Becker Muscular Dystrophy (BMD) is named after the German doctor Peter Emil Becker, who first described this variant of DMD in the 1950s. DMD is one of the most frequent inherited diseases in males, affecting one in 3,500 boys. DMD occurs when the dystrophin gene, located on the short arm of the X chromosome, is broken. Since males only carry one copy of the X chromosome, they only have one copy of the dystrophin gene. Without the dystrophin protein, muscle is easily damaged during cycles of contraction and relaxation. While early in the disease muscle compensates by regeneration, later on muscle progenitor cells cannot keep up with the ongoing damage and healthy muscle is replaced by non-functional fibro-fatty tissue. A dual effect on muscle and bone may be useful in muscular dystrophy patients.

BMD results from different mutations in the dystrophin gene. BMD patients have some dystrophin, but it is either insufficient in quantity or poor in quality. Having some dystrophin protects the muscles of those with BMD from degenerating as badly or as quickly as those of people with DMD.

For example, recent researches demonstrate that blocking or eliminating function of GDF8 (an ActRIIb ligand) in vivo can effectively treat at least certain symptoms in DMD and BMD patients. Thus, the subject ActRIIb antagonists may act as GDF8 inhibitors (antagonists), and constitute an alternative means of blocking the functions of GDF8 and/or ActRIIb in vivo in DMD and BMD patients. An ActRIIb-Fc protein has been shown to increase muscle mass in a mouse model of muscular dystrophy (see U.S. Publication No. 2009/0005308).

Similarly, the subject ActRIIb antagonists provide an effective means to increase muscle mass in other disease conditions that are in need of muscle growth. For example, ALS, also called Lou Gehrig's disease (motor neuron disease) is a chronic, incurable, and unstoppable CNS disorder that attacks the motor neurons, components of the CNS that connect the brain to the skeletal muscles. In ALS, the motor neurons deteriorate and eventually die, and though a person's brain normally remains fully functioning and alert, the command to move never reaches the muscles. Most people who get ALS are between 40 and 70 years old. The first motor neurons that weaken are those leading to the arms or legs. Those with ALS may have trouble walking, they may drop things, fall, slur their speech, and laugh or cry uncontrollably. Eventually the muscles in the limbs begin to atrophy from disuse. This muscle weakness will become debilitating and a person will need a wheel chair or become unable to function out of bed. Most ALS patients die from respiratory failure or from complications of ventilator assistance like pneumonia, 3-5 years from disease onset. An ActRIIb-Fc protein has been shown shown to improve the appearance, muscle mass and lifespan of a mouse model of ALS (see U.S. Publication No. 2009/0005308).

ActRIIb antagonists-induced increased muscle mass might also benefit those suffering from muscle wasting diseases. It has been observed that GDF8 expression correlates inversely with fat-free mass in humans and that increased expression of the GDF8 gene is associated with weight loss in men with AIDS wasting syndrome. By inhibiting the function of GDF8 in AIDS patients, at least certain symptoms of AIDS may be alleviated, if not completely eliminated, thus significantly improving quality of life in AIDS patients.

Since loss of GDF8 (an ActRIIb ligand) function is also associated with fat loss without diminution of nutrient intake, the subject ActRIIb antagonists may further be used as a therapeutic agent for slowing or preventing the development of obesity and type II diabetes. This approach is confirmed and supported by the data shown herein, whereby an ActRIIb-Fc protein was shown to improve metabolic status in obese mice.

The cancer anorexia-cachexia syndrome is among the most debilitating and life-threatening aspects of cancer. Progressive weight loss in cancer anorexia-cachexia syndrome is a common feature of many types of cancer and is responsible not only for a poor quality of life and poor response to chemotherapy, but also a shorter survival time than is found in patients with comparable tumors without weight loss. Associated with anorexia, fat and muscle tissue wasting, psychological distress, and a lower quality of life, cachexia arises from a complex interaction between the cancer and the host. It is one of the most common causes of death among cancer patients and is present in 80% at death. It is a complex example of metabolic chaos effecting protein, carbohydrate, and fat metabolism. Tumors produce both direct and indirect abnormalities, resulting in anorexia and weight loss. Currently, there is no treatment to control or reverse the process. Cancer anorexia-cachexia syndrome affects cytokine production, release of lipid-mobilizing and proteolysis-inducing factors, and alterations in intermediary metabolism. Although anorexia is common, a decreased food intake alone is unable to account for the changes in body composition seen in cancer patients, and increasing nutrient intake is unable to reverse the wasting syndrome. Cachexia should be suspected in patients with cancer if an involuntary weight loss of greater than five percent of premorbid weight occurs within a six-month period.

Since systemic overexpression of GDF8 in adult mice has been shown to induce profound muscle and fat loss analogous to that seen in human cachexia syndromes, the subject ActRIIb antagonists as pharmaceutical compositions can be beneficially used to prevent, treat, or alleviate the symptoms of the cachexia syndrome, where muscle growth is desired.

In other instances, the present disclosure provides compositions and methods for regulating body fat content in an animal and for treating or preventing conditions related thereto, and particularly, health-compromising conditions related thereto. According to the present disclosure, to regulate (control) body weight can refer to reducing or increasing body weight, reducing or increasing the rate of weight gain, or increasing or reducing the rate of weight loss, and also includes actively maintaining, or not significantly changing body weight (e.g., against external or internal influences which may otherwise increase or decrease body weight). One instance of the present disclosure relates to regulating body weight by administering to an animal (e.g., a human) in need thereof an ActRIIb antagonist.

In one specific instance, the present disclosure relates to methods and compounds for reducing body weight and/or reducing weight gain in an animal, and more particularly, for treating or ameliorating obesity in patients at risk for or suffering from obesity. In another specific instance, the present disclosure is directed to methods and compounds for treating an animal that is unable to gain or retain weight (e.g., an animal with a wasting syndrome). Such methods are effective to increase body weight and/or mass, or to reduce weight and/or mass loss, or to improve conditions associated with or caused by undesirably low (e.g., unhealthy) body weight and/or mass.

### 7. Dosing

In certain instances, the disclosure provides methods for dosing a patient with an ActRIIb polypeptide. Methods of the disclosure include administering an ActRIIb protein to a patient on a dosing schedule that provides an optimal serum concentration of the ActRIIb protein that is maintained over a desired time period. A patient may be dosed on a schedule that involves a desired combination of the amount of an ActRIIb polypeptide administered to the patient as well as the frequency with which the ActRIIb polypeptide is administered to the patient. An ActRIIb polypeptide for use in such a dosing schedule may be any of those disclosed herein, such as a polypeptide comprising an amino acid sequence selected from SEQ ID NOs: 2, 3, 13, 17, or 20, or comprising an amino acid sequence that is at least 80%, 85%, 90%, 95%, 97%, 98% or 99% identical to an amino acid sequence selected from SEQ ID NOs: 2, 3, 13, 17, or 20. An ActRIIb polypeptide may include a functional fragment of a natural ActRIIb polypeptide, such as one comprising at least 10, 20 or 30 amino acids of a sequence selected from SEQ ID NOs: 1-3 or a sequence of SEQ ID NO: 2, lacking the C-terminal 10 to 15 amino acids (the "tail"). In certain embodiments the ActRIIb polypeptide of the invention comprises an amino acid sequence having at least 80%, 85%, 90%, 95%, 97%, 98% or 99% identity to a portion of ActRIIb, wherein the N-terminus of the ActRIIb polypeptide corresponds to an amino acid residue selected from amino acids 19-25 of SEQ ID NO:1 and the C-terminus of the ActRIIb polypeptide corresponds to an amino acid residue selected from amino acids 109-134 of SEQ ID NO:1. The ActRIIb polypeptide is an ActRIIb-Fc fusion protein disclosed herein.

A patient may be dosed so as to achieve an optimal serum concentration of the ActRIIb polypeptide. An optimal serum concentration may be, for example, a serum concentration that is sufficient to promote a desired biological effect *in vivo.* Such biological effects include any of therapeutic effects disclosed herein, including promoting bone growth, increasing bone density, treating or preventing a disorder associated with low bone density, treating neuromuscular disorders, adipose tissue disorders, metabolic disorders, neurodegenerative disorders, muscle wasting disorders, or cancer.

As disclosed herein, ActRIIb polypeptides of the disclosure demonstrate consistent biological efficacy when patients reach and maintain an ActRIIb polypeptide serum concentration of at least 10 µg/mL. Therefore, in certain instances, a patient may be administered an ActRIIb polypeptide on a dosing schedule that maintains an ActRIIb serum concentration of at least 8 µg/mL, 10 µg/mL, 12.5 µg/mL, 15 µg/mL, 20 µg/mL, 25 µg/mL, 30 µg/mL, 35 µg/mL, 40 µg/mL, 50 µg/mL, or at least 70 µg/mL. In certain instances, it may be desirable to maintain the serum concentration of the ActRIIb polypeptide within an optimal range, such as, for example, within a range of from about 8 µg/mL to about 100 µg/mL (e.g., 8-100 µg/mL, 8-70 µg/mL, 8-50 µg/mL, 8-40 µg/mL, 8-35 µg/mL, 8-30 µg/mL, 8-25 µg/mL, 8-20 µg/mL, 8-15 µg/mL, 8-12.5 µg/mL, 8-10 µg/mL, 10-100 µg/mL, 10-70 µg/mL, 10-50 µg/mL, 10-40 µg/mL, 10-35 µg/mL, 10-30 µg/mL, 10-25 µg/mL, 10-20 µg/mL, 10-15 µg/mL, 10-12.5 µg/mL, 12-100 µg/mL, 12-70 µg/mL, 12-50 µg/mL, 12-40 µg/mL, 12-35 µg/mL, 12-30 µg/mL, 12-25 µg/mL, 12-20 µg/mL, 12-15 µg/mL, 15-100 µg/mL, 15-70 µg/mL, 15-50 µg/mL, 15-40 µg/mL, 15-35 µg/mL, 15-30 µg/mL, 15-25 µg/mL, 15-20 µg/mL, 20-70 µg/mL, 20-50 µg/mL, 20-35 µg/mL, or 20-30 µg/mL).

In certain embodiments, dosing schedules of the disclosure include administering an ActRIIb polypeptide of the invention in an amount and at an interval sufficient to maintain a desired serum concentration of the ActRIIb protein for a desired time period. The desirable duration for maintaining a desired ActRIIb serum concentration in a patient will depend on the desired biological effect (e.g., treatment of a bone disorder). For example, a patient may be administered an ActRIIb protein in an amount and at an interval sufficient to maintain the desired serum concentration over the course of about a week to a year or more (e.g., maintain the desired serum concentration over 5 days, 7 days, 10 day, 11 days, 12 days, 13 days, 14 days, 15 days, 16 days, 20 days, 25 days, 30 days, one to two weeks, one week to a month, two months, four months, six months, a year, or two years or more.) Preferably, a patient is administered an ActRIIb protein in an amount sufficient to maintain the desired serum concentration over the course of a therapeutic treatment. The dosing schedule is sufficient to maintain the desired serum concentration of ActRIIb between dosing intervals (e.g., between ActRIIb administrations).

As disclosed herein, the biological effects of ActRII polypeptides may be achieved with doses of 0.3 mg/kg or greater. Therefore, in certain instances, a patient may be dosed with about 0.3 to about 15 mg/kg (e.g., 0.3, 0.5, 1.0, 1.5, 2.0, 3.0, 4.0, 5.0, 7.0, 10.0, 12.0, or 15.0 mg/kg) of an ActRIIb polypeptide. In preferred embodiments, a patient is administered at least 1.0 mg/kg. In addition, the experiments shown below indicate that the serum half-life of an ActRIIb-Fc fusion protein is between about 10 and 16 days. Therefore, in certain aspects, a patient may be dosed with an ActRIIb protein of the disclosure at least once a month (e.g., once every 5-30 days, 10-16 days, 5 days, 7 days, 10 days, 11 days, 12 days, 13 days, 14 days, 15 days, or 16 days; or on a monthly or a bi-monthly basis).

### 8. Pharmaceutical Compositions

In certain embodiments, ActRIIb polypeptides of the present invention are formulated with a pharmaceutically acceptable carrier. For example, an ActRIIb polypeptide can be administered alone or as a component of a pharmaceutical formulation (therapeutic composition). The subject compounds may be formulated for administration in any convenient way for use in human or veterinary medicine.

In certain instances, the therapeutic method of the disclosure includes administering the composition systemically, or locally as an implant or device. When administered, the therapeutic composition for use in this invention is in a pyrogen-free, physiologically acceptable form. Therapeutically useful agents other than the ActRIIb antagonists which may also optionally be included in the composition as described above, may be administered simultaneously or sequentially with the subject compounds (e.g., ActRIIb polypeptides) in the methods of the disclosure.

Typically, ActRIIb antagonists will be administered parentally, and particularly intravenously or subcutaneously. Pharmaceutical compositions suitable for parenteral administration may comprise one or more ActRIIb polypeptides in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents. Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

Further, the composition may be encapsulated or injected in a form for delivery to a target tissue site (e.g., bone or muscle). In certain embodiments, compositions of the present invention may include a matrix capable of delivering one or more therapeutic compounds (e.g., ActRIIb polypeptides) to a target tissue site (e.g., bone or muscle), providing a structure for the developing tissue and optimally capable of being resorbed into the body. For example, the matrix may provide slow release of the ActRIIb polypeptides. Such matrices may be formed of materials presently in use for other implanted medical applications.

The choice of matrix material is based on biocompatibility, biodegradability, mechanical properties, cosmetic appearance and interface properties. The particular application of the subject compositions will define the appropriate formulation. Potential matrices for the compositions may be biodegradable and chemically defined calcium sulfate, tricalciumphosphate, hydroxyapatite, polylactic acid and polyanhydrides. Other potential materials are biodegradable and biologically well defined, such as bone or dermal collagen. Further matrices are comprised of pure proteins or extracellular matrix components. Other potential matrices are non-biodegradable and chemically defined, such as sintered hydroxyapatite, bioglass, aluminates, or other ceramics. Matrices may be comprised of combinations of any of the above mentioned types of material, such as polylactic acid and hydroxyapatite or collagen and tricalciumphosphate. The bioceramics may be altered in composition, such as in calcium-aluminate-phosphate and processing to alter pore size, particle size, particle shape, and biodegradability.

In certain instances, methods of the disclosure can be administered for orally, e.g., in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of an agent as an active ingredient. An agent may also be administered as a bolus, electuary or paste.

In solid dosage forms for oral administration (capsules, tablets, pills, dragees, powders, granules, and the like), one or more therapeutic compounds of the present invention may be mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose, and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

Liquid dosage forms for oral administration include pharmaceutically acceptable emulsions, microemulsions, solutions, suspensions, syrups, and elixirs. In addition to the active ingredient, the liquid dosage forms may contain inert diluents commonly used in the art, such as water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor, and sesame oils), glycerol, tetrahydrofuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, coloring, perfuming, and preservative agents.

Suspensions, in addition to the active compounds, may contain suspending agents such as ethoxylated isostearyl alcohols, polyoxyethylene sorbitol, and sorbitan esters, microcrystalline cellulose, aluminum metahydroxide, bentonite, agar-agar and tragacanth, and mixtures thereof.

The compositions of the invention may also contain adjuvants, such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption, such as aluminum monostearate and gelatin.

It is understood that the dosage regimen will be determined by the attending physician considering various factors which modify the action of the subject compounds of the invention. The various factors include, but are not limited to, amount of bone weight desired to be formed, the degree of bone density loss, the site of bone damage, the condition of the damaged bone, the patient's age, sex, and diet, the severity of any disease that may be contributing to bone loss, time of administration, and other clinical factors. Optionally, the dosage may vary with the type of matrix used in the reconstitution and the types of compounds in the composition. The addition of other known growth factors to the final composition, may also affect the dosage. Progress can be monitored by periodic assessment of bone growth and/or repair, for example, X-rays (including DEXA), histomorphometric determinations, and tetracycline labeling.

Experiments shown below demonstrate the effects of ActRIIb-Fc on bone may be achieved with a single dose of 1 mg/kg or greater. The observed serum half-life of the molecule is between about 10 and 16 days. Thus a sustained effective serum level may be achieved, for example, by dosing with about 0.5 to 5 mg/kg on a weekly or biweekly basis. For example, doses of 0.3, 0.5, 1, 2, 3 or 5 mg/kg, or values in between, might be used once every 7 days, once every 10 days, once every 15 days, or on a monthly or bimonthly basis. Other exemplary dosing regimens are provided above.

In certain embodiments, the present invention also provides gene therapy for the in vivo production of ActRIIb polypeptides. Such therapy would achieve its therapeutic effect by introduction of the ActRIIb polynucleotide sequences into cells or tissues having the disorders as listed above. Delivery of ActRIIb polynucleotide sequences can be achieved using a recombinant expression vector such as a chimeric virus or a colloidal dispersion system. Preferred for therapeutic delivery of ActRIIb polynucleotide sequences is the use of targeted liposomes.

Various viral vectors which can be utilized for gene therapy as taught herein include adenovirus, herpes virus, vaccinia, or, preferably, an RNA virus such as a retrovirus. Preferably, the retroviral vector is a derivative of a murine or avian retrovirus. Examples of retroviral vectors in which a single foreign gene can be inserted include, but are not limited to: Moloney murine leukemia virus (MoMuLV), Harvey murine sarcoma virus (HaMuSV), murine mammary tumor virus (MuMTV), and Rous Sarcoma Virus (RSV). A number of additional retroviral vectors can incorporate multiple genes. All of these vectors can transfer or incorporate a gene for a selectable marker so that transduced cells can be identified and generated. Retroviral vectors can be made target-specific by attaching, for example, a sugar, a glycolipid, or a protein. Preferred targeting is accomplished by using an antibody. Those of skill in the art will recognize that specific polynucleotide sequences can be inserted into the retroviral genome or attached to a viral envelope to allow target specific delivery of the retroviral vector containing the ActRIIb polynucleotide. In a preferred embodiment, the vector is targeted to bone or cartilage.

Alternatively, tissue culture cells can be directly transfected with plasmids encoding the retroviral structural genes gag, pol and env, by conventional calcium phosphate transfection. These cells are then transfected with the vector plasmid containing the genes of interest. The resulting cells release the retroviral vector into the culture medium.

Another targeted delivery system for ActRIIb polynucleotides is a colloidal dispersion system. Colloidal dispersion systems include macromolecule complexes, nanocapsules, microspheres, beads, and lipid-based systems including oil-in-water emulsions, micelles, mixed micelles, and liposomes. The preferred colloidal system of this invention is a liposome. Liposomes are artificial membrane vesicles which are useful as delivery vehicles in vitro and in vivo. RNA, DNA and intact virions can be encapsulated within the aqueous interior and be delivered to cells in a biologically active form (see e.g., Fraley, et al., Trends Biochem. Sci., 6:77, 1981). Methods for efficient gene transfer using a liposome vehicle, are known in the art, see e.g., Mannino, et al., Biotechniques, 6:682, 1988. The composition of the liposome is usually a combination of phospholipids, usually in combination with steroids, especially cholesterol. Other phospholipids or other lipids may also be used. The physical characteristics of liposomes depend on pH, ionic strength, and the presence of divalent cations.

Examples of lipids useful in liposome production include phosphatidyl compounds, such as phosphatidylglycerol, phosphatidylcholine, phosphatidylserine, phosphatidylethanolamine, sphingolipids, cerebrosides, and gangliosides. Illustrative phospholipids include egg phosphatidylcholine, dipalmitoylphosphatidylcholine, and distearoylphosphatidylcholine. The targeting of liposomes is also possible based on, for example, organ-specificity, cell-specificity, and organelle-specificity and is known in the art.

### EXEMPLIFICATION

The invention now being generally described, it will be more readily understood by reference to the following examples, which are included merely for purposes of illustration of certain embodiments and embodiments of the present invention, and are not intended to limit the invention.

### Example 1: ActRIIb-Fc Fusion Proteins.

Applicants constructed a soluble ActRIIb fusion protein that has the extracellular domain of human ActRIIb fused to a human or mouse Fc domain with a minimal linker (three glycine amino acids) in between. The constructs are referred to as ActRIIb-hFc and ActRIIb-mFc, respectively.

ActRIIb-hFc is shown below as purified from CHO cell lines (SEQ ID NO: 13)

The ActRIIb-hFc and ActRIIb-mFc proteins were expressed in CHO cell lines. Three different leader sequences were considered:
(i) Honey bee mellitin (HBML): MKFLVNVALVFMVVYISYIYA (SEQ ID NO: 14)
(ii) Tissue Plasminogen Activator (TPA): MDAMKRGLCCVLLLCGAVFVSP (SEQ ID NO: 15)
(iii) Native: MGAAAKLAFAVFLISCSSGA (SEQ ID NO: 16).

The selected form employs the TPA leader and has the following unprocessed amino acid sequence:

This polypeptide is encoded by the following nucleic acid sequence (SEQ ID NO:18):

N-terminal sequencing of the CHO-cell produced material revealed a major sequence of -GRGEAE (SEQ ID NO: 19). Notably, other constructs reported in the literature begin with an -SGR... sequence.

Purification could be achieved by a series of column chromatography steps, including, for example, three or more of the following, in any order: protein A chromatography, Q sepharose chromatography, phenylsepharose chromatography, size exclusion chromatography, and cation exchange chromatography. The purification could be completed with viral filtration and buffer exchange.

ActRIIb-Fc fusion proteins were also expressed in HEK293 cells and COS cells. Although material from all cell lines and reasonable culture conditions provided protein with muscle-building activity in vivo, variability in potency was observed perhaps relating to cell line selection and/or culture conditions. Purification was achieved as described in the specification above.

### Example 2: Generation of ActRIIb-Fc Mutants.

Applicants generated a series of mutations in the extracellular domain of ActRIIb and produced these mutant proteins as soluble fusion proteins between extracellular ActRIIb and an Fc domain. The background ActRIIb-Fc fusion has the sequence (Fc portion underlined)(SEQ ID NO:20):

Various mutations, including N- and C-terminal truncations, were introduced into the background ActRIIb-Fc protein. Based on the data presented in Example 1, it is expected that these constructs, if expressed with a TPA leader, will lack the N-terminal serine. Mutations were generated in ActRIIb extracellular domain by PCR mutagenesis. After PCR, fragments were purified through a Qiagen column, digested with SfoI and AgeI and gel purified. These fragments were ligated into expression vector pAID4 (see WO2006/012627) such that upon ligation it created fusion chimera with human IgG1. Upon transformation into E. coli DH5 alpha, colonies were picked and DNAs were isolated. For murine constructs (mFc), a murine IgG2a was substituted for the human IgG1. All mutants were sequence verified.

All of the mutants were produced in HEK293T cells by transient transfection. In summary, in a 500ml spinner, HEK293T cells were set up at 6x10⁵ cells/ml in Freestyle (Invitrogen) media in 250ml volume and grown overnight. Next day, these cells were treated with DNA:PEI (1:1) complex at 0.5 ug/ml final DNA concentration. After 4 hrs, 250 ml media was added and cells were grown for 7 days. Conditioned media was harvested by spinning down the cells and concentrated.

Mutants were purified using a variety of techniques, including, for example, protein A column and eluted with low pH (3.0) glycine buffer. After neutralization, these were dialyzed against PBS.

Mutants were also produced in CHO cells by similar methodology.

Mutants were tested in binding assays and/or bioassays described below. In some instances, assays were performed with conditioned medium rather than purified proteins.

### Example 3. Bioassay for GDF-11 and Activin-mediated signaling.

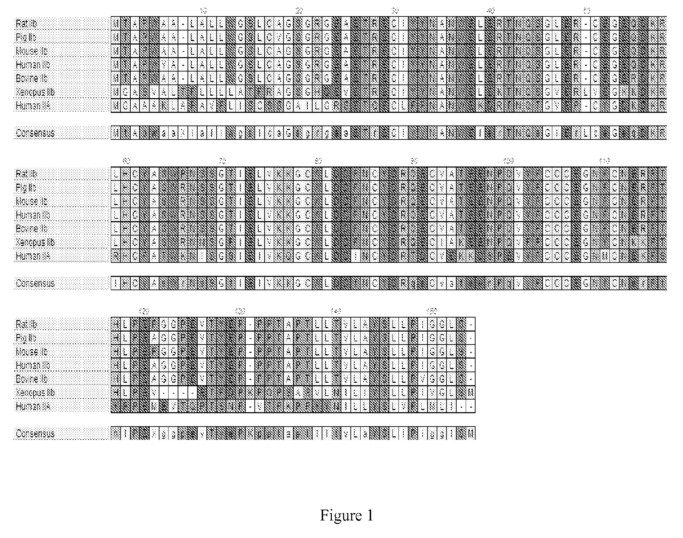

An A-204 Reporter Gene Assay was used to evaluate the effects of ActRIIb-Fc proteins on signaling by GDF-11 and Activin A. Cell line: Human Rhabdomyosarcoma (derived from muscle). Reporter vector: pGL3(CAGA)12 (Described in Dennler et al, 1998, EMBO 17: 3091-3100.) See Figure 1. The CAGA12 motif is present in TGF-Beta responsive genes (PAI-1 gene), so this vector is of general use for factors signaling through Smad2 and 3.
Day 1: Split A-204 cells into 48-well plate.
Day 2: A-204 cells transfected with 10 ug pGL3(CAGA)12 or pGL3(CAGA)12(10 ug)+ pRLCMV (1 ug) and Fugene.
Day 3: Add factors (diluted into medium+ 0.1 % BSA). Inhibitors need to be preincubated with Factors for 1 hr before adding to cells. 6 hrs later, cells rinsed with PBS, and lyse cells.

This is followed by a Luciferase assay. In the absence of any inhibitors, Activin A showed 10 fold stimulation of reporter gene expression and an ED50 ∼ 2 ng/ml. GDF-11: 16 fold stimulation, ED50: ∼ 1.5 ng/ml.

ActRIIb(R64, 20-134) is a potent inhibitor of activin, GDF-8 and GDF-11 activity in this assay. Variants were tested in this assay as well.

### Example 4: Human Clinical Trial, Single Ascending Dose.

The ActRIIb(20-134)-hFc protein described in Example 1 was administered to human patients in a randomized, double-blind, placebo-controlled study that was conducted to evaluate, primarily, the safety of the protein in healthy, postmenopausal women. Forty-eight subjects were randomized into six cohorts of 8 subjects to receive either a single dose of ActRIIb-hFc or placebo (6 active:2 placebo). Dose levels ranged from 0.02 to 3.0 mg/kg administered subcutaneously (SC). All subjects were followed for 57 days. Subjects were excluded from study participation if they took medications affecting bone metabolism within 6 months of study entry. Safety evaluations were conducted following each cohort to determine dose escalation. In addition to pharmacokinetic (PK) analyses, the biologic activity of ActRIIb-hFc was also assessed by measurement of biochemical markers of bone formation and resorption, and FSH levels.

No serious adverse events were reported in this study.

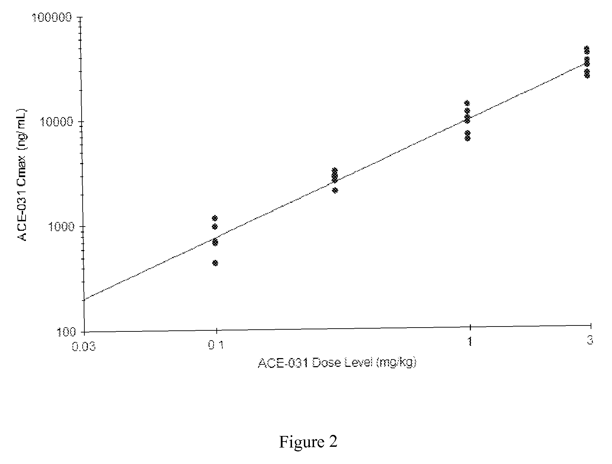

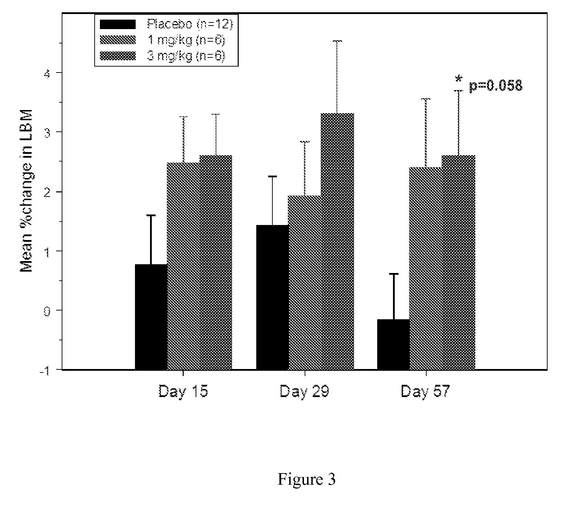

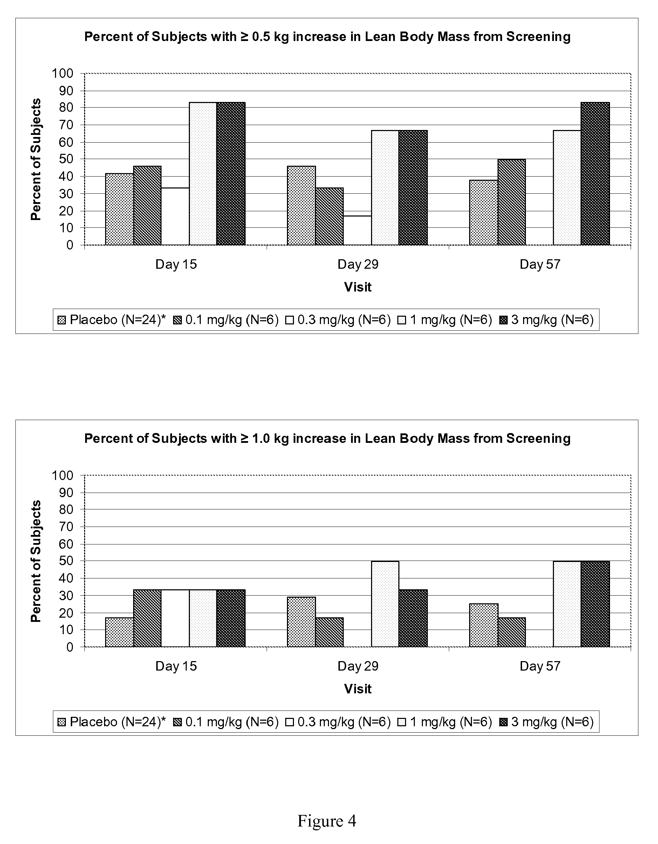

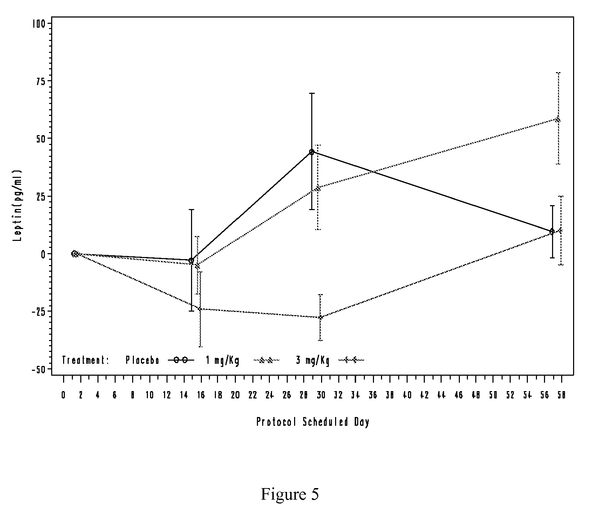

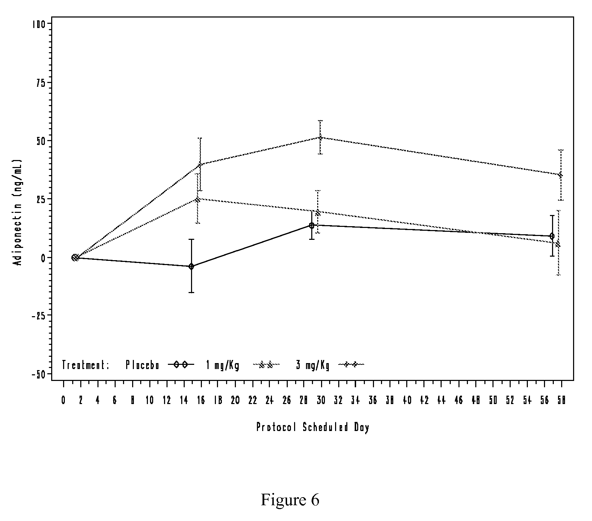

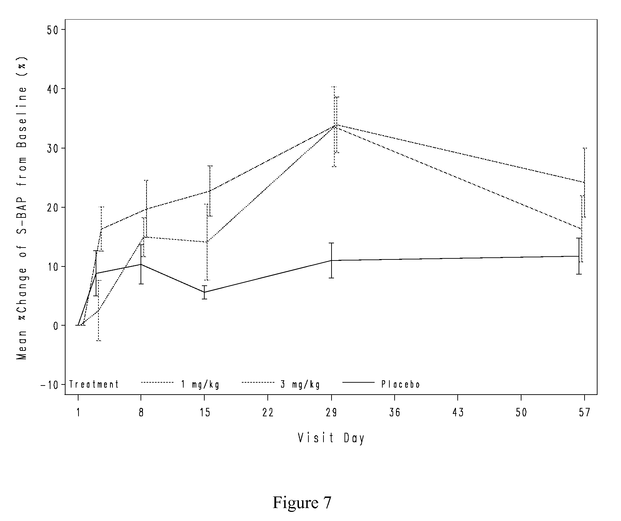

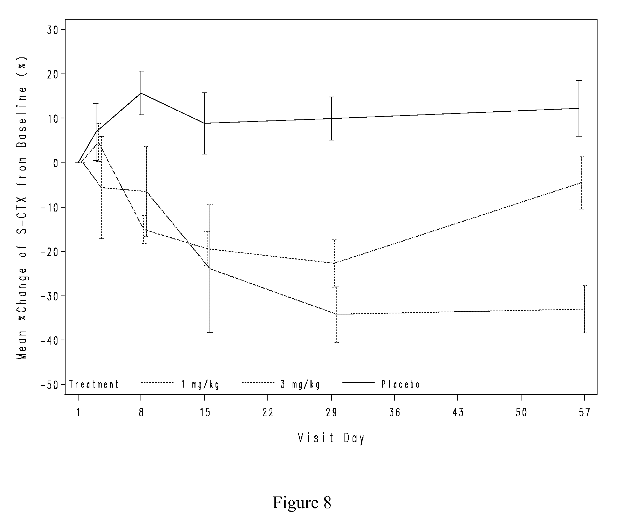

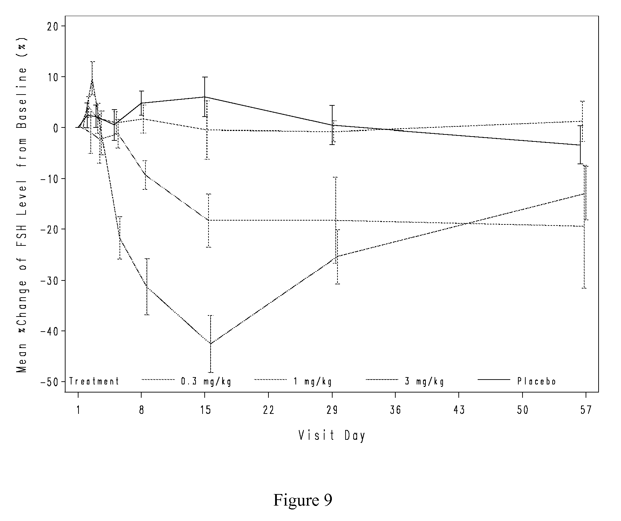

PK analysis of ActRIIb-hFc displayed a linear profile with dose, and a mean half-life of approximately 10-16 days (see Figure 2). The area-under-curve (AUC) for ActRIIb-hFc was linearly related to dose, and the absorption after SC dosing was essentially complete. At higher doses, ActRIIb-hFc caused in increases in lean body mass (LBM) as demonstrated using dual energy X-ray absorptiometry (DXA) scans (see Figure 3). The mean increase in LBM as compared to baseline was 2.6% (0.92 kg) at two months post dose at 3 mg/kg. The placebo treated group decreased by 0.2%. More subjects at the higher doses (1 and 3 mg/kg) had at least a 0.5 kg increase in LBM as early as 15 days after dosing with ActRIIb-hFc that was sustained until day 57 when compared to placebo and the lower doses (see Figure 4). In subjects dosed with ActRIIb-hFc at 1 and 3 mg/kg, significant dose-dependent changes were measured in serum biomarkers of fat metabolism (increased adiponectin and decreased leptin) (see Figures 5 and 6). ActRIIb-hFc caused a rapid, sustained dose-dependent increase in serum levels of bone-specific alkaline phosphatase (BSAP), which is a marker for anabolic bone growth, and a dose-dependent decrease in C-terminal type 1 collagen telopeptide (CTX), which is a marker for bone resorption (see Figures 7 and 8). BSAP levels showed near saturating effects at the highest dosage of drug, indicating that half-maximal effects on this anabolic bone biomarker could be achieved at a dosage of 1 mg/kg or lower. These bone biomarker changes were sustained for approximately 57 days at the highest dose levels tested. There was also a dose-dependent decrease in serum FSH levels consistent with inhibition of activin (see Figure 9).

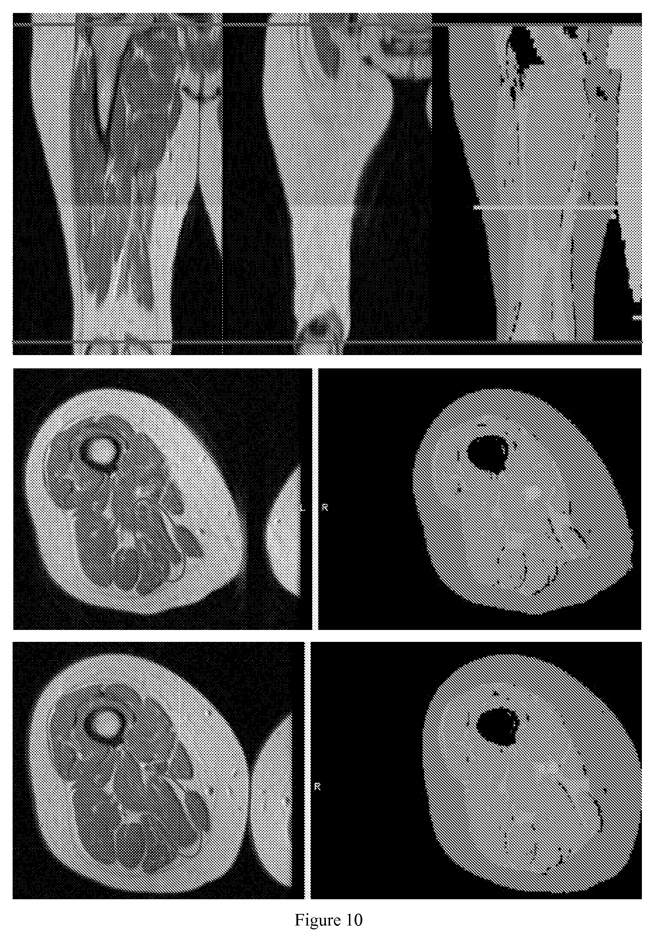

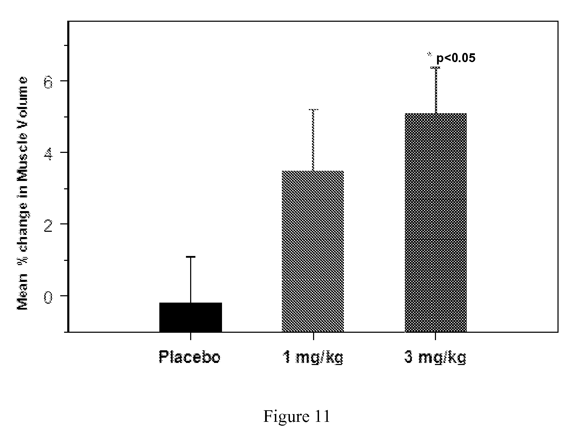

Increases in thigh muscle volume were observed by magnetic resonance imaging (MRI) at 29 days after single doses of ActRIIb-hFc at 1 and 3 mg/kg, with a significant increase at the highest dose (3 mg/kg) as compared to placebo (see Figure 10). In particular, at 29 days after administration of a single dose of 3 mg/kg of ActRIIb-hFc, a 16.4% increase in muscle area, an 11.5% increase in muscle volume, and a 2.2% decrease in subcutaneous fat was observed. The mean percent change from baseline in thigh muscle volume at day 29 with 1 and 3 mg/kg dosage levels as compared to placebo is shown in Figure 11.

A single dose of ActRIIb-hFc given to healthy postmenopausal women was safe and well-tolerated for the range of dose levels tested. This clinical trial demonstrates that, in humans, ActRIIb-hFc is an osteoanabolic agent with biological evidence of both an increase in bone formation and a decrease in bone resorption. ActRIIb-hFc also increases muscle size and function.

### Example 5: Human Clinical Trial, Multiple Ascending Doses.

A randomized, double-blind, placebo-controlled, multiple-dose, dose-escalating study was conducted to evaluate, primarily, the safety, tolerability, PK, and PD effects of ActRIIb-hFc in healthy postmenopausal women. The study included six cohorts of 10 subjects each. Subjects in each cohort were randomized to receive either ActRIIb-hFc or placebo (8 active and 2 placebo subjects per cohort).

Cohorts were dosed as follows: Cohort 1 (0.1 mg/kg), Cohort 2 (0.3 mg/kg) and Cohort 3 (1 mg/kg) received ActRIIb-hFc subcutaneously (SC) every 14 days for a total of 3 doses on Days 1, 15, and 29. Cohort 4 (1 mg/kg), Cohort 5 (2 mg/kg), and Cohort 6 (3 mg/kg) received ActRIIb-hFc SC every 28 days for a total of 2 doses on Days 1 and 29.

The following data were evaluated: adverse events, clinical laboratory tests (hematology, chemistry, urinalysis, endocrine function tests, adrenal function tests, ACTH stimulation tests), vital signs including supine (after at least 5 minutes) and standing (after 2 minutes ± 30 seconds) blood pressure, ECG, echocardiogram (ECHO), physical examinations, and anti-drug antibodies. Pharmacokinetics: Area under the serum ActRIIb-hFc concentration curve (AUC), Peak concentration (Cmax), Time to peak concentration (Tmax), Elimination half-life (t1/2), Clearance (Cl/F) and Volume of distribution (Vz/F). Pharmacodynamics: FSH, Lean body mass as measured by total body DXA scans, Bone mineral density (BMD) as measured by lumbar spine, hip and total body DXA scans, Muscle size as measured by MRI scans in Cohorts 3-6, and other markers of pharmacodynamic effect.

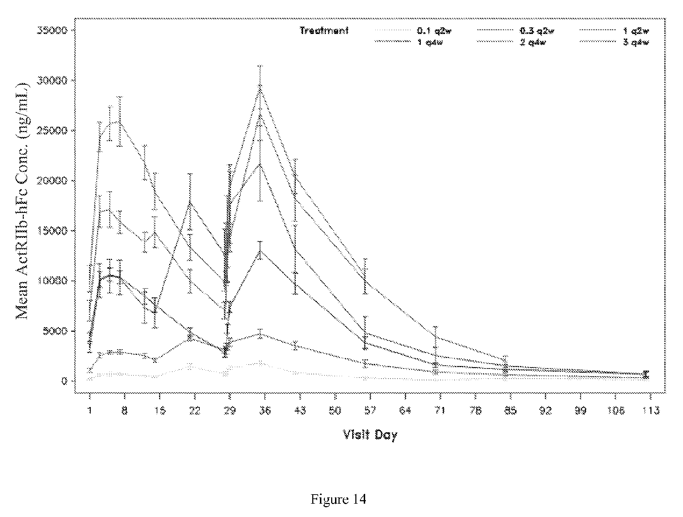

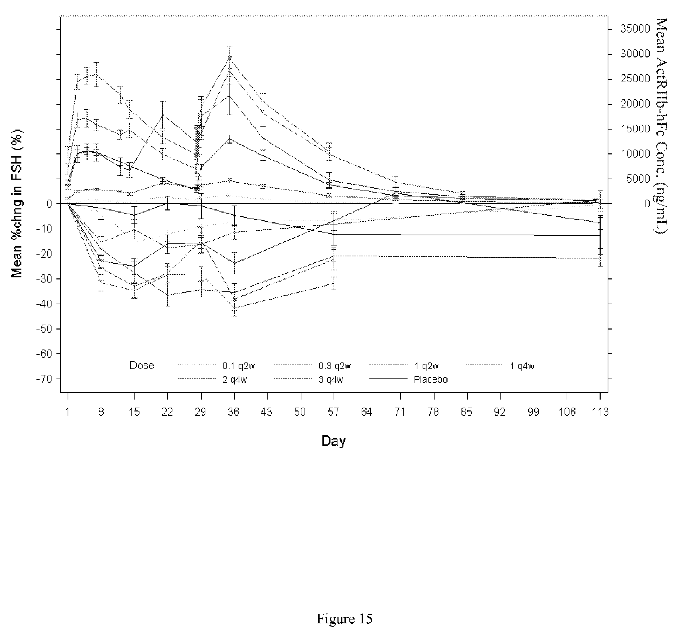

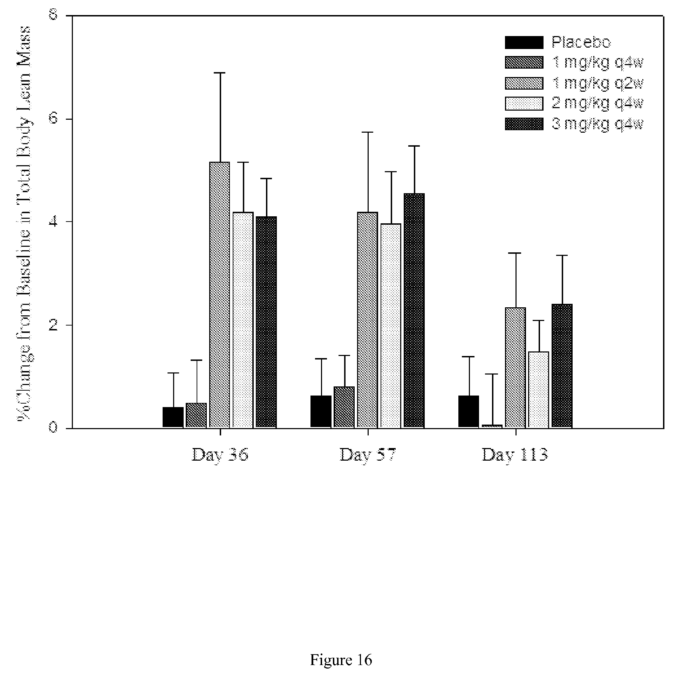

As shown in Figure 14, serum concentrations of ActRIIb-hFc were related to the dose amount and frequency. Notably, the 2 mg/kg and 3 mg/kg doses given every 28 days achieved high peak concentrations with marked troughs. The 1 mg/kg dose given every 14 days achieved a rising concentration with smaller troughs. After the second dose, the 1 mg/kg given every 14 days maintained a mean serum concentration in patients above approximately 12 micrograms/mL, while the 2 mg/kg and 3 mg/kg doses given every 28 days exhibited troughs to a mean of approximately 8 and 10 micrograms/mL, respectively. As shown in Figure 15, the troughs in serum concentration were reflected in effects on FSH. FSH production is thought to be stimulated by activins, and therefore the use of an activin antagonist such as ActRIIb-hFc is expected to inhibit FSH production. In fact all doses of ActRIIb-hFc exhibited some inhibition of FSH. This is shown by the traces dipping below the X-axis in Figure 15. Interestingly, the 1 mg/kg dose given every 14 days exhibited no mean diminishment in FSH inhibition during the dosing period, while the 2 mg/kg and 3 mg/kg doses given every 28 days did exhibit a substantial increase in FSH production (hence a decrease in FSH suppression) that corresponded to the trough in serum concentration (roughly, between study days 15 and 36). We conclude that dosing ActRIIb-hFc proteins in a manner that allows serum concentration to drop as low as 8 or 10 micrograms/mL causes a transient release in the suppression of activin signaling by the drug, and, perhaps a transient release in the suppression of other ligands, such as myostatin or GDF-11. Notably, the 1 mg/kg dose given every 14 days appeared to cause a greater increase in muscle mass, as measured by total body lean mass (see Figure 16) than did the less frequent 2 mg/kg and 3 mg/kg doses. While patients on the 2 mg/kg and 3 mg/kg doses given every 28 days received a total of 4 or 6 mg/kg of drug during the study, patients on the 1 mg/kg dose given every 14 days received a total of only 3 mg/kg of drug during the study. Nonetheless, the lower, more frequent dose provided an apparent greater effect on muscle mass (exhibited in this study as a trend not reaching statistical significance). It is expected that this superior efficacy at a lower dose is due to the more sustained ligand suppression that occurs with the more frequent dosing regimen, as shown by the effects on FSH. As a consequence, it is expected that dosing regimen that maintain a serum concentration above about 8, 10 or 12 micrograms per mL will provide an optimal efficacy for drugs based on the ActRIIb-hFc mode of action. This conclusion should be applicable to a wide array of ActRIIb-hFc proteins, regardless of serum half-life, provided that such proteins have an affinity for activins and myostatin/GDF-11 that is in the same range as that for wild-type ActRIIb-hFc.

While specific embodiments of the subject matter have been discussed, the above specification is illustrative and not restrictive. Many variations will become apparent to those skilled in the art upon review of this specification and the claims below. The full scope of the invention should be determined by reference to the claims.

## Claims

1. An ActRIIb-Fc fusion protein for use in treating a bone-related disorder or a muscle-related disorder in a patient, wherein the ActRIIb-Fc fusion protein is for administration to the patient on a dosing schedule that maintains a serum concentration of the ActRIIb-Fc fusion protein from 8-20 µg/mL.

2. Use of an ActRIIb-Fc fusion protein in the manufacture of a medicament for treating a bone-related disorder or a muscle-related disorder in a patient, wherein the ActRIIb-Fc fusion protein is for administration to the patient on a dosing schedule that maintains a serum concentration of the ActRIIb-Fc fusion protein from 8-20 µg/mL.

3. The fusion protein for use according to Claim 1, or the use according to Claim 2, wherein the serum concentration of the ActRIIb-Fc fusion protein is maintained at a concentration of:
a. at least 10 µg/mL; or
b. at least 12 µg/mL.

4. The fusion protein for use according to Claim 1 or Claim 3, or the use according to Claim 2 or Claim 3, wherein the dosing schedule involves administering
a. at least 0.3 mg/kg;
b. at least 1 mg/kg;
c. 2mg/kg; or
d. 1 mg/kg
of the ActRIIb-Fc fusion protein to the patient.

5. The fusion protein for use according to any one of Claims 1, 3 or 4, or the use according to any one of Claims 2 to 4, wherein the dosing schedule involves administering the ActRIIb-Fc fusion protein
to the patient
a. once every 5-30 days;
b. once every 10-16 days; or
c. once every 14 days.

6. The fusion protein for use according to any one of Claims 1 or 3 to 5, or the use according to any one of Claims 2 to 5, wherein the dosing schedule involves administering 1 mg/kg of the ActRIIb-Fc fusion protein to the patient once every 14 days.

7. The fusion protein for use according to any one of Claims 1 or 3 to 6, or the use according to any one of Claims 2 to 6, wherein the ActRIIb-Fc fusion protein is selected from the group consisting of:
a. a polypeptide comprising an amino acid sequence at least 90% identical to SEQ ID NO: 2, 3 or 13;
b. a polypeptide comprising an amino acid sequence at least 95% identical to SEQ ID NO: 2, 3 or 13;
c. a polypeptide comprising the amino acid sequence of SEQ ID NO: 2, 3 or 13;
d. a polypeptide comprising at least 50 consecutive amino acids of SEQ ID NO: 2;
e. a polypeptide comprising an amino acid sequence at least 90% identical to a portion of ActRIIb, wherein the N-terminus corresponds to an amino acid residue selected from amino acids 19-25 of SEQ ID NO: 1 and the C-terminus corresponds to an amino acid residue selected from amino acids 109-134 of SEQ ID NO: 1;
f. a polypeptide comprising an amino acid sequence at least 95% identical to a portion of ActRIIb, wherein the N-terminus corresponds to an amino acid residue selected from amino acids 19-25 of SEQ ID NO: 1 and the C-terminus corresponds to an amino acid residue selected from amino acids 109-134 of SEQ ID NO: 1; and
g. a polypeptide comprising a portion of ActRIIb, wherein the N-terminus corresponds to an amino acid residue selected from amino acids 19-25 of SEQ ID NO: 1 and the C-terminus corresponds to an amino acid residue selected from amino acids 109-134 of SEQ ID NO: 1.

8. The fusion protein for use according to any one of Claims 1 or 3 to 7, or the use according to any one of Claims 2 to 7, wherein said ActRIIb-Fc fusion protein includes one or more modified amino acid residues selected from: a glycosylated amino acid, a PEGylated amino acid, a farnesylated amino acid, an acetylated amino acid, a biotinylated amino acid, an amino acid conjugated to a lipid moiety, and an amino acid conjugated to an organic derivatizing agent.

9. The fusion protein for use according to any one of Claims 1 or 3 to 8, or the use according to any one of Claims 2 to 8, wherein the ActRIIb-Fc fusion protein comprises the amino acid sequence of SEQ ID NO: 3.

10. The fusion protein for use according to any one of Claims 1 or 3 to 9, or the use according to any one of Claims 2 to 9, wherein the ActRIIb-Fc fusion protein comprises the amino acid sequence of SEQ ID NO: 2.

11. The fusion protein for use according to any one of Claims 1 or 3 to 10, or the use according to any one of Claims 2 to 10, wherein the ActRIIb-Fc fusion protein comprises the amino acid sequence of SEQ ID NO: 13.

12. The fusion protein for use according to any one of Claims 1 or 3 to 11, or the use according to any one of Claims 2 to 11, wherein the ActRIIb-Fc fusion protein has a serum half-life of between 10 and 16 days in normal, healthy humans.

13. The fusion protein for use according to any one of Claims 1 or 3 to 12, or the use according to any one of Claims 2 to 12, wherein the ActRIIb-Fc fusion protein binds to one or more of activin A, activin AB, activin B, GDF11, and GDF8.

## Patentansprüche

1. ActRIIb-Fc-Fusionsprotein zur Verwendung beim Behandeln einer knochenbezogenen Störung oder einer muskelbezogenen Störung in einem Patienten, wobei das ActRIIb-Fc-Fusionsprotein zum Verabreichen an den Patienten nach einem Dosierungsplan dient, der eine Serumkonzentration des ActRIIb-Fc-Fusionsproteins auf 8-20 µg/ml hält.

2. Verwendung eines ActRIIb-Fc-Fusionsproteins bei der Herstellung eines Medikaments zum Behandeln einer knochenbezogenen Störung oder einer muskelbezogenen Störung in einem Patienten, wobei das ActRIIb-Fc-Fusionsprotein zur Verabreichung an den Patienten nach einem Dosierungsplan dient, der eine Serumkonzentration des ActRIIb-Fc-Fusionsproteins auf 8-20 µg/ml hält.

3. Fusionsprotein zur Verwendung nach Anspruch 1 oder Verwendung nach Anspruch 2, wobei die Serumkonzentration des ActRIIb-Fc-Fusionsproteins auf einer Konzentration von:
a. wenigstens 10 µg/ml; oder
b. wenigstens 12 µg/ml
gehalten wird.

4. Fusionsprotein zur Verwendung nach Anspruch 1 oder Anspruch 3 oder Verwendung nach Anspruch 2 oder Anspruch 3, wobei der Dosierungsplan das Verabreichen von:
a. wenigstens 0,3 mg/kg;
b. wenigstens 1 mg/kg;
c. 2 mg/kg; oder
d. 1 mg/kg
des ActRIIb-Fc-Fusionsproteins an den Patienten beinhaltet.

5. Fusionsprotein nach Anspruch 1, 3 oder 4 oder Verwendung nach einem der Ansprüche 2 bis 4, wobei der Dosierungsplan das Verabreichen des ActRIIb-Fc-Fusionsproteins an den Patienten
a. einmal alle 5-30 Tage;
b. einmal alle 10-16 Tage; oder
c. einmal alle 14 Tage
beinhaltet.

6. Fusionsprotein zur Verwendung nach einem der Ansprüche 1 oder 3 bis 5 oder Verwendung nach einem der Ansprüche 2 bis 5, wobei der Dosierungsplan das Verabreichen von 1 mg/kg des ActRIIb-Fc-Fusionsproteins an den Patienten einmal alle 14 Tage beinhaltet.

7. Fusionsprotein zur Verwendung nach einem der Ansprüche 1 oder 3 bis 6 oder Verwendung nach einem der Ansprüche 2 bis 6, wobei das ActRIIb-Fc-Fusionsprotein ausgewählt ist aus der Gruppe bestehend aus:
a. einem Polypeptid, das eine Aminosäuresequenz umfasst, die zu wenigstens 90 % identisch mit SEQ ID Nr. 2, 3 oder 13 ist;
b. einem Polypeptid, das eine Aminosäuresequenz umfasst, die zu wenigstens 95 % identisch mit SEQ ID Nr. 2, 3 oder 13 ist;
c. einem Polypeptid, das die Aminosäuresequenz von SEQ ID Nr. 2, 3 oder 13 umfasst;
d. einem Polypeptid, das wenigstens 50 konsekutive Aminosäuren der SEQ ID Nr. 2 umfasst;
e. einem Polypeptid, das eine Aminosäuresequenz umfasst, die zu wenigstens 90 % identisch mit einem Abschnitt von ActRIIb ist, wobei der N-Terminus einem Aminosäurerest entspricht, ausgewählt aus den Aminosäuren 19-25 von SEQ ID Nr. 1, und der C-Terminus einem Aminosäurerest entspricht, ausgewählt aus den Aminosäuren 109-134 von SEQ ID Nr. 1;
f. einem Polypeptid, das eine Aminosäuresequenz umfasst, die zu wenigstens 95 % identisch mit einem Abschnitt von ActRIIb ist, wobei der N-Terminus einem Aminosäurerest entspricht, der aus den Aminosäuren 19-25 von SEQ ID Nr. 1 ausgewählt ist, und der C-Terminus einem Aminosäurerest entspricht, der aus den Aminosäuren 109-134 von SEQ ID Nr. 1 ausgewählt ist; und
g. einem Polypeptid, das einen Abschnitt von ActRIIb umfasst, wobei der N-Terminus einem Aminosäurerest entspricht, der ausgewählt ist aus den Aminosäuren 19-25 von SEQ ID Nr. 1, und der C-Terminus einem Aminosäurerest entspricht, der ausgewählt ist aus den Aminosäuren 109-134 von SEQ ID Nr. 1.

8. Fusionsprotein zur Verwendung nach einem der Ansprüche 1 oder 3 bis 7 oder Verwendung nach einem der Ansprüche 2 bis 7, wobei das genannte ActRIIb-Fc-Fusionsprotein einen oder mehrere modifizierte Aminosäurereste beinhaltet, ausgewählt aus: einer glykosylierten Aminosäure, einer PEGylierten Aminosäure, einer farnesylierten Aminosäure, einer acetylierten Aminosäure, einer biotinylierten Aminosäure, einer mit einem Lipidanteil konjugierten Aminosäure und einer mit einem organischen Derivatisierungsmittel konjugierten Aminosäure.

9. Fusionsprotein zur Verwendung nach einem der Ansprüche 1 oder 3 bis 8 oder Verwendung nach einem der Ansprüche 2 bis 8, wobei das ActRIIb-Fc-Fusionsprotein die Aminosäuresequenz von SEQ ID Nr. 3 umfasst.

10. Fusionsprotein zur Verwendung nach einem der Ansprüche 1 oder 3 bis 9 oder Verwendung nach einem der Ansprüche 2 bis 9, wobei das ActRIIb-Fc-Fusionsprotein die Aminosäuresequenz von SEQ ID Nr. 2 umfasst.

11. Fusionsprotein zur Verwendung nach einem der Ansprüche 1 oder 3 bis 10 oder Verwendung nach einem der Ansprüche 2 bis 10, wobei das ActRIIb-Fc-Fusionsprotein die Aminosäuresequenz von SEQ ID Nr. 13 umfasst.

12. Fusionsprotein zur Verwendung nach einem der Ansprüche 1 oder 3 bis 11 oder Verwendung nach einem der Ansprüche 2 bis 11, wobei das ActRIIb-Fc-Fusionsprotein eine Serum-Halbwertzeit zwischen 10 und 16 Tagen in normalen, gesunden Menschen hat.

13. Fusionsprotein zur Verwendung nach einem der Ansprüche 1 oder 3 bis 12 oder Verwendung nach einem der Ansprüche 2 bis 12, wobei das ActRIIb-Fc-Fusionsprotein sich an eines oder mehrere aus Activin A, Activin AB, Activin B, GDF11 und GDF8 bindet.

## Revendications

1. Protéine de fusion ActRIIb-Fc destinée à une utilisation dans le traitement d'une affection osseuse ou d'une affection musculaire chez un patient, la protéine de fusion ActRIIb-Fc étant destinée à une administration au patient selon une posologie qui maintient une concentration sérique de 8-20 µg/ml de la protéine de fusion ActRIIb-Fc.

2. Utilisation d'une protéine de fusion ActRIIb-Fc dans la fabrication d'un médicament pour le traitement d'une affection osseuse ou d'une affection musculaire chez un patient, la protéine de fusion ActRIIb-Fc étant destinée à une administration au patient selon une posologie qui maintient une concentration sérique de 8-20 µg/ml de la protéine de fusion ActRIIb-Fc.

3. Protéine de fusion destinée à une utilisation selon la revendication 1, ou utilisation selon la revendication 2, la concentration sérique de la protéine de fusion ActRIIb-Fc étant maintenue à une concentration de :
a. au moins 10 µg/ml ; ou
b. au moins 12 µg/ml.

4. Protéine de fusion destinée à une utilisation selon la revendication 1 ou la revendication 3, ou utilisation selon la revendication 2 ou la revendication 3, la posologie impliquant l'administration de
a. au moins 0,3 mg/kg ;
b. au moins 1 mg/kg ;
c. 2 mg/kg ; ou
d. 1 mg/kg
de la protéine de fusion ActRIIb-Fc au patient.

5. Protéine de fusion destinée à une utilisation selon l'une quelconque des revendications 1, 3 ou 4, ou utilisation selon l'une quelconque des revendications 2 à 4, la posologie impliquant l'administration de la protéine de fusion ActRIIb-Fc au patient
a. une fois tous les 5-30 jours ;
b. une fois tous les 10-16 jours ; ou
c. une fois tous les 14 jours.

6. Protéine de fusion destinée à une utilisation selon l'une quelconque des revendications 1 ou 3 à 5, ou utilisation selon l'une quelconque des revendications 2 à 5, la posologie impliquant l'administration de 1 mg/kg de la protéine de fusion ActRIIb-Fc au patient une fois tous les 14 jours.

7. Protéine de fusion destinée à une utilisation selon l'une quelconque des revendications 1 ou 3 à 6, ou utilisation selon l'une quelconque des revendications 2 à 6, la protéine de fusion ActRIIb-Fc étant sélectionnée dans le groupe constitué de :
a. un polypeptide comprenant une séquence d'acides aminés au moins 90 % identique à la SÉQ. ID n° 2, 3 ou 13 ;
b. un polypeptide comprenant une séquence d'acides aminés au moins 95 % identique à la SÉQ. ID n° 2, 3 ou 13 ;
c. un polypeptide comprenant la séquence d'acides aminés correspondant à la SÉQ. ID n° 2, 3 ou 13 ;
d. un polypeptide comprenant au moins 50 acides aminés consécutifs correspondant à la SÉQ. ID n° 2 ;
e. un polypeptide comprenant une séquence d'acides aminés au moins 90 % identique à une partie d'ActRIIb, la terminaison N correspondant à un résidu acide aminé sélectionné parmi les acides aminés 19-25 de la SÉQ. ID n° 1 et la terminaison C correspondant à un résidu acide aminé sélectionné parmi les acides aminés 109-134 de la SÉQ. ID n° 1 ;
f. un polypeptide comprenant une séquence d'acides aminés au moins 95 % identique à une partie d'ActRIIb, la terminaison N correspondant à un résidu acide aminé sélectionné parmi les acides aminés 19-25 de la SÉQ. ID n° 1 et la terminaison C correspondant à un résidu acide aminé sélectionné parmi les acides aminés 109-134 de la SÉQ. ID n° 1 ; et
g. un polypeptide comprenant une partie d'ActRIIb, la terminaison N correspondant à un résidu acide aminé sélectionné parmi les acides aminés 19-25 de la SÉQ. ID n° 1 et la terminaison C correspondant à un résidu acide aminé sélectionné parmi les acides aminés 109-134 de la SÉQ. ID n° 1.

8. Protéine de fusion destinée à une utilisation selon l'une quelconque des revendications 1 ou 3 à 7, ou utilisation selon l'une quelconque des revendications 2 à 7, ladite protéine de fusion ActRIIb-Fc contenant un ou plusieurs résidus acides aminés modifiés sélectionnés parmi : un acide aminé glycosylé, un acide aminé PEGylé, un acide aminé farnésylé, un acide aminé acétylé, un acide aminé biotinylé, un acide aminé conjugué à une fraction lipidique, et un acide aminé conjugué à un agent de dérivatisation organique.

9. Protéine de fusion destinée à une utilisation selon l'une quelconque des revendications 1 ou 3 à 8, ou utilisation selon l'une quelconque des revendications 2 à 8, la protéine de fusion ActRIIb-Fc comprenant la séquence d'acides aminés correspondant à la SÉQ. ID n° 3.

10. Protéine de fusion destinée à une utilisation selon l'une quelconque des revendications 1 ou 3 à 9, ou utilisation selon l'une quelconque des revendications 2 à 9, la protéine de fusion ActRIIb-Fc comprenant la séquence d'acides aminés correspondant à la SÉQ. ID n° 2.

11. Protéine de fusion destinée à une utilisation selon l'une quelconque des revendications 1 ou 3 à 10, ou utilisation selon l'une quelconque des revendications 2 à 10, la protéine de fusion ActRIIb-Fc comprenant la séquence d'acides aminés correspondant à la SÉQ. ID n° 13.

12. Protéine de fusion destinée à une utilisation selon l'une quelconque des revendications 1 ou 3 à 11, ou utilisation selon l'une quelconque des revendications 2 à 11, la protéine de fusion ActRIIb-Fc ayant une demi-vie sérique de 10 à 16 jours chez des humains normaux, en bonne santé.

13. Protéine de fusion destinée à une utilisation selon l'une quelconque des revendications 1 ou 3 à 12, ou utilisation selon l'une quelconque des revendications 2 à 12, la protéine de fusion ActRIIb-Fc se liant à une ou plusieurs de l'activine A, de l'activine AB, de l'activine B, de GDF11, et de GDF8.
